# FASCICULE DE BREVET EUROPEEN

(11) **EP 0 462 009 B1**
(45) Date de publication et mention de la délivrance du brevet: **28.10.1998**
(21) Numéro de dépôt: 91401570.6
(22) Date de dépôt: 13.06.1991
(51) Int. Cl.: C07D 501/24, A61K 31/545

(54) **Céphalosporines comportant en position 3 un radical propényle substitué par un ammonium quaternaire, leur procédé de préparation, leur application comme médicaments, les compositions les renfermant et les intermédiaires obtenus**
Cephalosporine mit in der Lage 3 ein Propenylradikal, substituiert durch eine quaternäre Ammoniumgruppe, ihr Verfahren zur Herstellung, ihre Anwendung als Medikamente, ihre sie enthaltende Präparate und ihre Zwischenprodukte
Cephalosporins having in position 3 a propenyl radical substituted by a quaternary ammonium, their process for preparation, their use as medicaments, compositions containing them and intermediates

(30) Priorité: 15.06.1990 FR 9007491
(43) Date de publication de la demande: 18.12.1991
(73) Titulaire: HOECHST MARION ROUSSEL, 92800 Puteaux (FR)
(72) Inventeur: Aszodi, Jozsef, F-94600 Choisy le Roi (FR); Chantot, Jean-François, F-77410 Gressy en France (FR); Gouin d'Ambrieres, Solange, F-75020 Paris (FR)
(74) Mandataire: Vieillefosse, Jean-Claude

(56) Documents cités:
- EP-A- 0 264 091
- EP-A- 0 266 060
- EP-A- 0 315 518
- EP-A- 0 333 154
- WO-A-87/03875
- GB-A- 2 134 522
- GB-A- 2 157 293

## Description

La présente invention concerne de nouvelles céphalosporines comportant en position 3 un radical propényle substitué par un ammonium quaternaire, leur procédé de préparation, leur application comme médicaments, les compositions les renfermant et les nouveaux intermédiaires obtenus.

On connait par les documents cités ci-après, à savoir GB-A-2 157293, GB-A-2 134522, EP-A-0 266060, EP-A-0 315518, EP-A-0 333154, EP-A-0 264091 et WO-A-87/03875, des composés de type céphalosporines comportant en 7 une chaine latérale amino thiazolyl acétamido substituée par une fonction alkyle oxime elle-même éventuellement substituée, et en 3, notamment un radical propényl substitué par un ammonium quaternaire, composés possédant des propriétés antibactériennes.

L'invention a pour objet les produits de formule générale (1) : 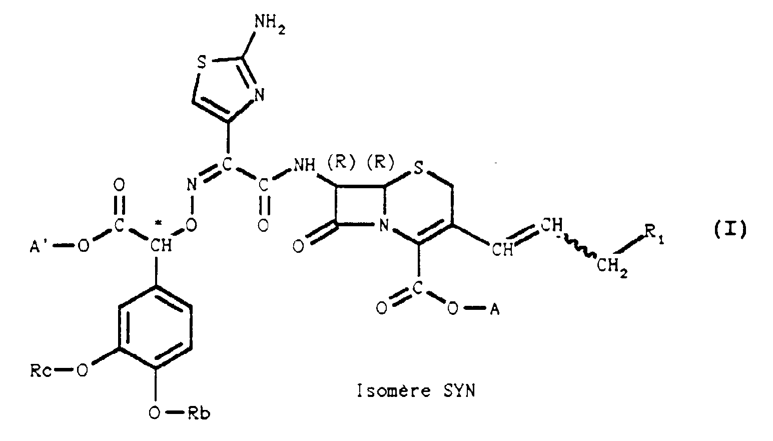 isomère syn, sous forme R ou S ou d'un mélange R, S, formule dans laquelle : R, représente un radical choisi parmi les radicaux suivants : 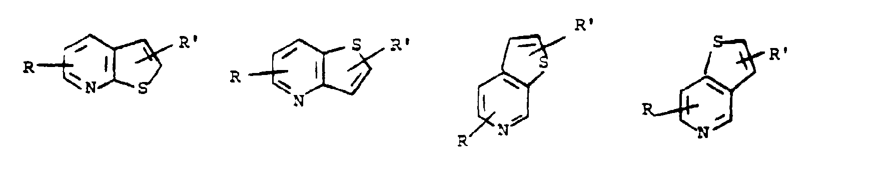 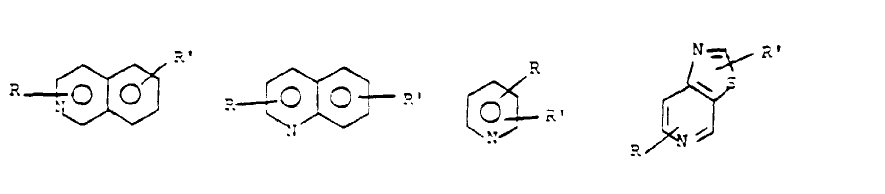 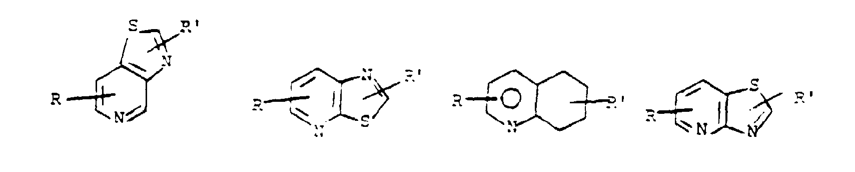 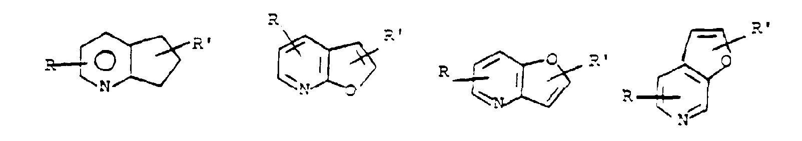 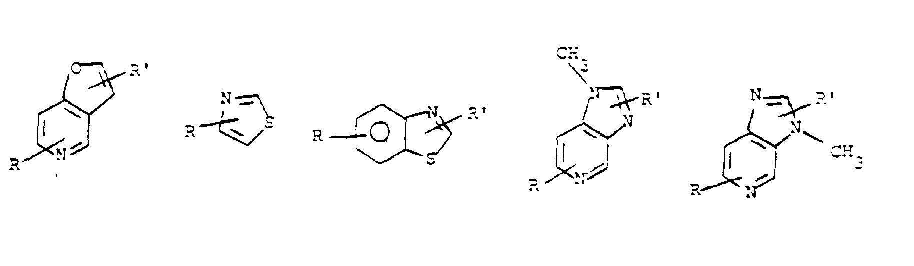 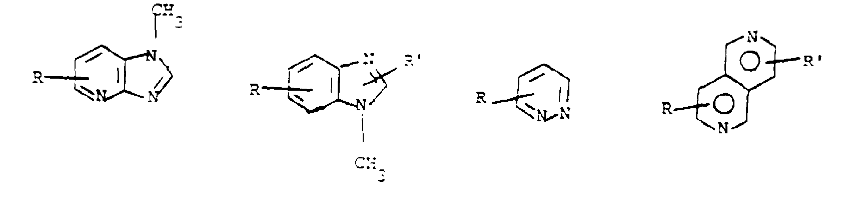 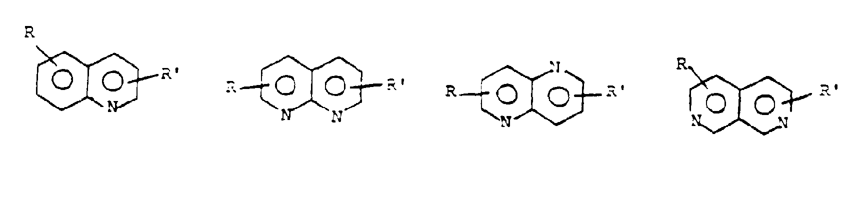 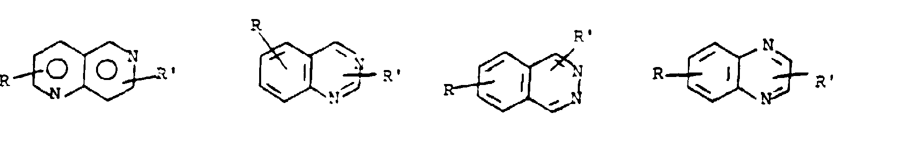 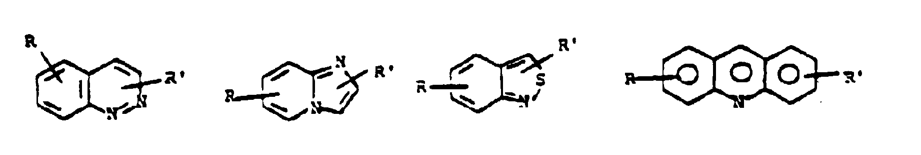 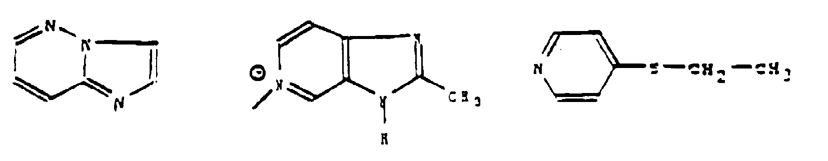 ou 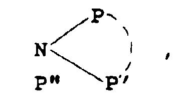 sous forme d'ammonium quaternaire, l'expression sous forme d'ammonium quaternaire indiquant que le radical R₁ est lié avec le groupement -CH=CH-CH₂ par le ou l'un des atomes d'azote qu'il comporte, dans lesquels R et R' identiques ou différents représentent un atome d'hydrogène, un radical alkyle renfermant de 1 à 4 atomes de carbone, un radical alcoxy renfermant de 1 à 4 atomes de carbone, un atome d'halogène, un radical C0₂-Q, 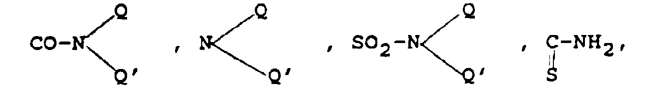 NH-CO-Q, CN CH₂-CN, CH₂-SQ dans lesquels Q et Q' identiques ou différents représentent un atome d'hydrogène ou un radical alkyle renfermant de 1 à 4 atomes de carbone, P, P' et P" identiques ou différents représentent un radical alkyle renfermant au plus 4 atomes de carbone, éventuellement substitué par un des substituants indiqués ci-dessus pour R et R', le symbole ;
indiqant que P et P' peuvent éventuellement former avec l'atome d'azote auquel ils sont liés, un hétérocycle à 5 ou 6 chaînons,
R_{b} et R_{c}, identiques ou différents représentent un atome d'hydrogène ou un groupement acyle, choisi parmi les radicaux acétyle, propionyle et benzoyle,
A et A' identiques ou différents représentent un atome d'hydrogène, un équivalent de métal alcalin, alcalino-terreux, de magnésium, d'ammonium ou d'une base organique aminée ou A et A' représentent le reste d'un groupement ester facilement clivable ou C0₂A représente OO₂ le trait ondulé signifie que le groupement CH₂R₁ peut se trouver dans la position E ou Z ainsi que les sels des produits de formule (1) avec les acides minéraux ou organiques.

Par radical alkyle renfermant de 1 à 4 atomes de carbone, on entend par exemple, méthyle, éthyle, propyle, isopropyle, butyle linéaire ou ramifié.

Lorsque P et P' forment un hétérocycle avec l'atome d'azote auquel ils sont liés, il peut s'agir d'une pyrrolidine, d'une morpholine ou d'une pipéridine.

Lorsque R_{b} et/ou Rₑ représentent un radical acyle, il peut s'agir des radicaux acétyle, propionyle ou benzoyle. La valeur acyle préférée est la valeur acétyle. La valeur préférée pour R_{b} et R_{c} est la valeur hydrogène.

Parmi les valeurs de A et de A' on peut citer un équivalent de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris[(hydroxyméthyl) amino] méthane, l'éthanolamine, la pyridine, la picoline, la dicyclohexylamine, la morpholine, la benzylamine, la procaïne, la lysine, l'arginine, l'histidine, la N-méthylglucamine.

On peut citer entre autres restes de groupements ester facilement clivables que peuvent représenter A et A', les groupements méthoxyméthyle, éthoxyméthyle, isopropyloxyméthyle, alpha-méthoxy éthyle, alpha-éthoxy éthyle, mé- thylthiométhyle, éthylthiométhyle, isopropylthiométhyle, pivaloyloxyméthyle, acétoxyméthyle, propionyloxyméthyle, butyryloxyméthyle, isobutyryloxyméthyle, valéryloxyméthyle, isovaléryloxyméthyle, tert-butylcarbonyloxyméthyle, hexadécanoyloxyméthyle, propionyloxyéthyle, isovaléryloxyéthyle, 1-acétyloxyéthyle, 1-propionyloxyéthyle, 1-butyryloxyéthyle, 1-tert-butylcarbonyloxyéthyle, 1-acétyloxypropyle, 1-hexadécanoyloxyéthyle, 1-propionyloxypropyle, 1-méthoxycarbonyloxyéthyle, méthoxycarbonyloxyméthyle, 1-acétyloxybutyle, 1-acétyloxyhexyle, 1-acétyloxyheptyle, phtalidyle, 5,6-diméthoxyphtali dyle, tert-butylcarbonylméthyle, allyle, 2-chloroallyle, méthoxycarbonylméthyle, benzyle ou tert-butyle.

On peut encore citer entre autres restes de groupements esters que peuvent représenter A et A', les groupements méthoxyéthoxyméthyle, diméthylaminoéthyle, cyanométhyle, tert-butoxycarbonylméthyle, 2,2-éthylènedioxyéthyle, cyanoéthyle, 2,2-diméthoxyéthyle ; 2-chloroéthoxyméthyle, 2-hydroxyéthoxyéthyle, 2,3-époxypropyle, 3-diméthylamino, 2-hydroxypropyle, 2-hydroxyéthyle, 2-méthylaminoéthoxyméthyle, 2-aminoéthoxyméthyle, 3-méthoxy 2,4-thiadia- zol-5-yle, 2-tétrahydropyrannyle, 1-méthoxy 1-méthyl éthyle, 2-hydroxy 1-méthyl éthyle, isopropyle ; carbamoylméthy- le, chlorométhyle, 2-chloroéthyle, acétylméthyle, 2-méthylthioéthyle ou thiocyanatométhyle.

On peut encore citer entre autres restes de groupements esters que peuvent représenter A et A', les groupements 2-chloro 1-acétyloxyéthyle, 2-bromo 1-acétyloxyéthyle, 2-fluoro 1-acétyloxyéthyle, 2-méthoxy 1-acétyloxyéthyle, 2-méthyl 1-acétyloxypropyle, 1-méthyl 1-acétyloxyéthyle, 1-méthoxyacétyloxyéthyle, 1-acétylcarbonyloxyéthyle, 1-hy- droxyacétyloxyéthyle, 1-formylcarbonyloxyéthyle, 1-(2-thiényl) carbonyloxyéthyle, 1-(2-furyl) carbonyloxyéthyle, 1-(5-nitro2-furyl) carbonyloxyéthyle, 1-(2-pyrrolyl) carbonyloxyéthyle, 1-(propionyloxycarbonyloxy) éthyle, 1-(propyloxy- carbonyloxy) éthyle, 1-(isopropyloxycarbonyloxy) éthyle, 1-(méthoxyéthoxycarbonyloxy) éthyle, 1-(allyloxycarbony- loxy) éthyle, isopropyloxycarbonyl méthyle, 1-[(2,3-époxy propyl) oxycarbonyloxy] éthyle, 1-[(2-furyl) méthyloxycarbo- nyloxy] éthyle, 1-(2-fluoro éthyl) oxycarbonyloxyéthyle, 1-(méthoxycarbonyloxy) propyle, 1-(méthoxycarbonyloxy) 1-méthyl éthyle, (méthoxycarbonyloxy) chlorométhyle, 1-(méthoxycarbonyloxy) 2-chloroéthyle, 1-(méthoxycarbonyloxy) 2-méthoxy éthyle, 1-(méthoxycarbonyloxy) allyle, ou un reste :

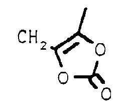

Les produits de formule (I) peuvent également se présenter sous forme de sels d'acides organiques ou minéraux.

Parmi les acides avec lesquels on peut salifier le ou les groupements amino des produits (1), on peut citer entre autres, les acides acétique, trifluoroacétique, maléïque, tartrique, méthanesulfonique, benzènesulfonique, para-toluène sulfonique, phosphorique, sulfurique, chlorhydrique, bromhydrique, iodhydrique.

Les produits peuvent également se présenter sous forme de sels internes.

Dans un mode préféré de l'invention, A' représente un atome d'hydrogène ou de sodium, de préférence hydrogène et C0₂A représente CO₂^{⊝}.

L'expression sous forme d'ammonium quaternaire indique que le radical R₁ est lié par le ou l'un des atomes d'azote qu'il comporte.

L'invention a particulièrement pour objet les produits de formule générale (I) telle que définie ci-dessus dans laquelle R₁ est choisi parmi les radicaux :

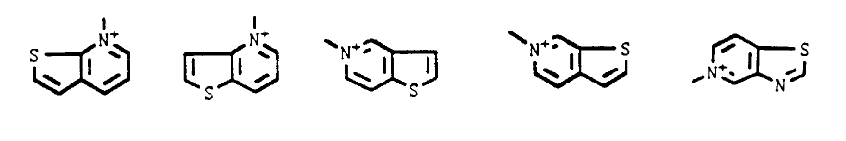

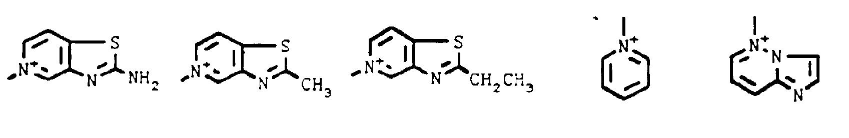

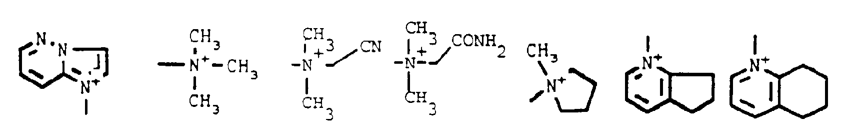

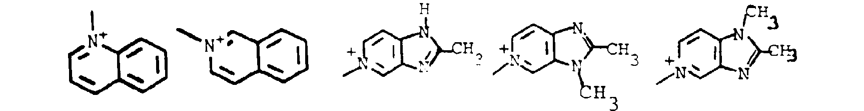

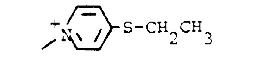

L'invention a plus particulièrement pour objet les produits de formule générale (I) telle que définie ci-dessus dans laquelle R₁ est choisi parmi les radicaux 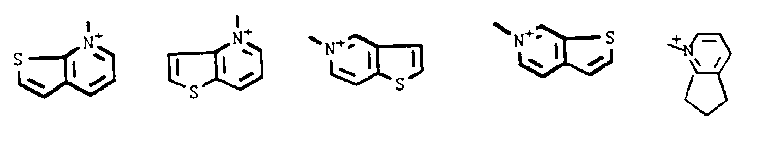 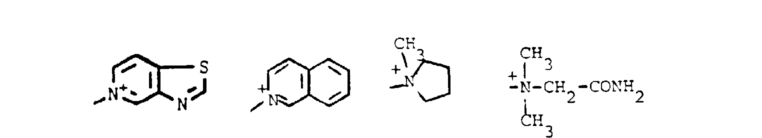 de préférence le radical :

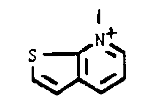

L'invention a tout particulièrement pour objet les produits décrits ci-après dans les exemples, à savoir :
le [6R-[3(E), 6alpha, 7béta(Z)]] 5-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,1 ]oct-2-en-3-yl] 2-propényl] thiazolo[4,5-c] pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, le [6R-[3(E), 6alpha, 7béta(Z)]] 7-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[2,3-b]pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables et particulièrement sous forme S,
le [6R-[3(E), 6alpha, 7béta(Z)]] 2-[3-[7-[[(2-amino 4-thiazolyl) [[1-(3,4-dihydrophényl) 2-hydroxy 2-oxoéthoxy] imino] acétyl] amino] 2-carboxy 8-oxo 5-thia 1-azanicyclo[4,2,0]oct-2-en-3-yl] 2-propényl] isoquinoléinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] 1-méthyl pyrrolidinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] 2-propényl] 6,7-dihydro 5H-pyrindinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] N-(2-amino 2-oxoéthyl) 3-[7-[[(2-amino4-thiazolyl) [[1-(3,4-dihydroxyphényl) 2-hydroxy 2-oxoéthoxy] imino] acétyl] amino] 2-carboxy 8-oxo 5-thia 1-azabicyclo-[4;2,0]oct-2-en-3-yl] N,N-diméthyl 2-propèn-1-aminium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables.

II est entendu que les produits de formule (I) précités peuvent exister :
soit sous la forme indiquée par ladite formule (1),
soit sous la forme de produits de formule (I)_{Z} : 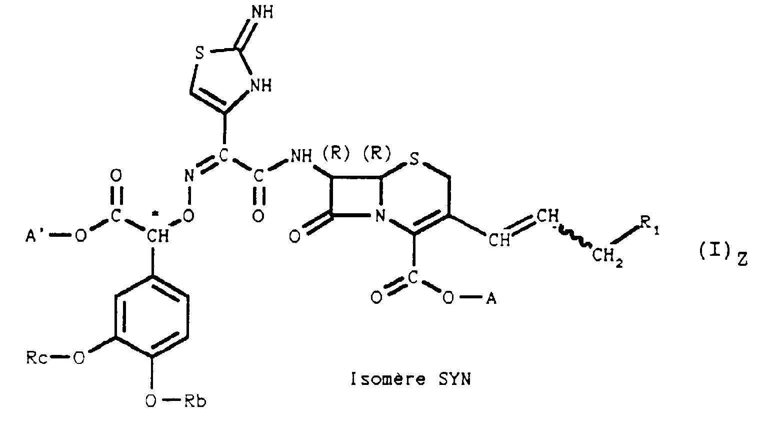 dans laquelle A, A', R₁, R_{b} et R_{c} ont la signification précédente.

L'invention a également pour objet un procédé de préparation des produits de formule (I) telle que définie ci-dessus, caractérisé en ce que l'on fait agir un produit de formule (II): 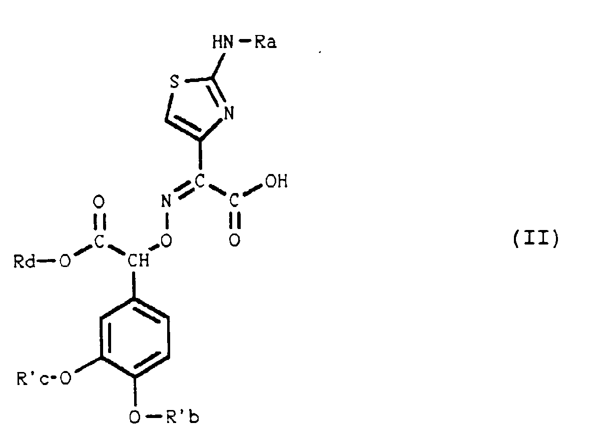 isomère syn, racémique ou optiquement actif ou un dérivé fonctionnel du produit de formule (II), dans laquelle Rₐ représente un atome d'hydrogène ou un groupement protecteur du radical amino, R'_{b} et R'_{c} identiques ou différents représentent un atome d'hydrogène ou un groupement protecteur du radical hydroxyle, R_{d} représente un atome d'hydrogène ou le reste d'un groupement ester facilement éliminable, avec un produit de formule (III) : 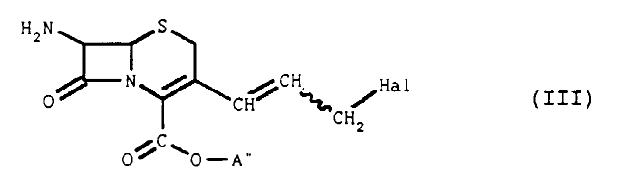 dans laquelle Hal représente un atome d'halogène, A" représente un atome d'hydrogène ou le reste d'un groupement ester facilement éliminable et le trait ondulé signifie que le groupement CH₂Hal peut se trouver dans la position E ou Z pour obtenir un produit de formule (IV) : 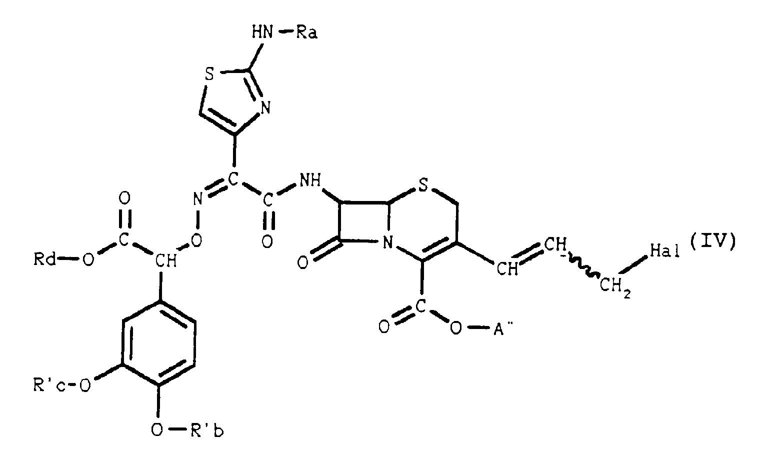 que l'on fait agir avec un réactif capable d'introduire le radical R₁ pour obtenir un produit de formule (V) : 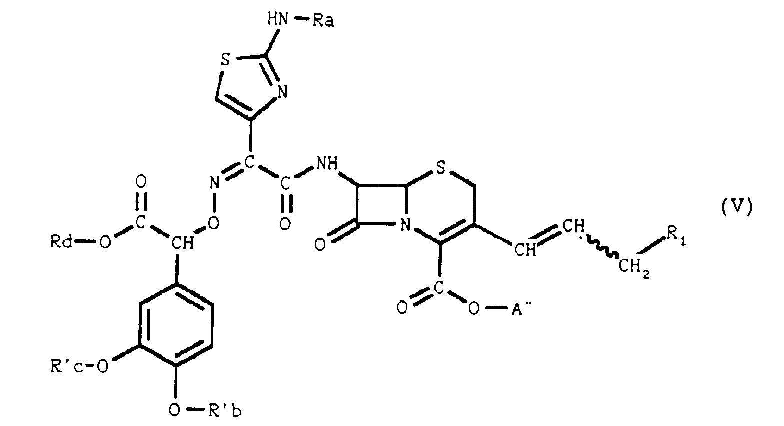 que, si désiré, l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque:
a) coupure par hydrolyse ou par action de lathiourée de tout ou partie des groupements esters ou des groupements de protection du radical amino ou des radicaux hydroxyles,
b) estérification ou salification par une base du ou des radicaux carboxyliques,
c) salification par un acide du radical amino,
d) séparation des produits sous forme de mélange R,S en R ou S.

Par réactif capable d'introduire le radical R₁, on entend un réactif constitué du radical R₁ lui-même, celui-ci n'étant pas sous forme d'ammonium quaternaire ; notamment si l'on veut introduire un radical pyridinium, on opérera avec la pyridine.

Parmi les réactifs préférés, on peut citer ceux répondant aux formules :

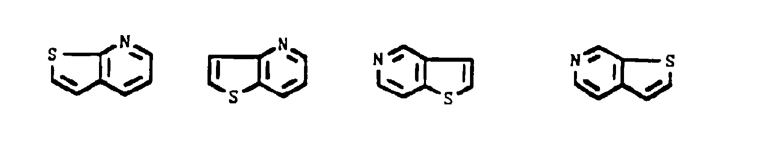

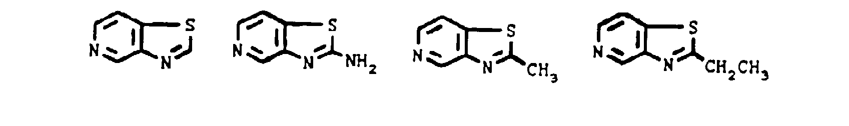

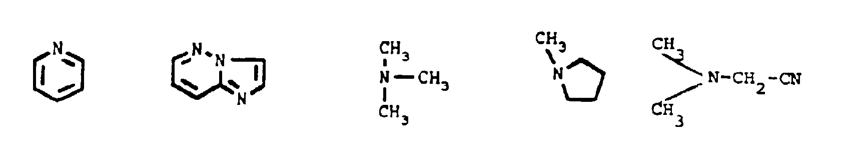

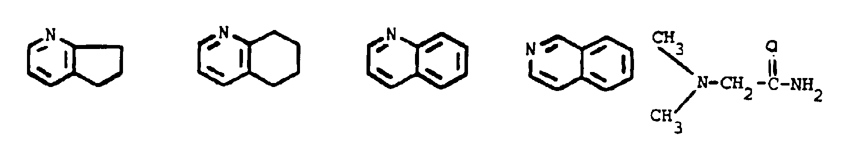

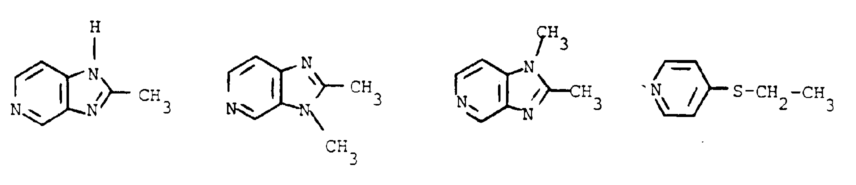

En plus des groupements cités ci-dessus, les groupements ester facilement éliminables que peuvent représenter A" et R_{d} peuvent être par exemple l'ester formé avec les radicaux butyle, isobutyle, tert-butyle, pentyle, hexyle, acétoxyméthyle, propionyloxyméthyle, butyryloxyméthyle, valéryloxyméthyle, pivaloyloxyméthyle, 2-acétoxyéthyle, 2-propionyloxyéthyle, 2-butyryloxyéthyle.

On peut également citer les radicaux 2-iodoéthyle, 2,2,2-trichloro éthyle, vinyle, allyle, éthynyle, propynyle, benzyle, 4-méthoxybenzyle, 4-nitrobenzyle, phényléthyle, trityle, diphénylméthyle, 3,4-diméthoxyphényle.

On peut également citer les radicaux phényle, 4-chloro phényle, tolyle, tert-butylphényle.

On préfère le radical diphénylméthyle pour R_{d} et 4-méthoxybenzyle ou diphénylméthyle pour A".

Le groupement protecteur du radical amino que peut représenter Rₐ peut être par exemple un radical alkyle de 1 à 6 atomes de carbone tel que, préférentiellement, tert-butyle ou tert-amyle.

Rₐ peut également représenter un groupement acyle aliphatique, aromatique ou hétérocyclique ou un groupe carbamoyle. On peut citer les groupements alcanoyles inférieurs tel que par exemple formyle, acétyle, propionyle, butyryle, isobutyryle, valéryle, isovaléryle, oxalyle, succinyle, pivaloyle.

Rₐ peut également représenter un groupe alkoxy ou cycloalkoxycarbonyle inférieur tel que par exemple, méthoxycarbonyle, éthoxycarbonyle, propoxycarbonyle, 1-cyclopropyléthoxycarbonyle, isopropyloxycarbonyle, butyloxycarbonyle, tert-butyloxycarbonyle, pentyloxycarbonyle, hexyloxycarbonyle, un groupe benzoyle, toluolyle, naphtoyle, phtaloyle, mésyle, phénylacétyle, phénylpropionyle, un groupe aralcoxycarbonyle, tel que benzyloxycarbonyle.

Les groupements acyles peuvent être substitués par exemple par un atome de chlore, de brome, d'iode ou de fluor. On peut citer les radicaux chloroacétyle, dichloroacétyle, trichloroacétyle, bromoacétyle ou trifluoroacétyle.

Rₐ peut également représenter un groupement aralkyle inférieur tel que benzyle, 4-méthoxybenzyle, phényléthyle, trityle, 3,4-diméthoxybenzyle ou benzhydryle.

Rₐ peut également représenter un groupe haloalkyle tel que trichloroéthyle.

Rₐ peut également représenter un groupement chlorobenzoyle, para-nitrobenzoyle, para-tert-butylbenzoyle, phénoxyacétyle, caprylyle, n-décanoyle, acryloyle, trichloroéthoxycarbonyle.

Rₐ peut également représenter un groupement méthyl carbamoyle, phénylcarbamoyle, naphtylcarbamoyle, ainsi que les thiocarbamoyles correspondants.

On préfère le groupement trityle.

La liste ci-dessus n'est pas limitative, il est évident que d'autres groupements protecteurs des amines, groupements connus en particulier dans la chimie des peptides, peuvent également être utilisés.

Le groupement de protection des radicaux hydroxyles que peuvent représenter R'_{b} et R'_{c}, peut être choisi dans la liste ci-dessous :
R'_{b} et R'ₑ peuvent représenter un groupe acyle tel que par exemple formyle, acétyle, propionyle, chloroacétyle, bromoacétyle, dichloro-acétyle, trichloroacétyle, trifluoroacétyle, méthoxyacétyle, phénoxyacétyle, benzoyle, benzoylfor- myle, p-nitrobenzoyle. On peut citer également les groupements éthoxy carbonyle, méthoxycarbonyle, propoxycarbonyle, 2,2,2-trichloro-éthoxycarbonyle, benzyloxycarbonyle, tert-butoxycarbonyle, 1-cyclopropyléthoxycarbonyle, tétrahydropyrannyle, tétrahydrothiopyrannyle, méthoxytétrahydropyrannyle, trityle, benzyle, 4-méthoxybenzyle, benzhydryle, trichloroéthyle, 1-méthyl 1-méthoxyéthyle, phtaloyle.

On peut également citer d'autres acyles tels que butyryle, isobutyryle, valéryle, isovaléryle, oxalyle, succinyle et pivaloyle.

On peut également citer les radicaux phénylacétyle, phénylpropionyle, mésyle, chlorobenzoyle, para-nitrobenzoyle, para-tert-butylbenzoyle, caprylyle, acryloyle, méthylcarbamoyle, phénylcarbamoyle, naphtylcarbamoyle.

On peut également citer les radicaux alkoxy alkoxy méthyle tel que méthoxy éthoxy méthyle.

Les radicaux OR'_{b} et OR'ₑ peuvent également former avec le radical phényle auquel ils sont liés, les valeurs suivantes :

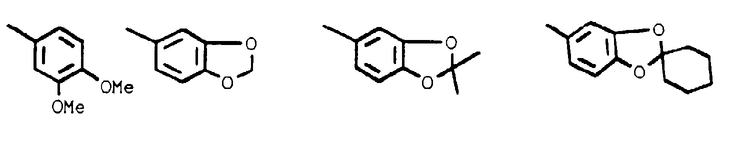

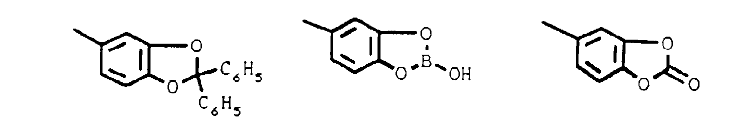

On préfère le groupement méthoxy éthoxy méthyle pour les substituants R'_{b} et R'_{b}.

Dans un mode d'exécution du procédé, on fait agir un dérivé fonctionnel du produit de formule (II). Ce dérivé fonctionnel peut être par exemple un halogénure, un anhydride symétrique ou mixte, l'amide, l'azide ou un ester activé.

Comme exemple d'anhydride mixte on peut citer par exemple celui formé avec le chloroformiate d'isobutyle et celui formé avec le chlorure de pivaloyle et les anhydrides mixtes carboxylique-sulfonique formé par exemple avec le chlorure de para-toluène sulfonyle.

Comme exemple d'ester activé, on peut mentionner l'ester formé avec le 2,4-dinitrophénol et celui formé avec l'hydroxybenzothiazole.

Comme exemple d'halogénure, on peut citer le chlorure ou le bromure.

L'anhydride peut être formé in situ par action de carbodiimide N,N'-disubstitué, par exemple le N,N-dicyclohexyl- carbodiimide.

La réaction d'acylation est conduite de préférence dans un solvant organique tel que le chlorure de méthylène. On peut cependant utiliser d'autres solvants tels que le tétrahydrofuranne, le chloroforme ou le diméthylformamide.

Lorsqu'on utilise un halogénure d'acide et de manière générale lorsqu'une molécule d'acide halohydrique est libérée au cours de la réaction, on réalise la réaction de préférence en présence d'une base telle que la soude, la potasse, les carbonates et carbonates acides de sodium ou de potassium, l'acétate de sodium, la triéthylamine, la pyridine, la morpholine ou la N-méthylmorpholine.

La température de réaction est en général inférieure ou égale à la température ambiante.

On peut également faire agir directement un produit de formule (II) avec un produit de formule (III) en présence d'un carbodiimide telle que le diisopropylcarbodiimide. Un exemple d'une telle préparation est donnée plus loin dans la partie expérimentale.

L'action des réactifs capable d'introduire le radical R₁ sur le produit de formule (IV) est effectuée dans les conditions suivantes :
Lorsque Hal représente par exemple un atome de chlore, on peut effectuer in situ ou séparément une substitution de l'atome de chlore par un atome d'iode en présence d'iodure de sodium puis ajoute ensuite le réactif désiré, en présence ou non d'un solvant organique tel que l'acétonitrile ou le tétrahydrofuranne. Des exemples de telles réactions sont décrits ci-après dans la partie expérimentale.

On peut également faire agir sur le produit de formule (IV) dans lequel Hal représente un atome de chlore, le réactif désiré en présence de tétrafluoroborate d'argent.

Un exemple d'une telle préparation se trouve également dans la partie expérimentale.

L'isomérie des produits de formule (V) peut être différente de celle des produits de formule (IV) utilisés au départ. Dans le cas où l'on isole l'isomère Z, on peut transformer cet isomère en isomère E selon les méthodes usuelles, notamment par action de l'iode.

Selon les valeurs de Rₐ, R'_{b}, R'_{c}, R_{d} et A", les produits de formule (V) peuvent ou non constituer des produits de formule (1).

Les produits de formule (V) constituent des produits de formule (1) lorsque Rₐ représente un atome d'hydrogène, lorsque R'_{b} et R'ₑ ne représentent pas un groupement protecteur du radical hydroxyle que l'on désire éliminer, c'est-à-dire lorsque R'_{b} et/ou R'_{c} représentent un radical acétyle, propionyle ou benzoyle et lorsque R_{d} et A" ne représentent pas, parmi les groupements esters facilement clivables, l'un de ceux que l'on désirerait éliminer

Dans les autres cas, l'action sur le produit de formule (V) d'un ou plusieurs agents d'hydrolyse, d'hydrogénolyse ou de la thiourée a pour but d'éliminer le radical Rₐ lorsque celui-ci représente un radical protecteur du radical amino, d'éliminer les radicaux R'_{b} et R'c lorsque ceux-ci représentent un groupement protecteur des radicaux hydroxyle et/ou d'éliminer les radicaux R_{d} et A" lorsque ceux-ci représentent, parmi les groupements esters facilement clivables l'un de ceux que l'on désire éliminer

Cependant, il est bien entendu possible d'éliminer Rₐ, R'_{b} et R'ₑ sans toucher aux substituants R_{I} et A" lorsque ceux-ci doivent être conservés. Il en est ainsi par exemple lorsque A" représente un groupement ester que l'on souhaite conserver tel qu'un groupement propionyloxyméthyle.

La nature des réactifs à mettre en jeu dans un tel cas est bien connu de l'homme de métier. Des exemples de telles réactions sont donnés plus loin dans la partie expérimentale.

On trouvera par exemple une description des différentes méthodes d'élimination des différents groupements pro- lecteurs dans la demande de brevet français B.F. 2.499.995.

Etant donné les groupements protecteurs préférés que l'on utilise : trityle pour R_{a;} méthoxy éthoxy méthyle pour R'_{b} et R'_{c}, diphénylméthyle pour R_{I} et 4-méthoxybenzyle ou diphénylméthyle pour A", on utilise de préférence l'acide trifluoroacétique sans solvant ou dans un solvant tel que l'anisole ou un mélange de solvants tels que anisole/chlorure de méthylène. On obtient alors un sel avec l'acide trifluoroacétique. On peut revenir à la base libre par action d'une base telle que le carbonate de triéthylamine.

La salification des produits peut être effectuée selon les méthodes usuelles.

La salification peut, par exemple, être obtenue par action, sur un produit sous forme acide ou sur un solvat, par exemple, le solvat éthanolique ou un hydrate de cet acide, d'une base minérale telle que l'hydroxyde de sodium ou de potassium, le carbonate ou le carbonate acide de sodium ou de potassium. On peut également utiliser les sels d'acides minéraux tels que le phosphate trisodique On peut également faire appel à des sels d'acides organiques.

Comme sels d'acides organiques, on peut mentionner, par exemple, les sels de sodium d'acides carboxyliques aliphatiques, linéaires ou ramifiés, saturés ou insaturés de 1 à 18 et de préférence de 2 à 10 atomes de carbone. Les chaînes aliphatiques de ces acides peuvent être interrompues par un ou plusieurs hétéroatomes tels que l'oxygène ou le soufre ou substituées par des radicaux aryle, comme, par exemple : phényle, thiényle, furyle, par un ou plusieurs radicaux hydroxyles ou par un ou plusieurs atomes d'halogène tels que fluor, chlore ou brome, préférentiellement chlore, par un ou plusieurs radicaux carboxyliques ou alkoxycarbonyles inférieurs, de préférence méthoxycarbonyle, éthoxycarbonyle ou propyloxycarbonyle, par un ou plusieurs radicaux aryloxy, de préférence phénoxy.

De plus, on peut utiliser comme acides organiques, des acides aromatiques suffisamment solubles comme, par exemple, des acides benzoïques substitués, de préférence par des radicaux alkyles inférieurs.

Comme exemples de tels acides organiques, on peut mentionner :
les acides formique, acétique, acrylique, butyrique, adipique, isobutyrique, n-caproïque, isocaproïque, chloropropio- niques, crotonique, phénylacétique, 2-thiénylacétique, 3-thiénylacétique, 4-éthylphénylacétique, glutarique, l'ester mo- noéthylique de l'acide adipique, les acides hexanoïque, heptanoïque, décanoïque, oléïque, stéarique, palmitique, 3-hy- droxypropionique, 3-méthoxypropionique, 3-méthylthiobutyrique, 4-chlorobutyrique, 4-phénylbutyrique, 3-phénoxybu- tyrique, 4-éthylbenzoïque, 1-propylbenzoïque.

On utilise cependant de préférence comme sels de sodium, l'acétate de sodium, le 2-éthyl hexanoate de sodium ou le diéthyl acétate de sodium.

La salification peut également être obtenue par action d'une base organique comme la triéthylamine, la diéthylamine, la triméthylamine, la propylamine, la N,N-diméthyl éthanolamine, le tris[(hydroxyméthyl) amino] méthane, la méthylamine, l'éthanolamine, la pyridine, la picoline, la dicyclohexyl amine, la morpholine et la benzylamine.

Elle peut également être obtenue par action de l'arginine, de la lysine, de la procaïne, de l'histidine, de la N-méthyl glucamine.

Cette salification est réalisée de préférence dans un solvant ou un mélange de solvants tels que l'eau, l'éther éthylique, le méthanol, l'éthanol ou l'acétone.

Les sels sont obtenus sous forme amorphe ou cristallisée selon les conditions réactionnelles employées.

Les sels cristallisés sont préparés de préférence en faisant réagir les acides libres avec l'un des sels des acides carboxyliques aliphatiques mentionnés ci-dessus, de préférence, avec l'acétate de sodium.

La salification des produits par les acides minéraux ou organiques est effectuée dans les conditions usuelles.

L'estérification éventuelle des produits est effectuée dans les conditions classiques. On opère en général en faisant réagir l'acide de formule (1) ou un dérivé fonctionnel avec un dérivé de formule : 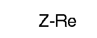 dans laquelle Z représente un radical hydroxyle ou un atome d'halogène tel que le chlore, le brome, l'iode et Re désigne le groupement ester à introduire, groupement dont une liste non exhaustive figure ci-dessus. Dans certains cas, il peut être avantageux d'effectuer une estérification sur un produit dont l'amine et/ou les groupements réactionnels présents sur l'oxyimino sont bloqués avant d'enlever le groupement protecteur de l'amine et du groupement réactionnel présents sur l'oxyimino.

Les produits de formule (1) comportent plusieurs carbones asymétriques. Dans le noyau cephème, qui comporte deux carbones asymétriques, les deux carbones sont dans la configuration R. Par ailleurs le radical présent sur la fonction oxyimino comporte également un carbone asymétrique : 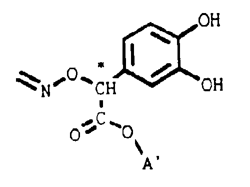 qui peut être sous forme R ou S ou sous forme d'un mélange R,S. La séparation des deux diastéréoisomères peut être effectuée par les moyens connus de l'homme du métier, par exemple par chromatographie

Les produits de formule générale (1) possèdent une très bonne activité antibiotique sur les bactéries gram (+) telles que les staphylocoques, les streptocoques et notamment sur les staphylocoques pénicillino-résistants. Leur efficacité sur les bactéries gram (-) notamment sur les bactéries coliformes, les klebsiella, les salmonella, les proteus et les pseudomonas, est particulièrement remarquable.

Ces propriétés rendent aptes lesdits produits ainsi que leurs sels d'acides pharmaceutiquement acceptables à être utilisés comme médicaments dans le traitement des affections à germes sensibles et notamment dans celui des staphylococcies, telles que septicémies à staphylocoques, staphylococcies malignes de la face ou cutanée, pyodermites, plaies septiques ou suppurantes, anthrax, phlegmons, érésipèles, staphylococcies aiguës primitives ou post grippales, broncho-pneumonies, suppurations pulmonaires.

Ces produits peuvent également être utilisés comme médicaments dans le traitement des colibacilloses et infections associées, dans les infections à proteus, à klebsiella et à salmonella et dans d'autres affections provoquées par des bactéries à gram (-).

La présente invention a donc également pour objet, à titre de médicaments et notamment de médicaments antibiotiques, les produits de formule (1) tels que définis ci-dessus ainsi que leurs sels d'acides pharmaceutiquement acceptables.

L'invention a plus particulièrement pour objet à titre de médicaments les produits de formule (1) telle que décrite ci-dessus dans laquelle Rᵢ est choisi parmi les radicaux : 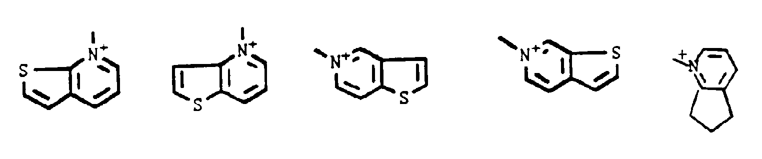 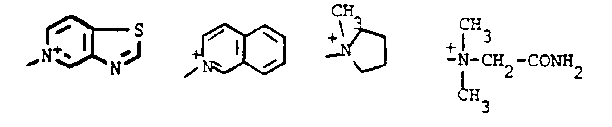 de préférence le radical :

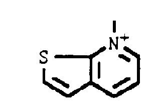

L'invention a spécialement pour objet, à titre de médicaments et notamment de médicaments antibiotiques, les produits décrits ci-après dans les exemples, à savoir :
le [6R-[3(E), 6alpha, 7béta(Z)]] 5-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] 2-propényl] thiazolo[4,5-c]pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, pharmaceutiquement acceptables,
le [6R-[3(E), 6alpha, 7béta(Z)]] 7-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] 2-propényl] thiéno[2,3-b]pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables et particulièrement sous forme S, pharmaceutiquement acceptables,
le [6R-[3(E), 6alpha, 7béta(Z)]] 2-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] 2-propényl] isoquinoléinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, pharmaceutiquement acceptables,
le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] 2-propényl] 1-méthyl pyrrolidinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, pharmaceutiquement acceptables,
le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] 2-propényl] 6,7-dihydro 5H-pyrindinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, pharmaceutiquement acceptables,
le [6R-[3(E), 6alpha, 7béta(Z)]] N-(2-amino 2-oxoéthyl) 3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,O]oct-2-en-3-yl] N,N-diméthyl 2-propèn-1-aminium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables, pharmaceutiquement acceptables.

L'invention s'étend aux compositions pharmaceutiques renfermant, comme principe actif, au moins un des médicaments définis ci-dessus

Ces compositions peuvent être administrées par voie buccale, rectale, parentérale, notamment intramusculaire ou par voie locale en application topique sur la peau et les muqueuses.

Les produits de formule (I) et notamment ceux dans laquelle A représente un ester clivable peuvent être administrés par voie orale.

Les compositions selon l'invention peuvent être solides ou liquides et se présenter sous les formes pharmaceutiques couramment utilisées en médecine humaine comme par exemple, les comprimés simples ou dragéifiés, les gélules, les granulés, les suppositoires, les préparations injectables, les pommades, les crèmes, les gels ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Ces compositions peuvent notamment se présenter sous forme d'une poudre destinée à être dissoute extemporanément dans un véhicule approprié, par exemple, de l'eau stérile apyrogène.

La dose administrée est variable selon l'affection traitée, le sujet en cause, la voie d'administration et le produit considéré Elle peut être, par exemple, comprise entre 0,250 g et 4 g par jour, par voie orale chez l'homme, avec le produit décrit à l'exemple 1 ou encore comprise entre 0,500 g et 1 g trois fois par jour par voie intramusculaire.

Les produits de formule (I) peuvent également être utilisés comme désinfectants des instruments chirurgicaux.

L'invention a enfin pour objet, à titre de produits industriels nouveaux et notamment à titre de produits intermédiaires nécessaires à la préparation des produits de formule (I) telle que définie ci-dessus, les produits de formule (IV) et les produits de formule (V) dans laquelle Rₐ représente un groupement protecteur du radical amino, les formules (IV) et (V) étant telles que définies ci-dessus.

Les produits de formule (II) sont connus dans la littérature, notamment dans les demandes de brevets européens EP 0.238.061 ou EP 0.266.060 ou peuvent être préparés selon les méthodes usuelles ;

Les produits de formule (III) sont également connus dans la littérature, notamment dans les demandes de brevets britanniques GB 2.134.522 ou allemandes DE 3512225.

Les exemples suivants illustrent l'invention sous toutefois la limiter.

### EXEMPLE 1 : [6R-[3(E), 6alpha, 7béta(Z)]] 7-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[1,2,0]oct-2-en-3-yl] 2-propényl]thiéno [2,3-b]pyridinium trifluoroacétate tétrafluoroborate

### STADE A : [6R-[3(E), 6alpha, 7béta(Z)]] diphénylméthyl 7-[[[[1-[3,4-bis[(2-méthoxy éthoxy) méthoxy] phényl] 2-(diphé- nylméthoxy) 2-oxoéthoxy] imino] [2-[(triphénylméthyl) amino] 4-thiazolyl] acétyl] amino] 3-(3-chloro 1-propényl) 8-oxo 5-thia 1-azabicyclo[4,2,0]oct-2-ène-2-carboxylate.

On ajoute 0,372 cm³ de disopropylcarbodiimide dans 1 cm³ de chlorure de méthylène à un mélange de 1,876 g d'acide [[[3,4-bis[(2-méthoxy éthoxy) méthoxy] phényl] (diphényl méthoxy carbonyl) méthoxy] imino] [2-(triphényl méthyl amino) 4-thiazolyl] acétique isomère syn, décrit dans le brevet européen EP 238061, 0,955 g de diphénylméthyl 7-amino 3-(3-chloro 1-propényl) 8-oxo 5-thia 1-azabicyclo[4,2,0]oct-2-ène-2-carboxylate décrit dans le brevet allemand DE 35 12 225 et 200 cm³ de chlorure de méthylène séché.

On agite 45 minutes puis évapore le solvant sous pression réduite et chromatographie sur silice (éluant : chlorure de méthylène 87,5 - acétate d'éthyle 12,5).

On obtient 2,1 g de produit jaune (Rf = 0,42 en chromatographie sur couche mince, éluant : chlorure de méthylène-acétate d'éthyle (8-2)).

### Infra-rouge

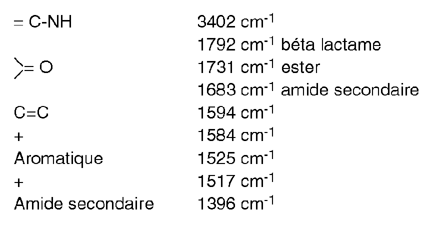

Ultra-violet
1) Dans EtOH + 1 cm³ CHCl₂ 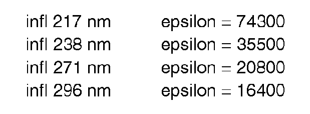
2) Dans EtOH + HCI 0,1N 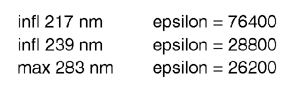
infl 271, 291 et 305 nm.

### STADE B : [6R-[3(E), 6alpha, 7béta(Z)]] 7-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(diphénylméthoxy) carbonyl] 8-oxo 5-thia 1-azabicyclo[4,2,0]oct-2-en-3-yl] 2-propényl] thiéno[2,3-b]pyridinium tétrafluoroborate

On traite aux ultrasons un mélange de 55,9 mg de tétrafluoroborate d'argent, 38,8 mg de thiéno[2,3-b]pyridine et 5 cm³ de chlorure de méthylène puis ajoute 136 mg de produit obtenu au stade A légèrement dilué dans le chlorure de méthylène et agite pendant 1 h 15.

On filtre, évapore, reprend le résidu par de l'éther, on obtient un solide que l'on lave par 3 fois 3 cm³ d'éther et obtient 207 mg de produit que l'on purifie par chromatographie sur silice (éluant : chlorure de méthylène-méthanol). On évapore les fractions et obtient 62 mg de produit. Rf = 0,28 en chromatographie sur couche mince (éluant : chlorure de méthylène-méthanol (9:1)).

### Ultra-violet

1) Dans EtOH 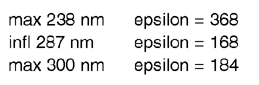
2) Dans EtOH, HCI 0,1 N

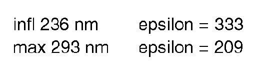

### STADE C : [6R-[3(E), 6alpha, 7béta(Z)]] 7-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[2,3-b]pyridinium trifluoroacétate tétrafluoroborate

On mélange à 0°C deux solutions :
a) 0,180 g de produit obtenu au stade B, 4,3 cm³ de chlorure de méthylène et 0,86 cm³ d'anisole,
b) 8,6 cm³ d'acide trifluoroacétique et 4,3 cm³ de chlorure de méthylène, et agite pendant une heure à 0°C.

On évapore, reprend à l'éther le produit obtenu qui se concrète On filtre, lave à l'éther et obtient 100,6 mg de produit que l'on place dans 3,3 cm³ de solution d'acide trifluoroacétique à 10 % d'anisole.

On agite une heure à 0°C, évapore puis précipite le produit à l'éther. On filtre, rince et obtient 87,9 mg de produit attendu.

### EXEMPLE 2 : [6R-[3(E), 6alpha, 7béta(Z)]] 7-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[1,2,0]oct-2-en-3-yl] 2-propényl] thiéno [2,3-b]pyridinium sous forme de sel interne

On élue sur colonne de silice RP 18 par un mélange CH₃CN-H₂0 (50-50) un mélange de 89,4 mg de produit obtenu, comme à l'exemple 1, 2,84 cm³ d'acétonitrile et 2,84 cm³ de solution de carbonate de triéthylamine à 0,1 N.

On lyophilise les fractions intéressantes et obtient 50,8 mg de produit attendu.

### Infra-rouge (Nujol)

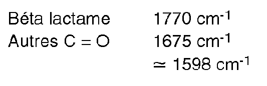

### Ultra-violet, dans EtOH, HCI 0,1 N

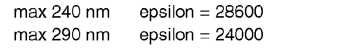

### EXEMPLE 3 : [6R-[3(E), 6alpha, 7béta(Z)]] 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo-[4,2,0]oct-2-en-3-yl] 2-propényl] thiazolo[4,5-c]-c]pyridinium trifluoroacétate iodure

### STADE A : [6R-[3(E), 6alpha, 7béta(Z)]] diphénylméthyl 7-[[[1-[3,4-bis[(2-méthoxy éthoxy) méthoxy] phényl] 2-(diphé- nylméthoxy) 2-oxoéthoxy] imino] [2-[(triphénylméthyl) amino] 4-thiazolyl] acétyl] amino] 3-(3-iodo 1-propényl) 8-oxo 5-thia 1-azabicyclo[4,2,0]oct-2-ène-2-carboxylate.

On agite pendant 2 heures à température ambiante un mélange de 650 mg de produit obtenu au stade A de l'exemple 1, 19,1 cm³ d'acétone et 216,3 mg d'iodure de sodium, évapore le solvant puis reprend le résidu par 26,5 cm³ d'acétate d'éthyle.

On lave la solution par 3 fois 15 cm³ de thiosulfate de sodium puis par 2 fois 15 cm³ d'eau.

On sèche sur sulfate de magnésium, filtre et rince, évapore, reprend par un mélange chlorure de méthylène-acétate d'éthyle (7-3), ajoute 5,3 g de silice, agite pendant 5 minutes puis filtre et rince

On obtient 445 mg de produit après évaporation (Rf = 0,54 sur chromatographie sur couche mince, éluant chlorure de méthylène-acétate d'éthyle (7-3).

### RMN dans CDCl₃

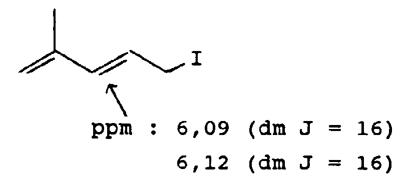

### STADEB: [6R-[3(E),6alpha,7béta(Z)]]5-[3-[7-[[[1-[3,4-bis[(2-méthoxyéthoxy)méthoxy]phényl]2-[(diphénylméthoxy) 2-oxoéthoxy] imino] [2-[(triphénylméthyl) amino] 4-thiazolyl] acétyl] amino] 2-[(diphénylméthoxy) carbonyl] 8-oxo 5-thia 1-azabicyclo[4,2,0]oct-2-en-3-yl] 2-propényl] thiazolo[4,5-c]pyridinium iodure

On agite pendant 5 heures un mélange de 445,2 mg de produit obtenu au stade A dans la plus petite quantité possible de diméthylsulfoxyde et 48,2 mg de thiazolo[4,5-c]pyridinepyridine puis élimine le solvant sous pression réduite

On lave le résidu visqueux par 3 fois 7 cm³ d'éther. On obtient 374,6 mg d'un solide que l'on purifie sur silice (éluant : chlorure de méthylène-méthanol (92-8)).

On obtient 24 mg de produit ayant l'isomère Z, 21,2 mg de mélange E+Z et 154,3 mg de produit ayant l'isomère E (Rf = 0,₁8 sur chromatographie sur couche mince, éluant : chlorure de méthylène-méthanol (9-1)). RMN (CDCl₃)

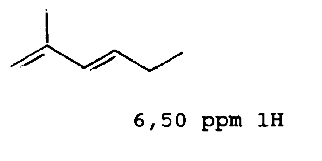

### STADE C : [6R-[3(E), 6alpha, 7béta(Z)]] 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiazolo[4,5-c] pyridinium trifluoroacétate iodure

On mélange à 0°C et agite pendant une heure les deux solutions suivantes :
a) 238,6 mg de produit obtenu au stade B, 5,7 cm³ de chlorure de méthylène et 1,14 cm³ d'anisole et
b) 11,4 cm³ d'acide trifluoroacétique dans 5,7 cm³ de chlorure de méthylène.

On évapore les solvants puis précipite le produit à l'éther. On filtre, lave et obtient 0,124 g de produit que l'on mélange à 4,14 cm³ d'acide trifluoroacétique et 0,46 cm³ d'anisole. On agite 40 minutes en maintenant la température à 0°C On évapore puis précipite le produit avec de l'éther. On filtre puis rince à l'éther. On sèche et obtient 95,8 mg de produit attendu.

### EXEMPLE 4 : [6R-[3(E), 6alpha, 7béta(Z)]] 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo-[4,2,0]oct-2-en-3-yl] 2-propényl] thiazolo[4,5-c]pyridinium sous forme de sel interne

On passe sur colonne de silice RP18, une solution de 95 mg de produit obtenu à l'exemple 3, 3,6 cm³ d'acétonitrile et 3,8 cm³ de carbonate de triéthylamine. On élue la colonne par un mélange acétonitrile-eau (50-50). On lyophilise les fractions intéressantes et obtient 63,8 mg de produit attendu.

### Ultra-violet, dans EtOH, HCI 0,1N

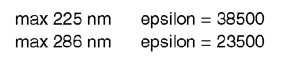

infl 274, 300 et 356 nm
Infra-rouge (Nujol)

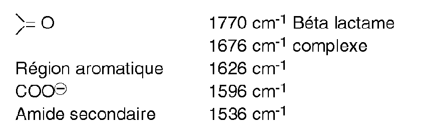

### EXEMPLE 5 : [6R-[3(E) 6alpha, 7béta (Z)]] 4-[3-[[[(2-amino 4-thiazolyl)[[1-(3,4-dihydroxyphényl)2-hydroxy 2-oxoéthoxy] imino] acétyl] amino] 2-carboxy 8-oxo 5-thia 1-azabicyclo [4,2,0] oct-2-èn-3-yl] 2(E)-propényl] thiéno[3,2-b]pyridinium trifluoroacétate tétrafluoroborate.

### STADE A : [6R-[3(E) 6alpha, 7béta (Z)]] 4-[3-[7-[[[3,4-bis [(2-méthoxyéthoxy) méthoxy] phényl] 2-[(diphényl méthoxy) 2-oxoéthoxy] imino] [2-(triphénylméthyl) amino] 4-thiazolyl] acétyl] amino] 2-[(diphénylméthoxy) carbonyl 8-oxo 5-thia 1-azabicyclo [4,2,0] oct-2-èn-3-yl] 2(E)-propényl] thiéno [3,2-b]pyridinium tétrafluoroborate.

On opère comme au stade B de l'exemple 1 en utilisant au départ 1,2 g de produit préparé comme indiqué au stade A de l'exemple 1, 346 mg de fluoroborate d'argent dans 44 cm³ de chlorure de méthylène et 0,24 cm³ de thiéno [3,2-b]pyridine et obtient après chromatographie sur silice (éluant : chlorure de méthylène-méthanol 92-8 puis 96-4) 337 mg de produit attendu.
RMN (CDCl₃ 300 Hz)

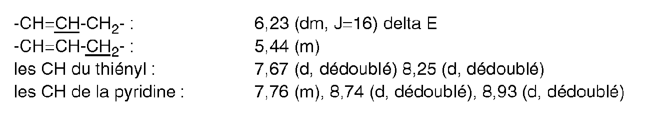

### STADE B : [6R-[3(E) 6alpha, 7béta (Z)]] 4-[3-[[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo [4,2,0]oct-2-èn-3-yl] 2(E)-propényl] thiéno[3,2-b]pyridinium trifluoroacétate tétrafluoroborate.

On opère comme au stade C de l'exemple 1 en utilisant au départ 316,1 mg de produit obtenu ci-dessus au stade A, 1,51 cm³ d'anisole dans 7,5 cm³ de chlorure de méthylène et 13,7 cm³ d'acide trofluoroacétique dans 7,5 cm³ de chlorure de méthylène. On obtient 183 mg de produit auquel on ajoute de nouveau 6,3 cm³ d'acide trifluoroacétique à 10% d'anisole et poursuit l'agitation à 0°C pendant 1 heure. On évapore le solvant, reprend le résidu à l'éther, filtre le précipité, le lave à l'éther et le sèche sous pression réduite. On recueille 124,1 mg de produit attendu.

### RMN (DMSO)

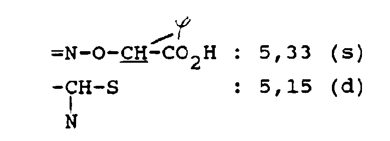

S-CH₂- : 3,49 (m) partiellement masqué
-CH=CH-CH₂ : 6,33 (dt, J=5 et 8) delta E 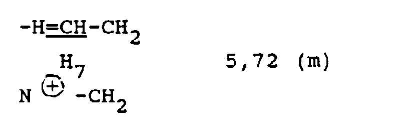 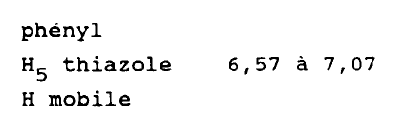
H₆', H₃'; H₂', H₇', H₅' du thiénopyridine : 8,04 à 9,36

### EXEMPLE 6 : [6R-[3(E) 6alpha, 7béta (Z)]] 4-[3-[[[(2-amino 4-thiazolyl) [[1·(3,4dihydroxyphenyl) 2-hydroxy 2-oxoéthoxy] imino] acétyl] amino] 2-carboxy 8-oxo 5-thia 1-azabicyclo[4,2,0]oct-2-èn-3-yl] 2(E)-propényl] thiéno[3,2-b]pyridinium trifluoroacétate tétrafluoroacétate.

### STADE A : [6R-[3(E), 6alpha, 7béta(Z)]] diphénylméthyl 7-[[[1-[3,4-bis[(2-méthoxy éthoxy) méthoxy] phényl] 2-(diphé- nylméthoxy) 2-oxoéthoxy] imino] [2-[(triphénylméthyl) amino] 4-thiazolyl] acétyl] amino] 3-(3-iodo 1-propényl) 8-oxo 5-thia 1-azabicyclo[4,2,0]oct-2-ène-2-carboxylate.

On opère comme au stade A de l'exemple 3 en utilisant 3 g de produit chloré obtenu au stade A de l'exemple 1 dans 100 cm³ d'acétone et 1,0 g d'iodure de sodium. On obtient 3,3 g de dérivé iodé identique à celui obtenu à l'exemple 3 utilisé tel quel pour le stade suivant.

### STADE B : [6R-[3(E), 6alpha, 7béta(Z)]] 4-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(diphénylméthoxy) carbonyl] 8-oxo 5-thia 1-azabicyclo[4,2,0]oct-2-en-3-yl] 2-propényl] thieno [3,2-b]pyridinium iodure.

On opère comme au stade B de l'exemple 3 en utilisant 3,3 g du dérivé iodé préparé au stade A, 1,5 cm³ de thiéno [2,3-b]pyridine et en remplaçant le diméthylsulfoxyde par du chlorure de méthylène. On obtient 1,08 g de produit attendu.

### RMN

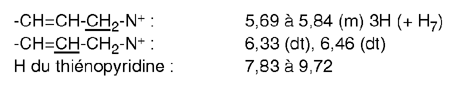

### STADE C : [6R-[3(E) 6alpha, 7béta (Z)]] 4-[3-[[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo [4,2,0]oct-2-èn-3-yl] 2(E)-propényl] thiéno[3,2-b]pyridinium trifluoroacétate tétrafluoroacétate.

On mélange à 0°C et agite pendant une heure les 2 solutions suivantes :
a) 55 cm³ d'acide trifluoroacétique, 5,5 cm³ d'anisole et 25 cm³ de chlorure de méthylène,
b) 1,19 g de produit obtenu comme au stade B dans 20 cm³ de chlorure de méthylène et poursuit la synthèse comme indiqué au stade C de l'exemple 3. On obtient 0,62 g de produit attendu.
   RMN (DMSO 400 Hz) 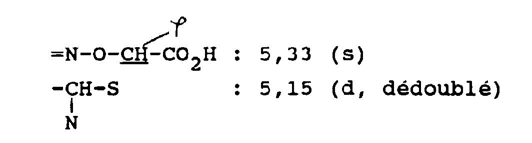
   S-CH₂- : 3,49 (m) partiellement masqué
   -CH=CH-CH₂ : 6,33 (dt, J=₁6 et 8) delta E 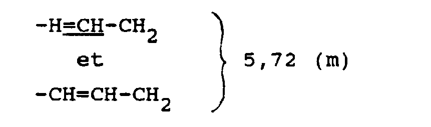 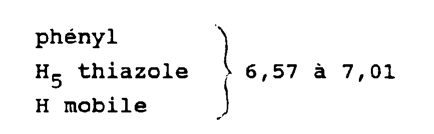
   H du thiénopyridine : 8,04 à 9,36

### EXEMPLE 7 : [6R-[3(E), 6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] pyridinium trifluoroacétate iodhydrate.

### STADE A : [6R-[3(E), 6alpha, 7béta(Z)]] 1-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(diphénylméthoxy) carbonyl] 8-oxo 5-thia 1-azabicyclo[4,2,0]oct-2-en-3-yl] 2-propényl] pyridinium iodure.

On opère comme au stade B de l'exemple 6 au départ de 1,47 g de dérivé iodé obtenu comme indiqué au stade A de l'exemple 3 et 480 microlitres de pyridine. On obtient 0,640 g de produit attendu.

### RMN (CDCl₃ 400 MHz)

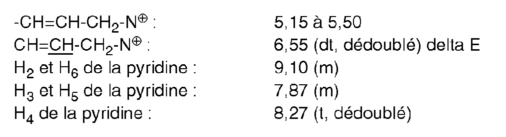

### STADE B : [6R-[3(E), 6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo-[4,2,0]oct-2-en-3-yl] 2-propényl] pyridinium trifluoroacétate iodhydrate.

On opère comme au stade C de l'exemple 6 en utilisant 0,638 g de dérivé obtenu au stade A précédent et obtient 0,314 g de produit attendu.

### RMN

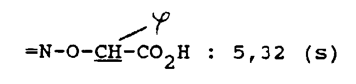

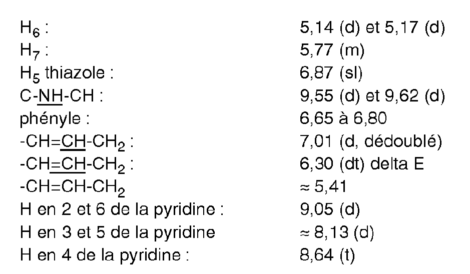

### EXEMPLE 8 : [6R-[3(E), 6alpha, 7béta(Z)]] 6-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo-[4,2,0]oct-2-en-3-yl] 2-propényl] thiéno [2,3-c]pyridinium trifluoroacétate iodhydrate.

### STADE A : [6R-[3(E), 6alpha, 7béta(Z)]] 6-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(diphénylméthoxy) carbonyl] 8-oxo 5-thia 1-azabicyclo[4,2,0]oct-2-en-3-yl] 2-propényl] thiéno[2,3-c]pyridinium iodure.

On opère comme au stade B de l'exemple 6 au départ de 2,08 g de dérivé iodé préparé comme indiqué au stade A de l'exemple 3 et 1 g de thiéno[2,3-c]pyridine. On obtient 0,98 g de produit attendu

### RMN

### 1) Dans EtOH :

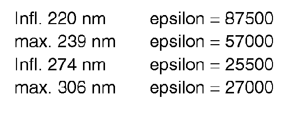

### 1) Dans EtOH/HCI 0,1N:

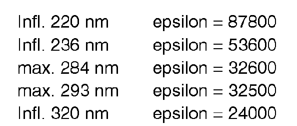

### STADE B : [6R-[3(E), 6alpha, 7béta(Z)]] 6-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[2,3-c]pyridinium trifluoroacétate iodhydrate.

On opère comme au stade C de l'exemple 6 en utilisant 0,966 g de dérivé iodé obtenu au stade A précédent et obtient 0,487 g de produit attendu.

### RMN

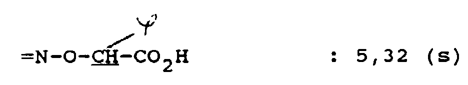

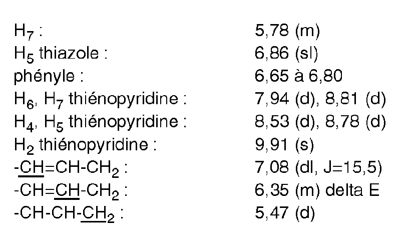

### EXEMPLE 9 : [6R-[3(E), 6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl]6,7-dihydro 5H-pyrindinium trifluoroacétate iodure.

### STADE A : [6R-[3(E), 6alpha, 7béta(Z)]] 5-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(diphénylméthoxy) carbonyl] 8-oxo 5-thia 1-azabicyclo[4,2,0]oct-2-en-3-yl] 2-propényl] 6,7-dihydro 5H-pyridinium iodure

On opère comme au stade B de l'exemple 6 au départ de 1,33 g de dérivé iodé préparé comme au stade A de l'exemple 3 et 0,585 cm³ de cyclopentyl pyridine. On obtient 1,07 g de produit attendu.

### STADE B : [6R-[3(E), 6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] 6,7-dihydro 5H-pyridinium trifluoroacétate iodure.

On opère comme au stade C de l'exemple 6 en utilisant 1,053 g du produit obtenu au stade A et obtient le produit attendu.

### RMN (DMSO 300 MHz)

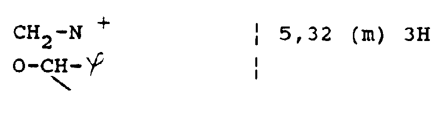

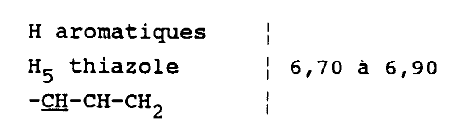

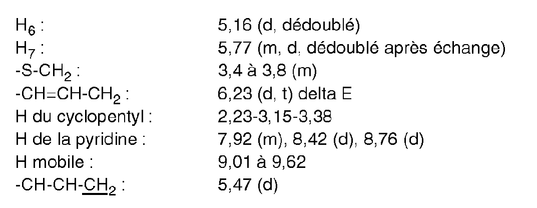

### EXEMPLE 10: [6R-[3(E), 6alpha, 7béta(Z)]] 2-amino 5-[3-[7-[[(2-amino4-thiazolyl) [[1-(3,4-dihydroxy phényl) 2-hydroxy 2-oxoéthoxy] imino] acétyl] amino] 2-carboxy 8-oxo 5-thia 1-azabicyclo-[4,2,0]oct-2-en-3-yl] 2-propényl] thiazolo [4,5-c]pyridinium trifluoroacétate iodure

### STADE A : [6R-[3(E), 6alpha, 7béta(Z)]] 2-amino 5-[3-[7-[[[1-[3,4-bis[(2-méthoxy éthoxy) méthoxy] phényl] 2-[(diphé- nylméthoxy) 2-oxoéthoxy] imino] [2-[(triphénylméthyl) amino] 4-thiazolyl] acétyl] amino] 2-[(diphénylméthoxy) carbonyl] 8-oxo 5-thia 1-azabicyclo [4,2,0] oct-2-en-3-yl] 2-propényl] thiazolo[4,5-c]pyridinium iodure.

On opère comme au stade B de l'exemple 6 au départ du dérivé iodé préparé comme au stade A de l'exemple 3 (à partir de 272 mg de dérivé chloré et 90 mg d'iodure de sodium) et de 30 mg d'amino thiazolo pyridine. On obtient 42 mg de produit attendu.

### STADE B : [6R-[3(E), 6alpha, 7béta(Z)]] 2-amino 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiazolo[4,5-c] pyridinium trifluoroacétate iodure.

On opère comme au stade C de l'exemple 3 en utilisant 130 mg du produit obtenu comme au stade A et obtient 11,5 mg de produit attendu.

### RMN (DMSO 400 MHz)

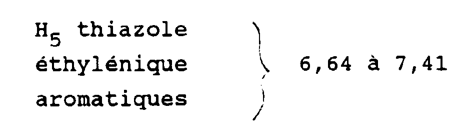

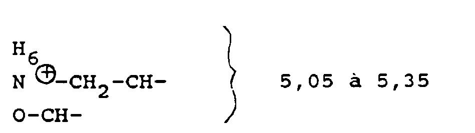

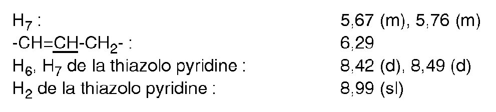

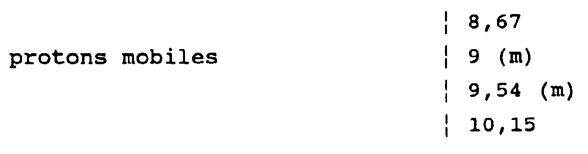

### EXEMPLE 11: [6R-[3(E), 6alpha, 7béta(Z)]] 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno [3,2-c] -c]pyridinium trifluoroacétate iodhydrate.

### STADE A : [6R-[3(E), 6alpha, 7béta(Z)]] p-méthoxybenzyl 7-[[[1-[3,4-bis[(2-méthoxy éthoxy) méthoxy] phényl] 2-(di- phénylméthoxy) 2-oxoéthoxy] imino] [2-[(triphénylméthyl) amino] 4-thiazolyl] acétyl] amino] 3-(3-chloro 1-propényl) 8-oxo 5-thia 1-azabicyclo[4,2,0]oct-2-èn-3-yl 2-carboxylate.

On refroidit à 0°C une suspension comprenant 3,75 g d'acide [[(3,4-bis [(2-méthoxy éthoxy) méthoxy] phényl] 2-(diphénylméthoxy) 2-oxoéthoxy] imino] [2-[(triphénylméthyl) amino] 4-thiazolyl] acétique isomère syn, décrit dans le brevet européen EP 238061 et 1,81 g de méthoxybenzyl 7-amino 3-(3-chloropropényl) 8-oxo 5-thia 1-azabicyclo[4,2,0] oct-2-èn-2-carboxylate (préparé comme indiqué dans le brevet européen EP 0 333 154 dans du chlorure de méthylène, et ajoute 0,920 g de chlorhydrate de N-(diméthylaminopropyl) N'-éthyl carbodiimide. On maintient la solution obtenue à 0°C sous agitation pendant 30 minutes. On lave la phase organique avec une solution aqueuse de chlorure de sodium, sèche et élimine les solvants. Après chromatographie du résidu sur silice (éluant : chlorure de méthylène- éther 85-15) et concrétion dans l'éther isopropylique, on obtient 4,546 g de produit attendu.

### RMN (CDCl₃ 400 MHz)

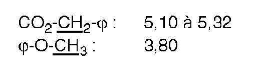

### STADE B : [6R-[3(E), 6alpha, 7béta(Z)]] p-méthoxybenzyl 7-[[[1-[3,4-bis[(2-méthoxy éthoxy) méthoxy] phényl] 2-(di- phénylméthoxy) 2-oxoéthoxy] imino] [2-[(triphénylméthyl) amino] 4-thiazolyl] acétyl] amino] 3-(3-iodo 1-propényl) 8-oxo 5-thia 1-azabicyclo[4,2,0]oct-2-èn-3-yl 2-carboxylate.

On agite pendant 1 heure à température ambiante un mélange de produit obtenu au stade A, 10 cm³ d'acétone et 341 mg d'iodure de sodium et environ 10 mg d'iode; évapore le solvant puis reprend le résidu par 80 cm³ de chlorure de méthylène. On lave la phase organique avec une solution aqueuse de thiosulfate de sodium puis à l'eau. On sèche, élimine les solvants, chromatographie sur silice (éluant : chlorure de méthylène-acétate d'éthyle 8-2) et obtient 853 mg de produit attendu.

### RMN (CDCl₃ 300MHz)

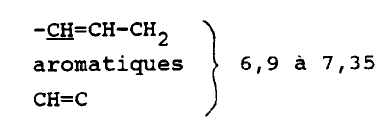

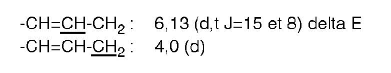

### STADE C : [6R-[3(E), 6alpha, 7béta(Z)]] 5-[3-[7-[[[1-[3,4-bis[(2-méthoxy éthoxy) méthoxy] phényl] 2-[(diphénylmé- thoxy) 2-oxoéthoxy] imino] [2-[(triphénylméthyl) amino] 4-thiazolyl] acétyl] amino] 2-[(paraméthoxy benzyloxy) carbonyl] 8-oxo 5-thia 1-azabicyclo[4,2,0]oct-2-en-3-yl] 2-propényl] thiéno [3,2-c]pyridinium iodure.

On dissout 2,48 g de dérivé iodé dans 10 cm³ de chlorure de méthylène et ajoute 1,2 g de thiéno[3,2-c]pyridine en solution dans 2 cm³ de chlorure de méthylène et triture pendant 1 heure à température ambiante. On ajoute 70 cm³ d'éther, filtre le précipité, le lave à l'éther, le chromatographie sur silice (éluant : chlorure de méthylène-méthanol 95-5) et obtient 1,117 g de produit attendu.

STADE D : [6R-[3(E), 6alpha, 7béta(Z)]] 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[3,2-c]pyridinium trifluoroacétate iodhydrate.

On opère comme au stade C de l'exemple 6 en utilisant 1,117 g du produit obtenu au stade C et obtient 0,618 g de produit attendu.

### RMN (DMSO 300 MHz)

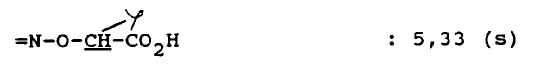

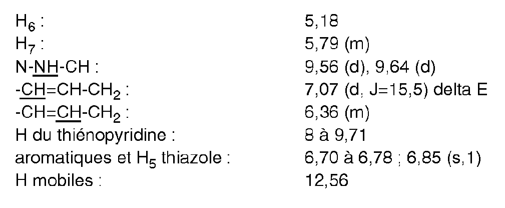

### EXEMPLE 12 : [6R-[3(E), 6alpha, 7béta(Z)]] 2-[3-[7-[[(2-amino 4-thiazolyl) [[1-(3,4dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] isoquinoléinium trifluoroacétate iodhydrate.

### STADE A : [6R-[3(E), 6alpha, 7béta(Z)]] 2-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] 2-propényl] isoquinoléinium iodure.

On opère comme au stade B de l'exemple 6 au départ de 2,48 g de dérivé iodé préparé comme au stade B de l'exemple 11 et 1,04 cm³ d'isoquinoléine. On obtient 1,26 g de produit attendu.

### STADE B : [6R-[3(E), 6alpha, 7béta(Z)]] 2-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] isoquinoléinium trifluoroacétate iodhydrate.

On opère comme au stade C de l'exemple 6 en utilisant 1,26 g du produit obtenu au stade A et obtient 0,673 g de produit attendu.

### RMN (DMSO 300 MHz)

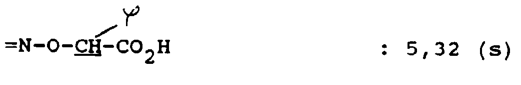

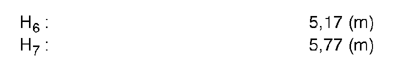

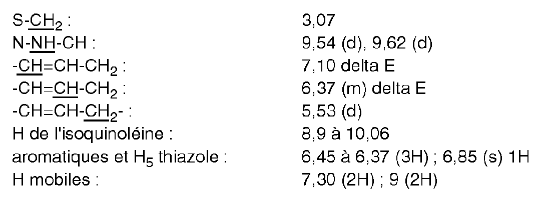

### EXEMPLE 13 : [6R-[3(E), 6alpha, 7béta(Z)]] 5-[3-[7-[[(2-amino 4-thiazolyl) [[1-(3,4-dihydroxy phényl) 2-hydroxy 2-oxoéthoxy imino] acétyl] amino] 2-carboxy 8-oxo 5-thia 1-azabicyclo-[4,2,0]oct-2-en-3-yl] 2-propényl] 2-méthyl 1H-imidazo[4,5-c] pyridinium trifluoroacétate iodhydrate.

### STADE A : [6R-[3(E), 6alpha, 7béta(Z)]] 5-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] 2-propényl] 2-méthyl 1 H-imidazo[4,5-c]pyridinium iodure.

On opère comme au stade B de l'exemple 6 au départ de 1,92 g de dérivé iodé préparé comme au stade B de l'exemple 11 et 0,29 g de 2-méthyl 1 H-imidazo[4,5-c]pyridine. On obtient 1,055 g de produit attendu.

### STADE B : [6R-[3(E), 6alpha, 7béta(Z)]] 5-[3-[7-[[(2-amino 4-thiazolyl) [[1-(3,4-dihydroxy phényl) 2-hydroxy 2-oxoéthoxy imino] acétyl] amino] 2-carboxy 8-oxo 5-thia 1-azabicyclo[4,2,0]oct-2-en-3-yl] 2-propényl] 2-méthyl 1 H-imidazo [4,5-c] pyridinium trifluoroacétate iodhydrate.

On opère comme au stade C de l'exemple 6 en utilisant 1,043 g du produit obtenu au stade A et obtient 0,608 g de produit attendu.

### RMN (DMSO 300 MHz)

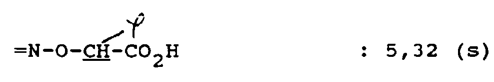

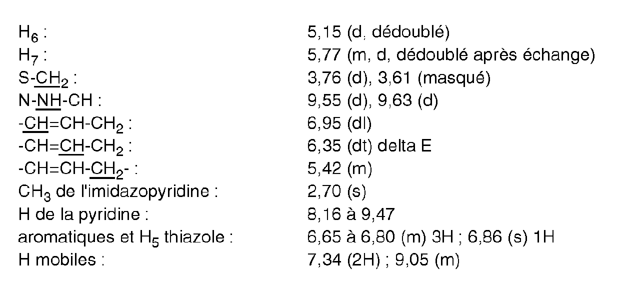

### EXEMPLE 14 : [6R-[3(E), 6alpha, 7béta(Z)]] 3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] N,N,N-triméthyl 2-propén-1-aminium trifluoroacétate iodure

### STADE A : [6R-[3(E), 6alpha, 7béta(Z)]] 3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] N,N,N-triméthyl 2-propén-1-aminium iodure.

On agite pendant 40 minutes à température ambiante, 365 mg de dérivé iodé comme au stade B de l'exemple 11, 0,7 cm³ de tétrahydrofuranne et 220 microlitres d'une solution de triméthylamine dans l'éther (2,37 M/I). On ajoute 20 cm³ d'éther, sépare le précipité, le chromatographie sur silice (éluant : chlorure de méthylène-méthanol 92-8), reprend le résidu à l'éther et obtient après élimination du solvant 276 mg de produit attendu.

### Infra Rouge

=C-NH 3404 cm⁻¹ + associé

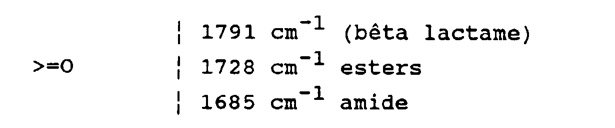

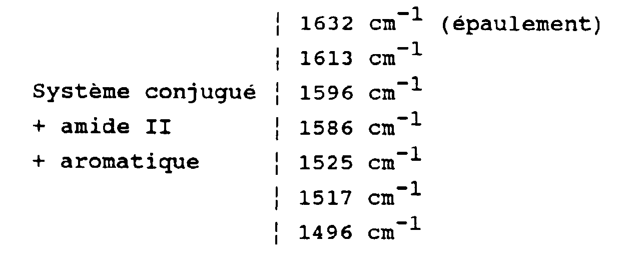

### Ultra Violet

1) Dans l'éthanol + 1cm₃ CH₂Cl₂ 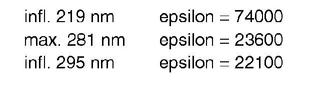 infl. 265, 276 nm
2) Dans l'éthanol HCI 0,1n

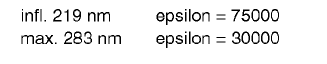

### STADE B : [6R-[3(E), 6alpha, 7béta(Z)]] 3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] N,N,N-triméthyl 2-propén-1- aminium trifluoroacétate iodure.

On agite 1 heure à température ambiante 247 mg de produit obtenu au stade A avec 2,5 cm³ d'acide trifluoroacétique à 10% d'anisole. On ajoute 25 cm³ d'éther isopropylique, agite 10 minutes, isole le précipité formé et sèche sous pression réduite à 20°C pendant 24 heures. On obtient 128 mg de produit attendu.

### RMN (DMSO 300 MHz)

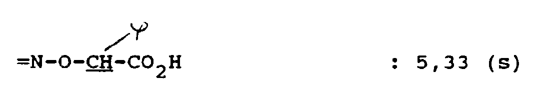

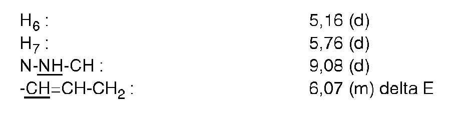

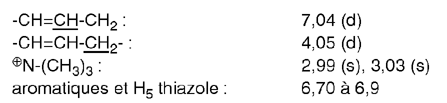

### EXEMPLE 15: [6R-[3(E), 6alpha, 7béta(Z)]] [3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] N-(cyanométhyl) N,N-diméthyl 2-propén-1-aminium] trifluoroacétate iodure.

### STADE A : [6R-[3(E), 6alpha, 7béta(Z)]] [3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] N-(cyanométhyl) N,N-diméthyl 2-propén-1-aminium] iodure.

On opère comme au stade A de l'exemple 14 à partir de 250 mg de dérivé iodé et 250 cm³ d'une solution de diméthylamino acétonitrile dans le tétrahydrofuranne (1-9). On obtient 172 mg de produit attendu.
RMN (CDCl₃ 400 MHz)
-CH=CH-CH₂ : 6,05 (d,t) delta E

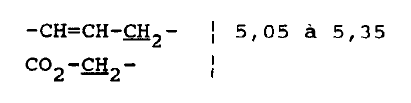

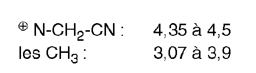

### STADE B : [6R-[3(E), 6alpha, 7béta(Z)]] [3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] N-(cyanométhyl) N,N-diméthyl 2-propén-1-aminium] trifluoroacétate iodure.

On opère comme au stade B de l'exemple 14 à partir de 172 mg de produit préparé au stade A. On obtient 72 mg de produit attendu.

### RMN (DMSO 300 MHz)

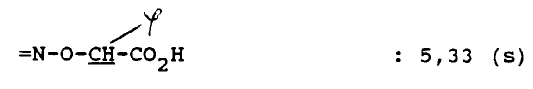

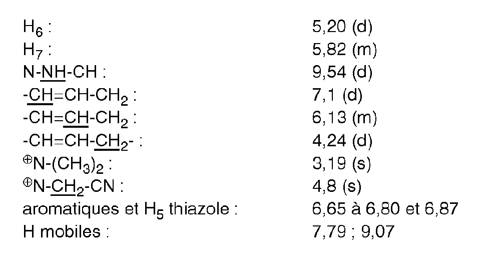

### EXEMPLE 16: [6R-[3(E), 6alpha, 7béta(Z)]] N-(2-amino 2-oxoéthyl) [3-[7-[[(2-amino 4-thiazolyl) [[1-(3,4-dihy- droxy phényl) 2-hydroxy 2-oxoéthoxy] imino] acétyl] amino] 2-carboxy 8-oxo 5-thia 1-azabicyclo-[4,2,0]oct-2-en-3-yl] N,N-diméthyl 2-propén-1-aminium] trifluoroacétate iodure.

### STADE A : [6R-[3(E), 6alpha, 7béta(Z)]] N-(2-amino2-oxoéthyl) [3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxyben- zyloxy) carbonyl] 8-oxo 5-thia 1-azabicyclo[4,2,0]oct-2-en-3-yl] N,N-diméthyl 2-propén-1-aminium] iodure.

On mélange 1 heure à 20°C 350 mg de dérivé iodé obtenu comme indiqué au stade B de l'exemple 11 avec 1,6 cm³ d'acétonitrile et 27 mg de diméthylaminoacétamide. On élimine les solvants sous pression réduite, chromatographie le résdu sur silice (éluant : chlorure de méthylène-méthanol 97-3 puis 92-8). On recueille 300 mg de produit attendu.

### RMN (CDCl₃ 300 MHz)

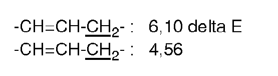

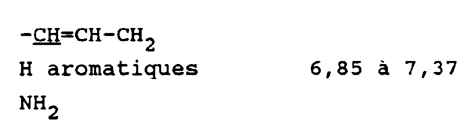

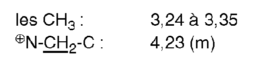

### STADE B : [6R-[3(E); 6alpha, 7béta(Z)]] N-(2-amino 2-oxoéthyl) [3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo-[4,2,0]oct-2-en-3-yl] N,N-diméthyl 2-propén-1-aminium] trifluoroacétate iodure.

On opère comme au stade B de l'exemple 14 à partir de 285 mg de produit préparé au stade A ci-dessus. On obtient 152 mg de produit attendu.

### RMN (DMSO 300 MHz)

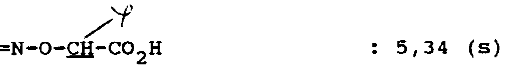

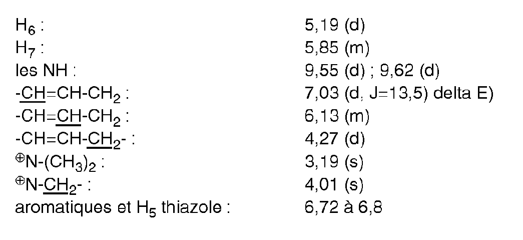

### H mobiles

### EXEMPLE 17 : [6R-[3(E), 6alpha, 7béta(Z)]] 1-[3-[7-[[(2-amino 4-thiazolyl) [[i-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] 1-méthyl pyrrolidinium trifluoroacétate iodure.

### STADE A : [6R-[3(E), 6alpha, 7béta(Z)]] [3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] 2-propényl] 1-méthyl pyrrolidinium iodure.

On dissout à 20°C, 357 mg de dérivé iodé obtenu comme indiqué au stade B de l'exemple 11 dans 7 cm³ d'éther et 1,3 cm³ de chlorure de méthylène. On ajoute 130 microlitres de méthylpyrrolidine, ajoute 5 cm³ d'éther, agite 10 minutes, isole le précipité et le sèche à 20°C sous pression réduite. On obtient 300 mg de produit attendu.

### STADE B : [6R-[3(E), 6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl]2-propényl] 1-méthyl pyrrolidinium trifluoroacétate iodure.

On opère comme au stade B de l'exemple 14 à partir de 290 mg de produit obtenu au stade A ci-dessus. On obtient 150 mg de produit attendu.

### RMN (DMSO 300 MHz)

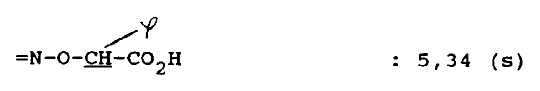

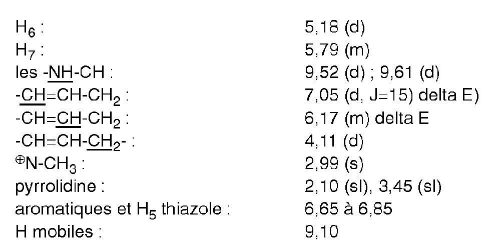

### EXEMPLE 18: [6R-[3(E), 6alpha, 7béta(Z)]] 7-[3-[7-[[(2-amino 4-thiazolyl) [[(R) 1-(3,4-dihydroxy phényl) 2-hydroxy 2-oxoéthoxy] imino] acétyl] amino] 2-carboxy 8-oxo 5-thia 1-azabicyclo-[4,2,0]oct-2-en-3-yl] 2-propényl] thiéno [2,3-b] pyridinium trifluoroacétate iodhydrate.

### STADE A : [6R-[3(E), 6alpha, 7béta (Z)]] p-méthoxybenzyl 7-[[[(R) 1-[3,4-dihydroxy) phényl] 2-(diphénylméthoxy) 2-oxoéthoxy] imino] [2-[(triphénylméthyl) amino] 4-thiazolyl] acétyl] amino] 3-(3-chloro 1-propényl) 8-oxo 5-thia 1-azabicyclo[4,2,0]oct-2-èn-3-yl 2-carboxylate.

On agite pendant 5 minutes 1,1 g d'acide [[(R) (3,4-dihydroxyphényl) (diphénylméthoxycarbonyl) méthoxy] imino] [2-(triphénylméthylamino) 4-thiazolyl] acétique isomère syn (préparé comme indiqué pour l'isomère S dans les Brevets européens EP 2 266 060 et 0 280 521 ou dans le brevet allemand DE 37 42 457 A1 ) dans 11,36 cm³ de chlorure de méthylène. On refroidit à -5°C la solution obtenue, ajoute 403,4 mg de dicyclocarbodiimide, agite 40 minutes et ajoute 668 mg de chlorhydrate de p-méthoxybenzyle 7-amino 3-(3-chloro 1-propényl) 8-oxo 5-thia 1-azabicyclo[4,2,0]oct-2-èn-2-carboxylate préparé comme indiqué dans le brevet européen EP 0 333 154. On agite 3 heures en laissant revenir à température ambiante, élimine les solvants, chromatographie le résidu sur silice (éluant : chlorure de méthylène-acétate d'éthyle 9-1) et obtient 712 mg de produit attendu.
RMN (CDCl₃ 300 MHz) 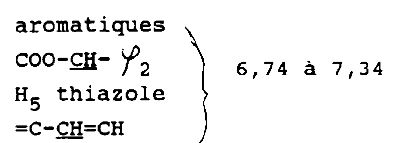
-CH=CH-CH₂: 6,25 (d, J=1) delta Z 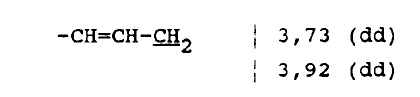 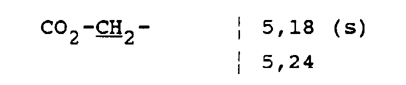
ϕ-OCH3: 3,81 (s)

### STADE B : [6R-[3(E), 6alpha, 7béta (Z)]] p-méthoxybenzyl 7-[[[(R)-1-[3,4-[(dihydroxy) phényl] 2-(diphénylméthoxy) 2-oxoéthoxy] imino] [2-[(triphénylméthyl) amino] 4-thiazolyl] acétyl] amino] 3-(3-iodo 1-propényl) 8-oxo 5-thia 1-azabicyclo [4,2,0]oct-2-èn-3-yl 2-carboxylate.

On agite pendant 2 heures à température ambiante un mélange de 590 mg de produit obtenu au stade A, 11,9 cm³ d'acétone et 216 mg d'iodure de sodium, évapore le solvant puis reprend le résidu par 5 cm³ de chlorure de méthylène. On lave la solution par 3 fois 10 cm³ de thiosulfate de sodium puis par 2 fois 10 cm3 d'une solution aqueuse de chlorure de sodium. On sèche, cristallise dans l'éther, et obtient 456,6 mg de produit attendu.
RMN (CDCl₃ 400 MHz)

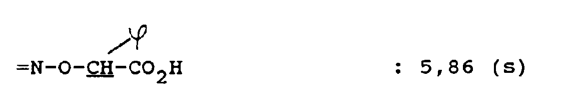

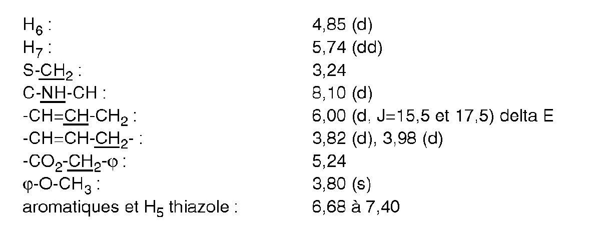

### STADE C : [6R-[3(E), 6alpha, 7béta(Z)]] 7-[3-[7-[[[(R)-1-[3,4-bis[(2-méthoxy éthoxy) méthoxy] phényl] 2-[(diphénylmé- thoxy) 2-oxoéthoxy] imino] [2-[(triphénylméthyl) amino] 4-thiazolyl] acétyl] amino] 2-[(p-méthoxybenzyloxy) carbonyl] 8-oxo 5-thia 1-azabicyclo[4,2,0]oct-2-en-3-yl] 2-propényl] thiéno-[2,3-b]pyridinium iodure.

On agite et triture pendant deux heures à température ambainte 446 mg de dérivé iodé obtenu au stade B et 0,44 cm³ de thiéno pyridine. On ajoute de l'éther et sèche sous pression réduite pendant 24 heures le solide obtenu. On obtient 442 mg de produit attendu.

### RMN

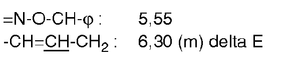

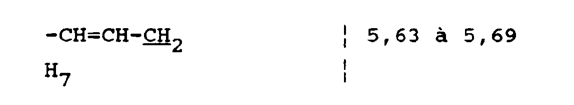

### H du thiéno pyridine : 7,89 à 9,21

STADE D : [6R-[3(E), 6alpha, 7béta(Z)]] 7-[3-[7-[[(2-amino 4-thiazolyl) [[(R) 1-(3,4-dihydroxy phényl) 2-hydroxy2-oxoéthoxy] imino] acétyl] amino] 2-carboxy 8-oxo 5-thia 1-azabicyclo[4,2,0]oct-2-en-3-yl] 2-propényl] thiéno [2,3-b] pyridinium trifluoroacétate iodhydrate.

On agite 1 heure à température ambiante 632 mg de produit obtenu comme au stade C dans 6,32 cm³ d'une solution d'acide trifluoroacétique à 10% d'anisole. On refroidit à +5°C, ajoute 65 cm³ d'éther isopropylique, agite 10 minutes, filtre et sèche sous pression réduite à température ambiante pendant 16 heures. On obtient 403 mg de produit attendu.

### RMN (DMSO 400 MHz)

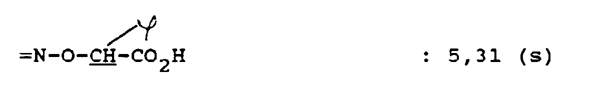

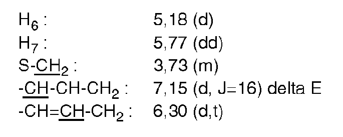

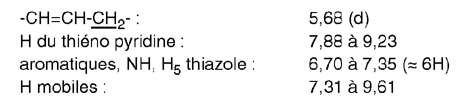

### EXEMPLE 19: [6R-[3(E), 6alpha, 7béta(Z)]] 7-[3-[7-[[(2-amino 4-thiazolyl) [[(S)-1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[2,3-b] pyridinium trifluoroacétate iodure.

### STADE A: [6R-[3(E), 6alpha, 7béta(Z)]] p-méthoxybenzyl 7-[[[(S) 1-[3,4-dihydroxy) phényl] 2-(diphénylméthoxy) 2-oxoéthoxy] imino] [2-[(triphénylméthyl) amino] 4-thiazolyl] acétyl] amino] 3-(3-chloro 1-propényl) 8-oxo 5-thia 1-azabicyclo[4,2,0]oct-2-èn-3-yl 2-carboxylate.

On opère comme au stade A de l'exemple 18 en utilisant au départ 678 mg d'acide [[(S) (3,4-dihydroxyphényl) (diphénylméthoxycarbonyl) méthoxy] imino] [2-(triphénylméthylamino) 4-thiazolyl] acétique isomère syn préparé comme indiqué dans les brevets européens EP 0 266 060 et 0 280 521 ou dans le brevet allemand DE 37 32 457 A1 et 412 mg de chlorhydrate de p-méthoxybenzyl de 7-amino 3-(3-chloro 1-propényl) 8-oxo 5-thia 1-azabicyclo[4,2,0]oct-2-èn 2-carboxylate. On obtient 590 mg de produit attendu.
RMN (CDCl₃ 400 MHz)
=N-O-CH- : 5,89 (s)

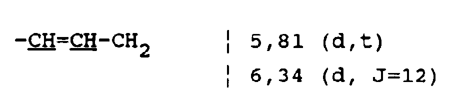

### STADE B : [6R-[3(E), 6alpha, 7béta(Z)]] p-méthoxybenzyl 7-[[[(S)-1-[3,4-[(dihydroxy) phényl] 2-(diphénylméthoxy) 2-oxoéthoxy] imino] [2-[(triphénylméthyl) amino] 4-thiazolyl] acétyl] amino] 3-(3-iodo 1-propényl) 8-oxo 5-thia 1-azabicyclo [4,2,0]oct-2-èn-3-yl 2-carboxylate.

On opère comme au stade B de l'exemple 18 en utilisant 850 mg du produit obtenu au stade A et 335 mg d'iodure de sodium. On obtient 595 mg de produit attendu.
Infra Rouge (CHCl₃) 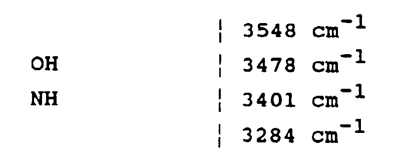 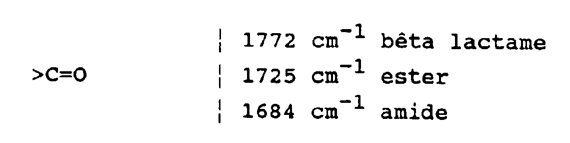 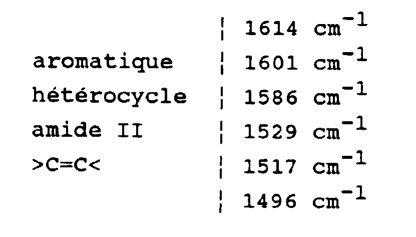
Ultra Violet

### 1) dans le dioxane :

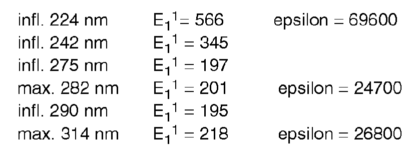

### 2) dans le dioxane/HCI 0;1N

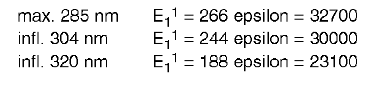

### STADE C : [6R-[3(E), 6alpha, 7béta(Z)]] 7-[3-[7-[[[(S)-1-[3,4-bis[(2-méthoxy éthoxy) méthoxy] phényl] 2-[(diphénylmé- thoxy) 2-oxoéthoxy] imino] [2-[(triphénylméthyl) amino] 4-thiazolyl] acétyl] amino] 2-[(p-méthoxybenzyloxy) carbonyl] 8-oxo 5-thia 1-azabicyclo[4,2,0]oct-2-en-3-yl] 2-propényl] thiéno[2,3-b] - pyridinium iodure.

On opère comme au stade C de l'exemple 18 en utilisant 430 mg de dérivé iodé obten au stade B et 470 mg de thiéno pyridine. On obtient 438 mg de produit attendu.

### Infra Rouge (CHCl₃)

Région NH/OH complexe

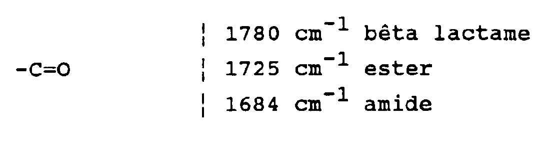

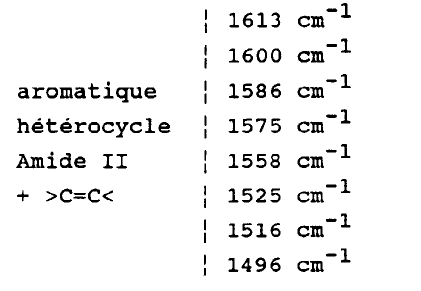

### STADE D : [6R-[3(E); 6alpha, 7béta(Z)]] 7-[3-[7-[[(2-amino4-thiazolyl) [[(S)-1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[2,3-b] pyridinium trifluoroacétate iodure.

On opère comme au stade D de l'exemple 18 en utilisant 400 mg de produit obtenu au stade C. On obtient 275 mg de produit attendu.

### RMN (DMSO 400 MHz)

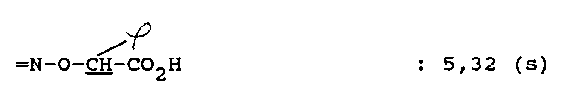

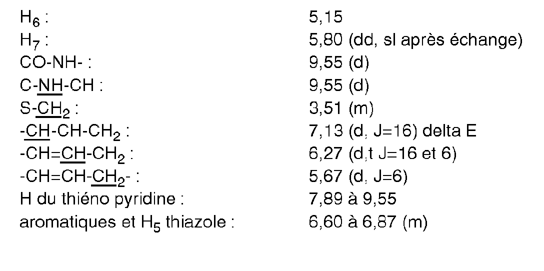

### EXEMPLE 20: [6R-[3(E), 6alpha, 7béta(Z)]] 5-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] 1,2-diméthylimidazo [4,5-c]pyridinium trifluoroacétate iodhydrate.

### STADE A : [6R-[3(E), 6alpha, 7béta(Z)]] 5-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] 2-propényl] 1,2-diméthylimidazo[4,5-c]pyridinium iodure.

On agite pendant 1 heure 1,08 g de produit obtenu comme indiqué au stade B de l'exemple 11 avec 170 mg de 1,2-diméthyl 4-aza benzimidazole dans 0,9 cm³ d'acétonitrile. On ajoute 40 cm³ d'éther, filtre le précipité, le rince à l'éther et le sèche 16 heures sous pression réduite. Après chromatographie sur silice (éluant : chlorure de méthylène-méthanol 94-6), on obtient 306 mg de produit attendu.

STADE B : [6R-[3(E), 6alpha, 7béta(Z)]] 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0] oct-2-en-3-yl] 2-propényl] 1,2-diméthylimidazo [4,5-c] pyridinium trifluoroacétate iodhydrate.

On opère comme à l'exemple 14 stade B à partir de 297 mg de produit obtenu au stade A. On obtient 155 mg de produit attendu.
RMN (DMSO 400 MHz)

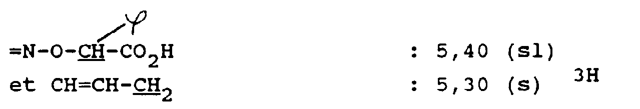

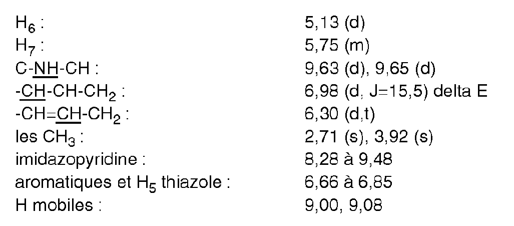

### EXEMPLE 21 : [6R-[3(E), 6alpha, 7béta(Z)]] 5-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] 2,3-diméthylimidazo[4,5-c] pyridinium trifluoroacétate iodhydrate.

### STADE A : [6R-[3(E), 6alpha, 7béta (Z)]] 5-[3-[7-[[[1-[3,4bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] 2-propényl] 2,3-diméthylimidazo[4,5-c]pyridinium iodure.

On opère comme indiqué au stade A de l'exemple 20 en utilisant 1,92 g de produit obtenu comme au stade B de l'exemple 11 et 303 mg de 2,3-diméthyl 4-aza benzimidazole. On obtient 877 mg de produit attendu.

### STADE B : [6R-[3(E), 6alpha, 7béta (Z)]] 5-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] 2,3-diméthylimidazo [4,5-c] pyridinium trifluoroacétate iodhydrate.

On opère comme au stade B de l'exemple 14 à partir de 865 mg de produit obtenu au stade A. On obtient 488 mg de produit attendu.
RMN (DMSO 400 MHz)

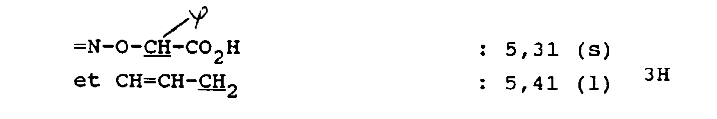

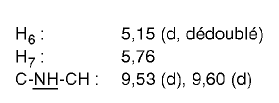

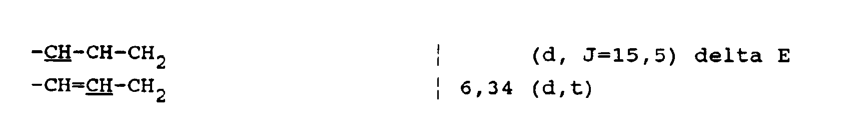

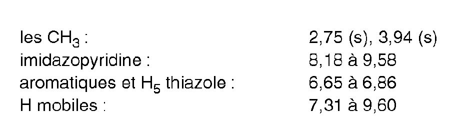

### EXEMPLE 22 : [6R-[3(E), 6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] quinoléinium trifluoroacétate iodhydrate.

### STADE A : [6R-[3(E), 6alpha, 7béta(Z)]] 1-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] 2-propényl] quinoléinium iodure.

On opère comme au stade B de l'exemple 6 au départ de 2,50 g de dérivé iodé préparé comme au stade B de l'exemple 11 et 0,63 g de quinoléine. On obtient 2,40 g de produit attendu que l'on purifie par chromatographie sur silice (éluant : chlorure de méthylène-méthanol 95-5).

### STADE B : [6R-[3(E), 6alpha, 7béta (Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] quinoléinium trifluoroacétate iodhydrate.

On opère comme au stade B de l'exemple 14 en utilisant 1,65 g du produit obtenu au stade A et obtient 0,94 g de produit attendu.
RMN (DMSO 400 MHz)

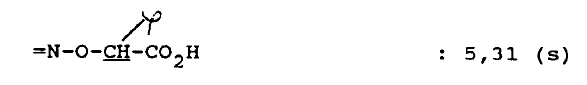

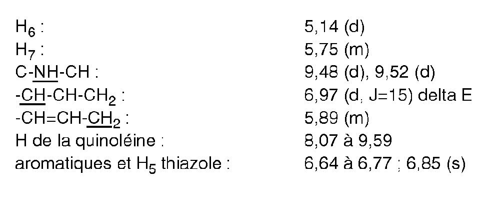

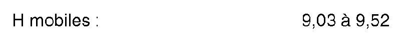

### EXEMPLE 23: [6R-[3(E),6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4,2,0]oct-2-en-3-yl] 2-propényl] 4-éthylthio pyridinium trifluoroacétate iodhydrate.

### STADE A: [6R-[3(E), 6alpha, 7béta(Z)]] 1-[3-[7-[[[1-[3,4-bis[(2-méthoxy éthoxy) méthoxy] phényl] 2-[(diphénylméthoxy) 2-oxoéthoxy] imino] [2-[(triphénylméthyl) amino] 4-thiazolyl] acétyl] amino] 2-[(paraméthoxybenzyloxy) carbonyl] 8-oxo 5-thia 1-azabicyclo[4,2,0]oct-2-en-3-yl] 2-propényl] 4-éthylthio pyridinium iodure.

On opère comme au stade B de l'exemple 6 au départ de 2,50 g de dérivé iodé préparé comme au stade B de l'exemple 11 et 1 cm³ de 4-éthylthio pyridine. On obtient 2,45 g de produit attendu que l'on purifie par chromatographie sur silice (éluant : chlorure de méthylène-méthanol 95-5).

### STADE B : [6R-[3(E), 6alpha, 7béta(Z)]] 1-[3-[7-[[(2-amino 4-thiazolyl) [[1-(3,4-dihydroxy phényl) 2-hydroxy 2-oxoéthoxy] imino] acétyl] amino] 2-carboxy 8-oxo 5-thia 1-azabicyclo[4;2,0]oct-2-en-3-yl] 2-propényl] 4-éthylthio pyridinium trifluoroacétate iodhydrate.

On opère comme au stade B de l'exemple 14 en utilisant 1,54 g du produit obtenu au stade A et obtient 0,807 g de produit attendu.
RMN (DMSO 400 MHz)

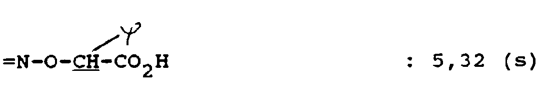

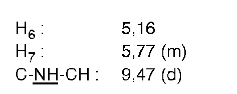

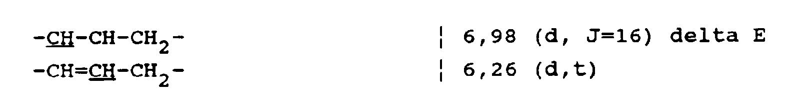

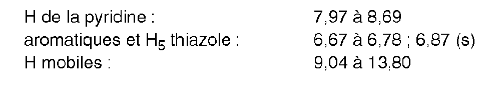

### EXEMPLE 24 : On a réalisé des préparations pour injections de formule:

Produit de l'exemple 2 500 mg
Excipient aqueux stérile q.s.p. 5 cm³

### ETUDE PHARMACOLOGIQUE DES PRODUITS DE L'INVENTION

Activité in vitro, méthode des dilutions en milieu solide.

On prépare une série de boites dans lesquelles on répartit une même quantité de milieu nutritif stérile, contenant des quantités croissantes du produit à étudier puis chaque boite est ensemencée avec plusieurs souches bactériennes.

Après incubation de 24 heures en étuve à 37°C, l'inhibition de la croissance est appréciée par l'absence de tout développement bactérien ce qui permet de déterminer les concentrations minimales inhibitrices (CMI) exprimées en microgrammes/cm³.

Les résultats sont exprimés en CMl₉₀ qui est la concentration minimum d'antibiotique permettant d'inhiber la croissance de 90 % des souches étudiées.

On a obtenu les résultats suivants :

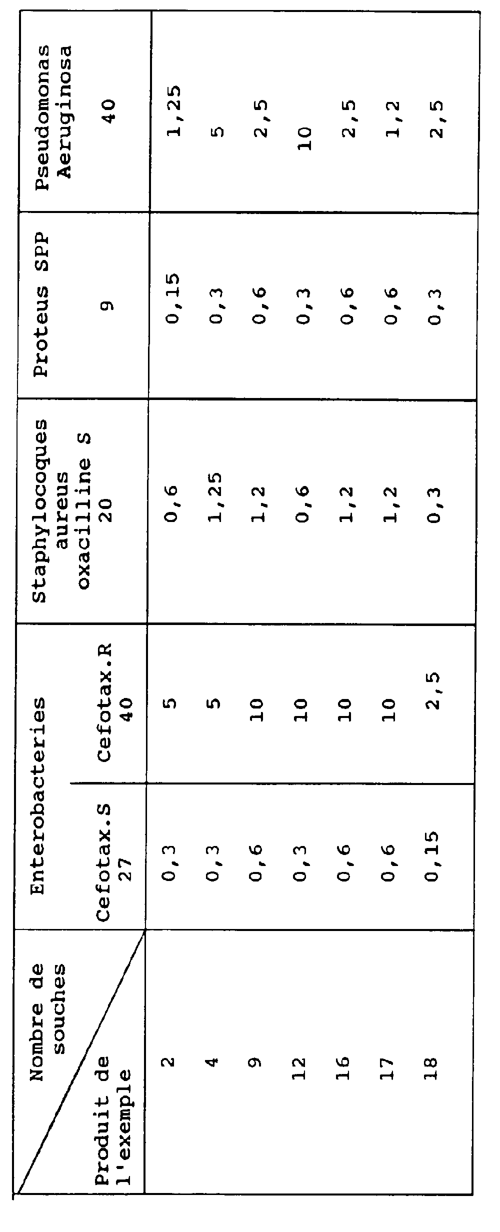

## Revendications (Revendications pour l'(les) Etat(s) contractant(s) suivant(s) : AT, BE, CH, DE, DK, FR, GB, IT, LI, LU, NL, SE)

1. Les produits de formule générale (I) : 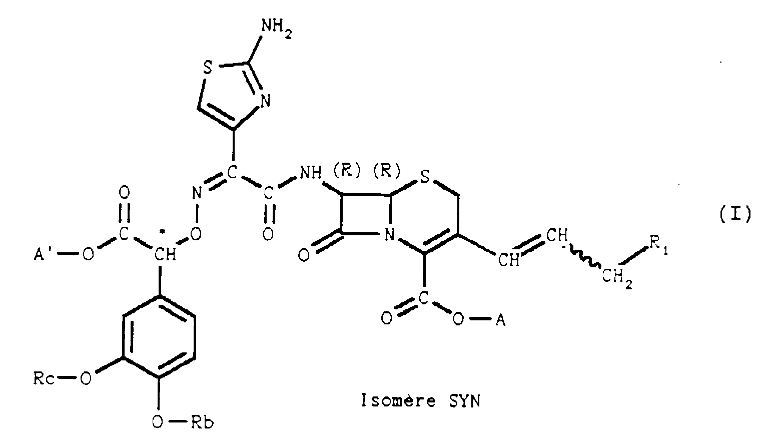 isomère syn, sous forme R ou S ou d'un mélange R, S, formule dans laquelle : R₁ représente un radical choisi parmi les radicaux suivants : 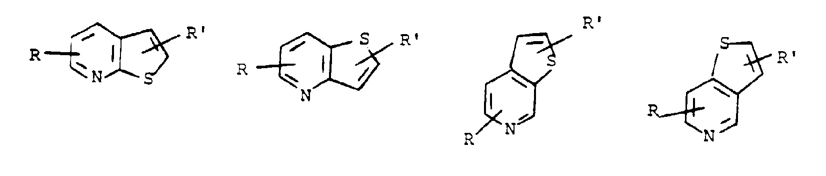 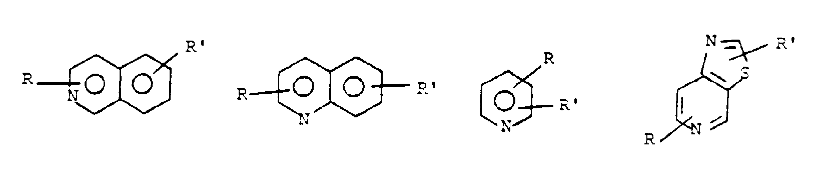 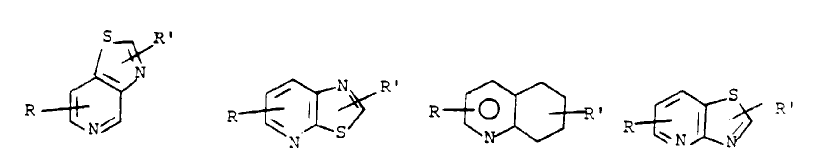 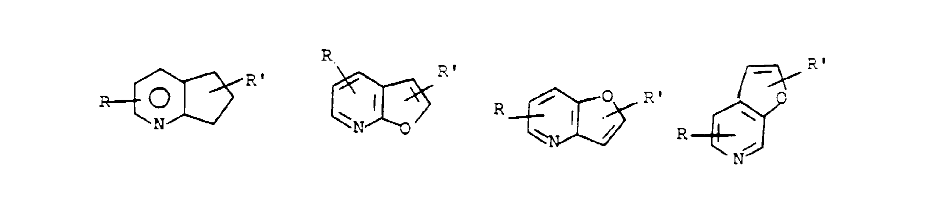 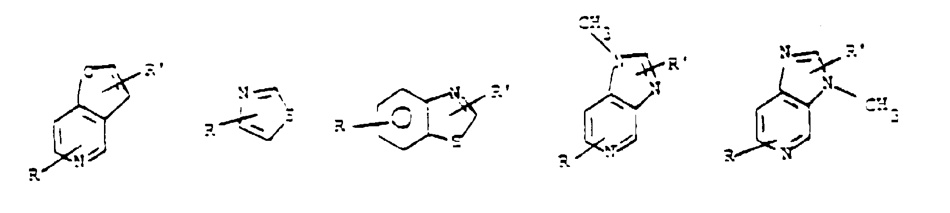 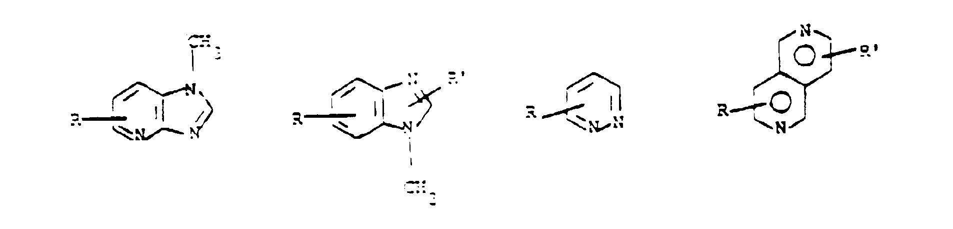 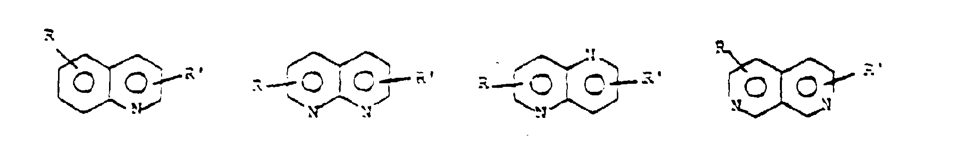 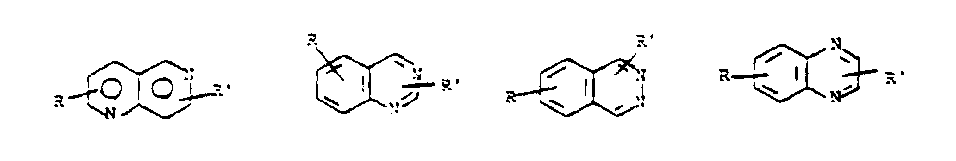 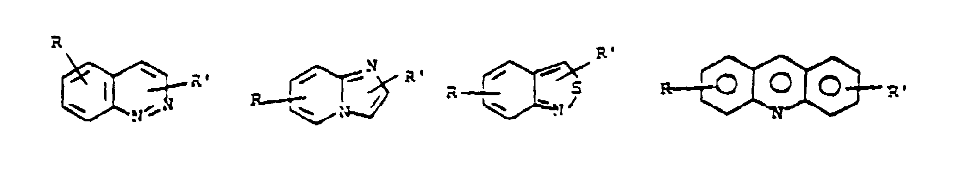 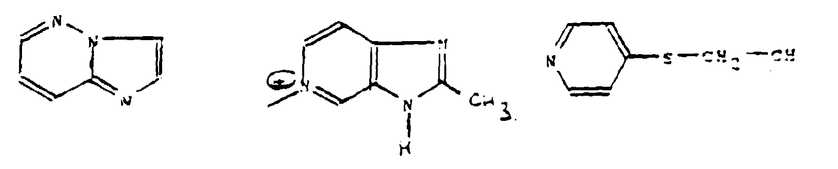 ou 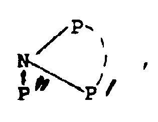 sous forme d'ammonium quaternaire, l'expression sous forme d'ammonium quaternaire indiquant que le radical R₁ est lié avec le groupement -CH=CH-CH₂- par le ou l'un des atomes d'azote qu'il comporte, dans lesquels R et R' identiques ou différents représentent un atome d'hydrogène, un radical alkyle renfermant de 1 à 4 atomes de carbone, un radical alcoxy renfermant de 1 à 4 atomes de carbone, un atome d'halogène, un radical CO₂-Q, 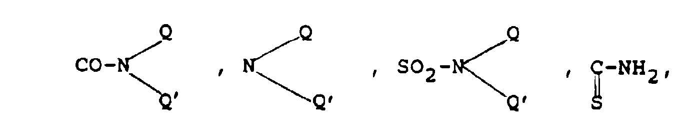 NH-CO-Q, CN CH₂-CN, CH₂-SQ dans lesquels Q et Q' identiques ou différents représentent un atome d"hydrogène ou un radical alkyle renfermant de 1 à 4 atomes de carbone, P, P' et P" identiques ou différents représentent un radical alkyle renfermant au plus 4 atomes de carbone, éventuellement substitué par un des substituants indiqués ci-dessus pour R et R', le symbole) indiqant que P et P' peuvent éventuellement former avec l'atome d'azote auquel ils sont liés, un hétérocycle à 5 ou 6 chaînons
R_{b} et R_{c}, identiques ou différents représentent un atome d'hydrogène ou un groupement acyle, choisi parmi les radicaux acétyle, propionyle et benzoyle,
A et A' identiques ou différents représentent un atome
d"hydrogène, un équivalent de métal alcalin, alcalino-terreux, de magnésium, d'ammonium ou d'une base organique aminée ou A et A' représentent le reste d'un groupement ester facilement
clivable ou C0₂A représente CO₂⁻ ; le trait ondulé signifie que le groupement CH₂R₁ peut se trouver dans la position E ou Z ainsi que les sels des produits de formule (I) avec les acides minéraux ou organiques.

2. Les produits de formule générale (I) telle que définie à la revendication 1 dans laquelle R₁ est choisi parmi les radicaux suivants :

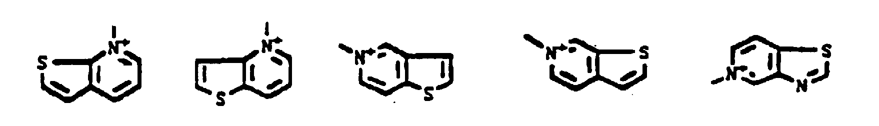

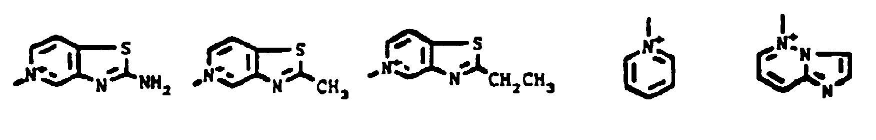

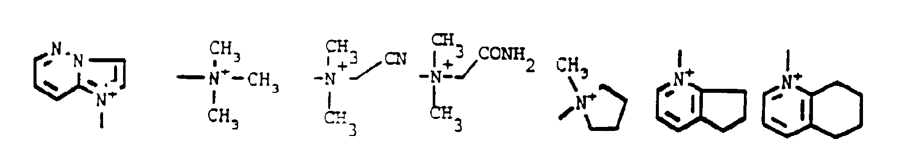

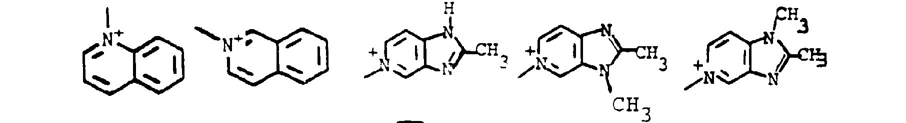

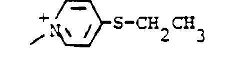

3. Les produits de formule générale (I) telle que définie à la revendication 1 ou 2 dans laquelle R₁ est choisi parmi les radicaux :

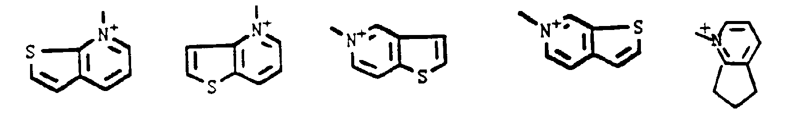

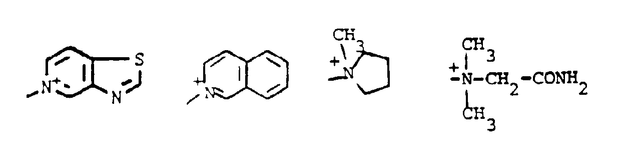

4. Les produits de formule générale (I) telle que définie à la revendication 3 dans laquelle R₁ représente le radical :

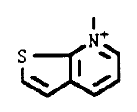

5. Le produit de formule générale (I) selon la revendication 1 dont le nom suit :
le [6R-[3(E), 6alpha, 7béta(Z)]] 5-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] thiazolo[4,5-c] pyridinium sous forme R ou S ou d'un mélange R,S et sous forme de sel interne ou de sel avec les métaux alcalins, alcalino-terreux, le magnésium, l'ammoniaque, les bases organiques aminées, les. acides et ses esters facilement clivables,
le [6R-[3(E), 6alpha, 7béta(Z)]] 7-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[2,3-b] pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables et particulièrement sous forme S,
le [6R-[3(E), 6alpha, 7béta (Z)]] 2-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] isoquinoléinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] 1-méthyl pyrrolidinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] 6,7-dihydro 5H-pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] N-(2-amino 2-oxoéthyl) 3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] N,N-diméthyl 2-propén-1-aminium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables.

6. Procédé de préparation des produits de formule (1) telle que définie à la revendication 1, caractérisé en ce que l'or fait agir un produit de formule (II) : 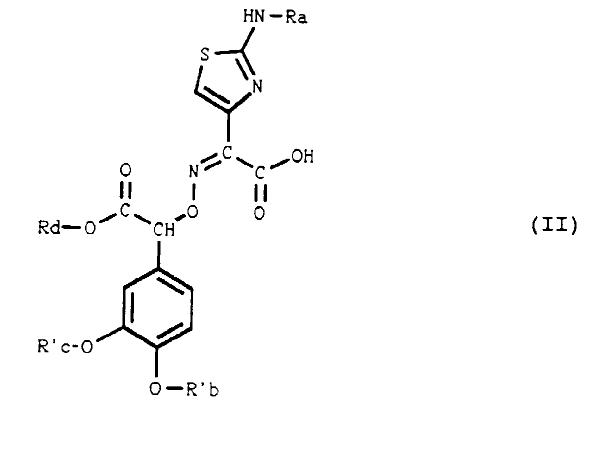 isomère syn, racémique ou optiquement actif ou un dérivé fonctionnel du produit de formule (II), dans laquelle Rₐ représente un atome d'hydrogène ou un groupement protecteur du radical amino, R'_{b} et R'_{c} identiques ou différents représentent un atome d'hydrogène ou un groupement protecteur du radical hydroxyle, R_{d} représente un atome d'hydrogène ou le reste d'un groupement ester facilement éliminable, avec un produit de formule (III) : 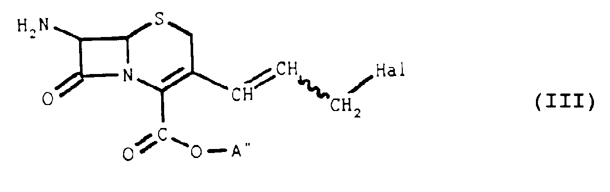 dans laquelle Hal représente un atome d'halogène, A" représente un atome d'hydrogène ou le reste d'un groupement ester facilement éliminable et le trait ondulé signifie que le groupement CH₂Hal peut se trouver dans la position E ou Z pour obtenir un produit de formule (IV) : 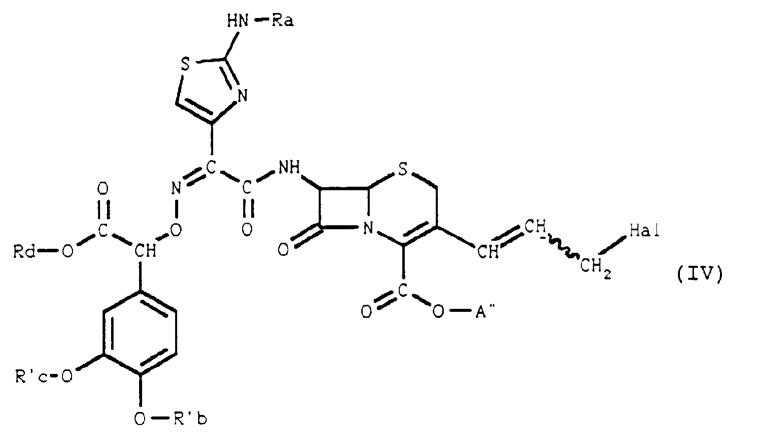 que l'on fait agir avec un réactif capable d'introduire le radical R₁ pour obtenir un produit de formule (V) : 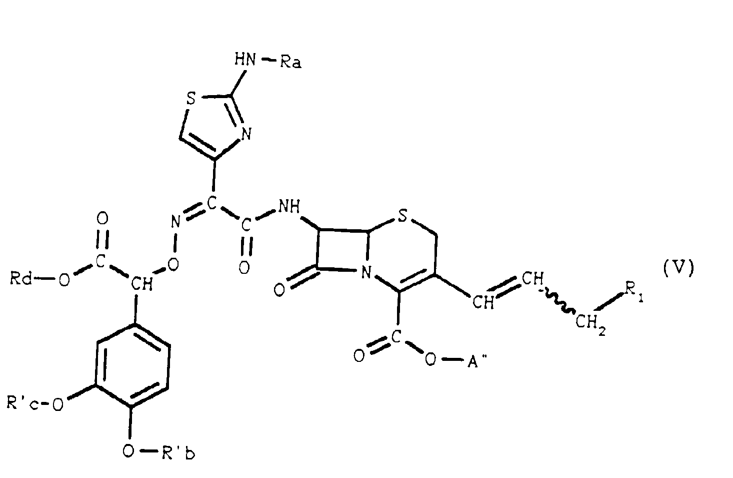 que, si désiré, l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :
a) coupure par hydrolyse ou par action de la thiourée de tout ou partie des groupements esters ou des groupements de protection du radical amino ou des radicaux hydroxyles,
b) estérification ou salification par une base du ou des radicaux carboxyliques,
c) salification par un acide du radical amino,
d) séparation des produits sous forme de mélange R,S en R ou S.

7. Procédé de préparation selon la revendication 6 caractérisé en ce que le réactif capable d'introduire le radical Rᵢ est choisi parmi les réactifs de formule :

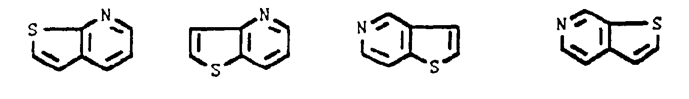

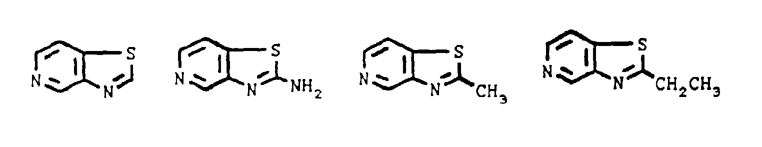

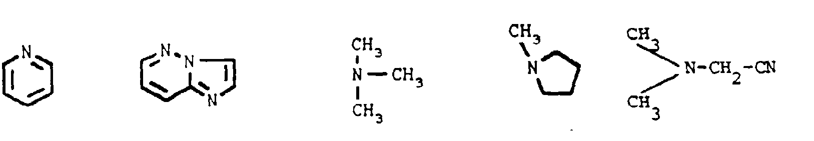

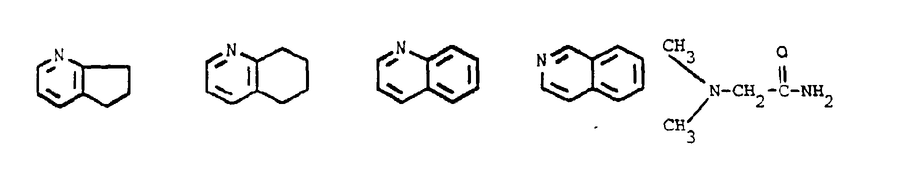

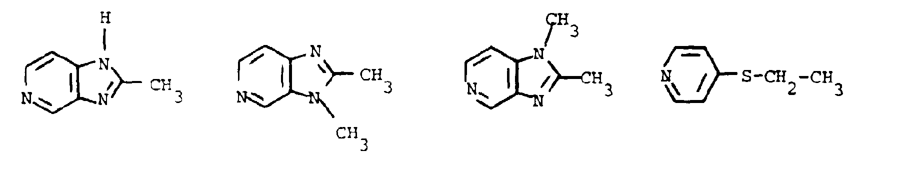

8. A titre de médicaments, les produits répondant à la formule (I) telle que définie à la revendication 1, ainsi que leurs sels d'acides, pharmaceutiquement acceptables.

9. A titre de médicaments, les produits tels que définis à l'une quelconque des revendications 2 à 5 ainsi que leurs sels d'acides, pharmaceutiquement acceptables.

10. Compositions pharmaceutiques contenant, à titre de principe actif, au moins un médicament selon l'une des revendications 8 ou 9.

11. A titre de produits industriels, les produits de formule (IV) et les produits de formule (V) dans laquelle Rₐ représente un groupement protecteur du radical amino, les formules (IV) et (V) étant telles que définies à la revendication 6.

## Revendications (Revendications pour l'(les) Etat(s) contractant(s) suivant(s) : ES)

1. Procédé pour préparer les produits de formule générale (I) 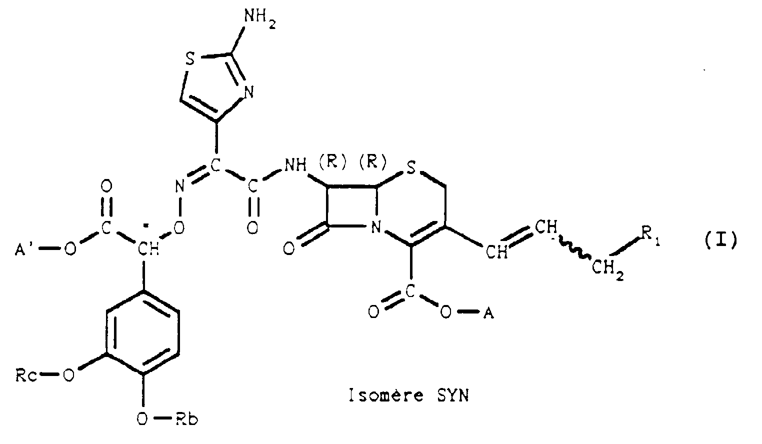 isomère sym, sous forme R ou S ou d'un mélange R, S, formule dans laquelle : R₁ représente un radical choisi parmi les radicaux suivants : 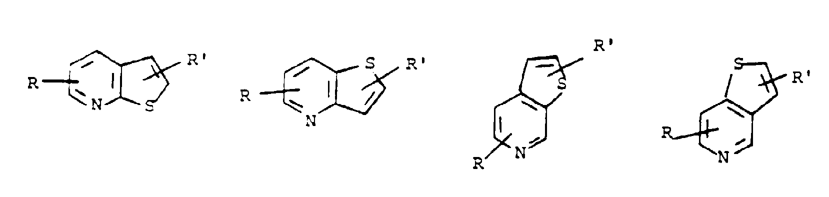 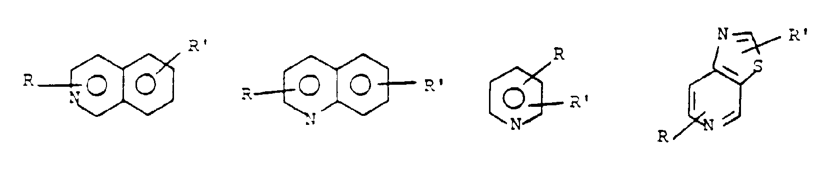 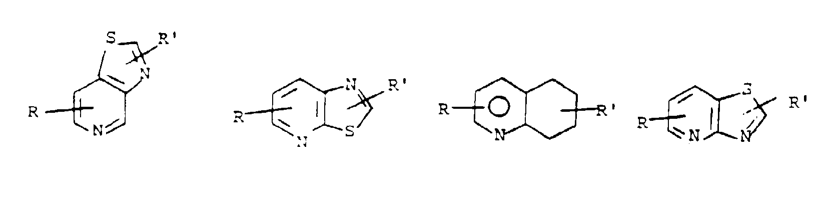 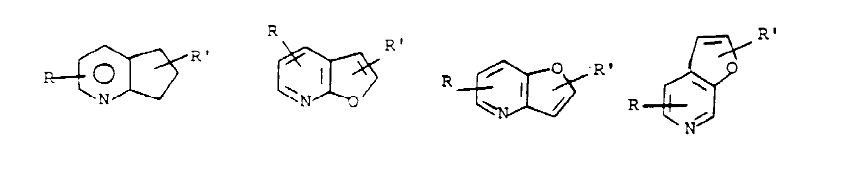 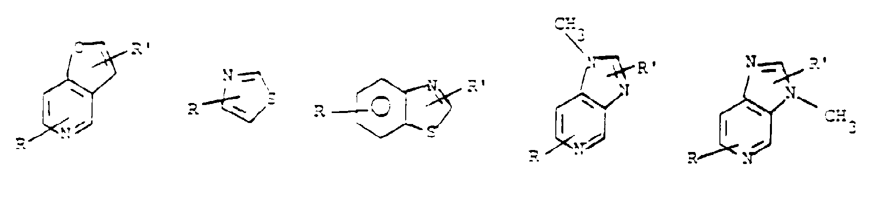 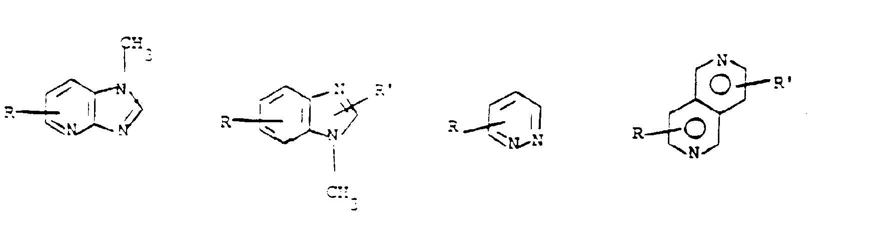 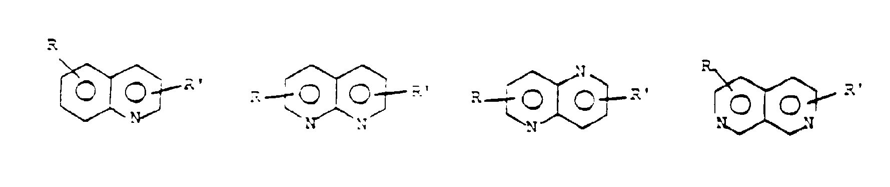 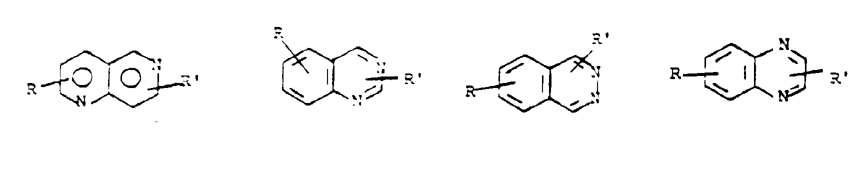 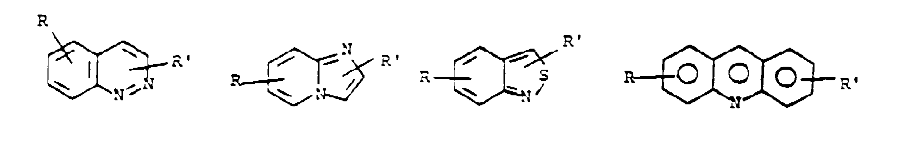 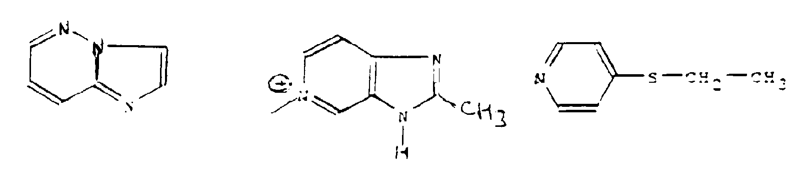 ou 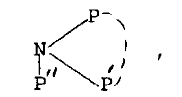 sous forme d'ammonium quaternaire, l'expression sous forme d'ammonium quartenaire indiquant que le radical R₁ est lié avec le groupement -CH=H-CH₂- par le ou l'un des atomes d'azote qu'il comporte,
dans lesquels R et R' identiques ou différents représentent un atome d'hydrogène, un radical alkyle renfermant de 1 à 4 atomes de carbone, un radical alcoxy renfermant de 1 à 4 atomes de carbone, un atome d'halogène, un radical CO₂-Q, 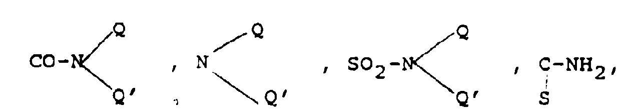 NH-CO-Q, CN, CH₂-CN, CH₂-SQ dans lesquels Q et Q' identiques ou différents représentent un atome d'hydrogène ou un radical alkyle renfermant de 1 à 4 atomes de carbone, P P' et P" identiques ou différents représentent un radical alkyle renfermant au plus 4 atomes de carbone, éventuellement substitué par un des substituants indiqués ci-dessus pour R et R', le symbole indiqant que P et P' peuvent éventuellement former avec l'atome d'azote auquel ils sont liés, un hétérocycle à 5 ou 6 chaînons.
R_{b} et R_{c}, identiques ou différents représentent un atome d'hydrogène ou un groupement acyle, choisi parmi les radicaux acétyle, propionyle et benzoyle,
A et A' identiques ou différents représentent un atome d'hydrogène, un équivalent de métal alcalin, alcalino-terreux, de magnésium, d'ammonium ou d'une base organique aminée ou A et A' représentent le reste d'un groupement ester facilement clivable ou C0₂A représente CO₂⁻; le trait ondulé signifie que le groupement CH₂R₁ peut se- trouver dans la position E ou Z ainsi que les sels des produits de formule (I) avec les acides minéraux ou organiques, caractérisé en ce que l'on fait agir un produit de formule (II) : 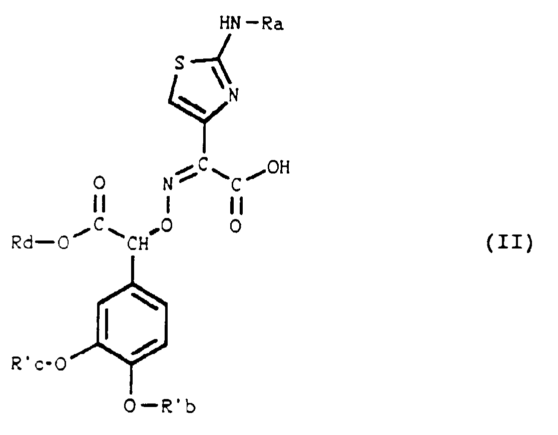 isomère syn, racémique ou optiquement actif ou un dérivé fonctionnel du produit de formule (II), dans laquelle Rₐ représente un atome d'hydrogène ou un groupement protecteur du radical amino, R'_{b} et R'_{c} identiques ou différents représentent un atome d'hydrogène ou un groupement protecteur du radical hydroxyle, R_{d} représente un atome d'hydrogène ou le reste d'un groupement ester facilement éliminable, avec un produit de formule (III) : 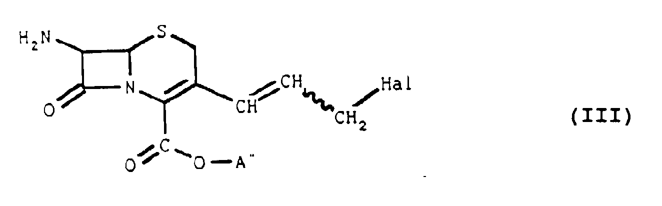 dans laquelle Hal représente un atome d'halogène, A" représente un atome d'hydrogène ou le reste d'un groupement ester facilement éliminable et le trait ondulé signifie que le groupement CH₂Hal peut se trouver dans la position E ou Z pour obtenir un produit de formule (IV) : 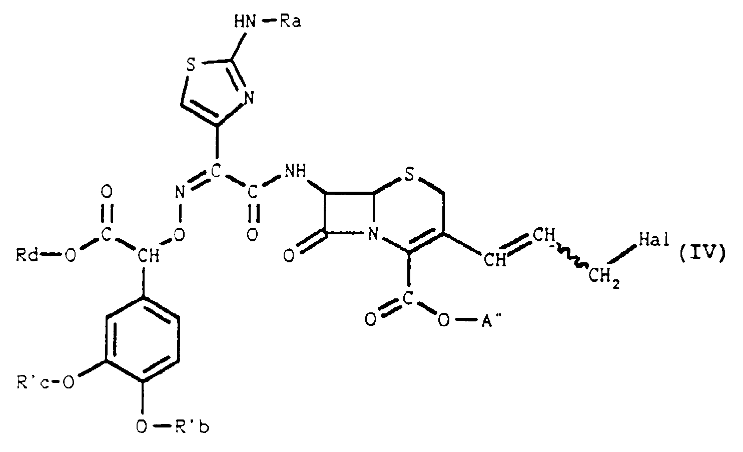 que l'on fait agir avec un réactif capable d'introduire le radical R, pour obtenir un produit de formule (V) : 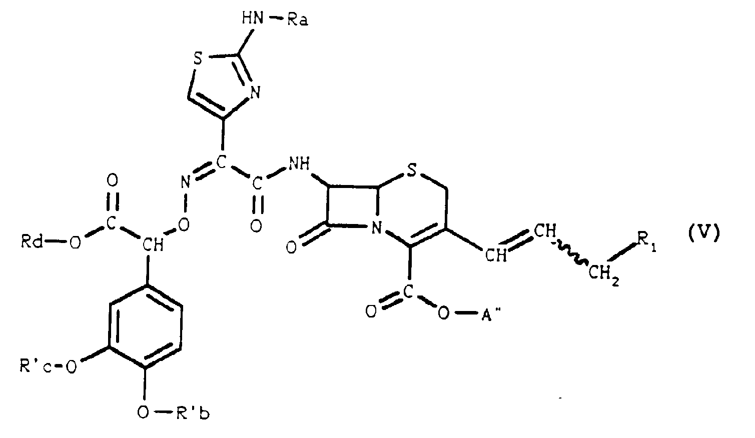 que, si désiré, l'on sépare en ses isomères E ou Z ou transforme les isomères Z en isomères E et produits de formule (v) que, si nécessaire ou si désiré, l'on soumet à une ou plusieurs des réactions suivantes, dans un ordre quelconque : a) coupure par hydrolyse ou par action de la thiourée de tout ou partie des groupements esters ou des groupements de protection du radical amino ou des radicaux hydroxyles,
b) estérification ou salification par une base du ou des radicaux carboxyliques,
c) salification par un acide du radical amino,
d) séparation des produits sous forme de mélange R,S en R ou S.

2. Procédé de préparation selon la revendication 1 caractérisé en ce que le réactif capable d'introduire le radical R₁ est choisi parmi les réactifs de formule :

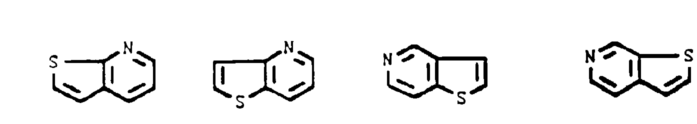

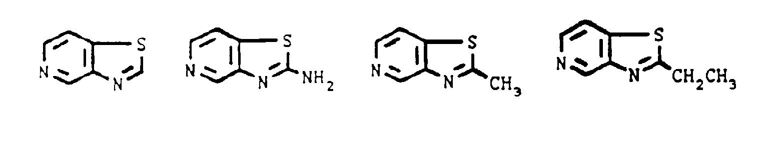

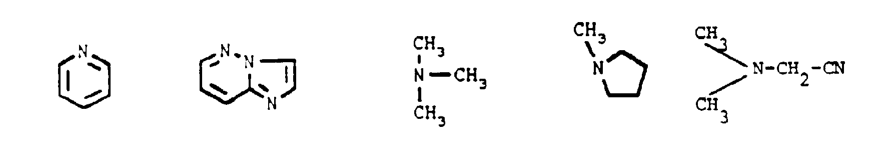

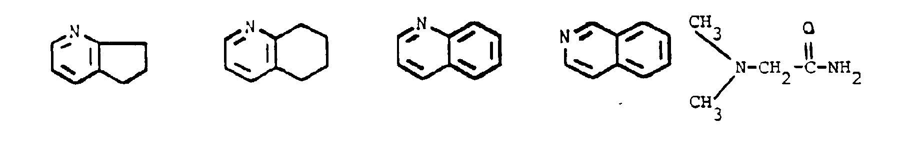

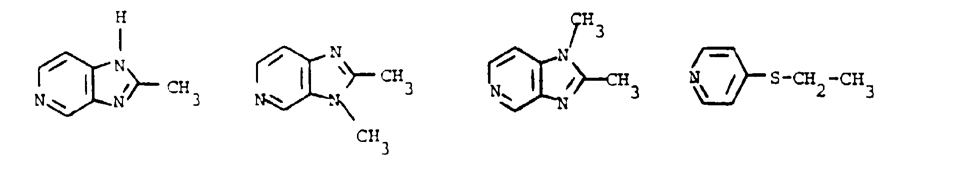

3. Procédé de préparation selon la revendication 1 ou 2, caractérisé en ce que le réactif capable d'introduire le radical R₁ est choisi parmi les réactifs : 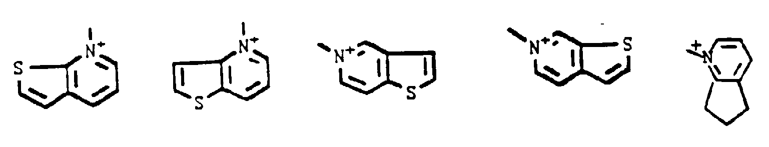 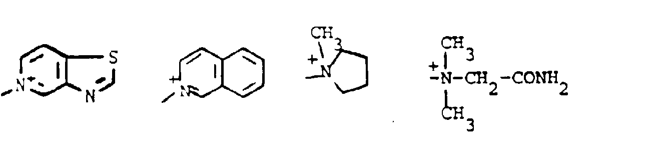 de préférence:

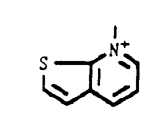

4. Procédé selon la revendication 1 pour la préparation des produits de formule (I) telle que définie à la revendication 1 répondant à la formule (I') : 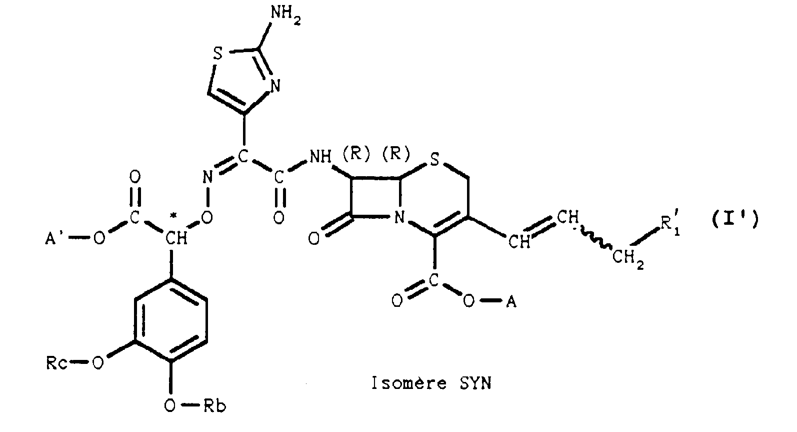 isomère sym, sous forme R ou S ou d'un mélange R, S, formule dans laquelle :
A, A', R_{b} et R_{c} conservent leur signification précédente et R'₁ représente un radical choisi parmi les radicaux suivants 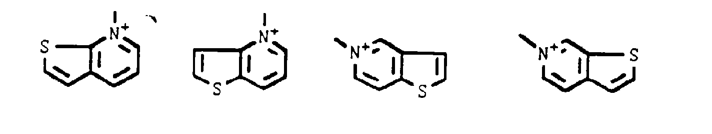 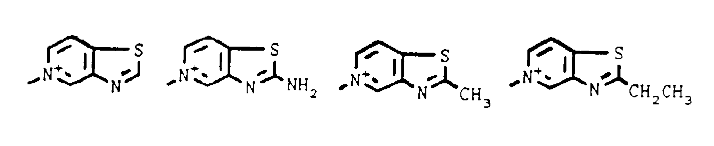 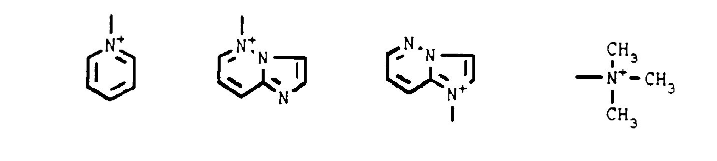 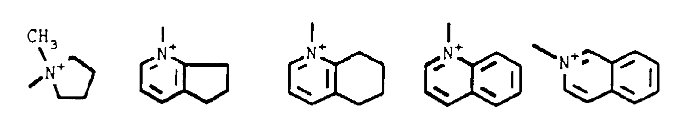 ainsi que les sels des produits de formule (I') avec les acides minéraux ou organiques, caractérisé en ce que l'on fait agir un produit de formule (II) sur un produit de formule (III) définis comme à la revendication 1 puis fait agir sur le produit de formule (IV) obtenu un réactif choisi parmi les réactifs de formule 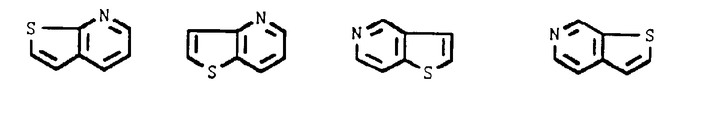 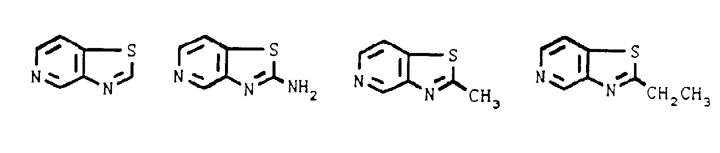 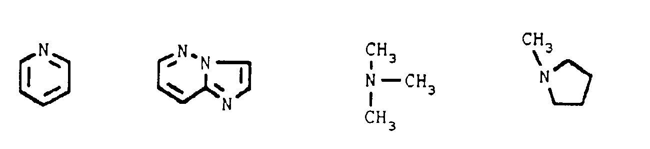 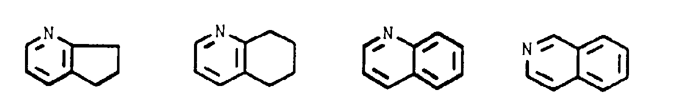 pour obtenir un produit de formule (V') : 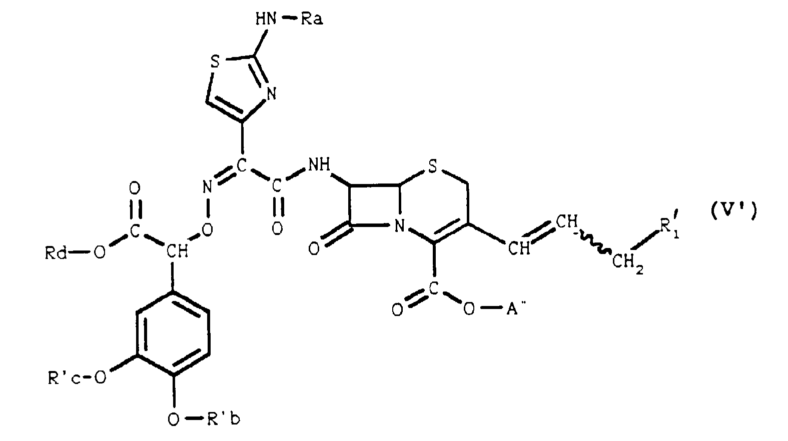 que, si désiré, l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :
a) coupure par hydrolyse ou par action de la thiourée de tout ou partie des groupements esters ou des groupements de protection du radical amino ou des radicaux hydroxyles,
b) estérification ou salification par une base du ou des radicaux carboxyliques,
c) salification par un acide du radical amino,
d) séparation des produits sous forme de mélange R,S en R ou S.

5. Procédé selon la revendication 4, caractérisé en ce que le réactif utilisé sur le produit de formule (IV) est choisi parmi les réactifs : 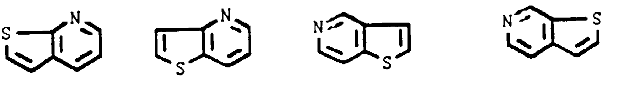 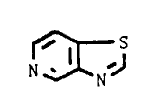 de préférence :

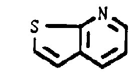

## Revendications (Revendications pour l'(les) Etat(s) contractant(s) suivant(s) : GR)

1. Procédé pour préparer les produits de formule générale (I) : 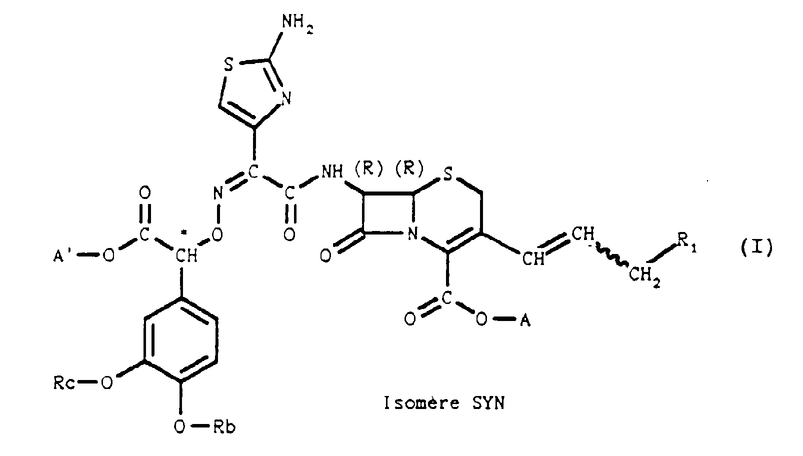 isomère sym, sous forme R ou S ou d'un mélange R, S, formule dans laquelle: R₁ représente un radical choisi parmi les radicaux suivants : 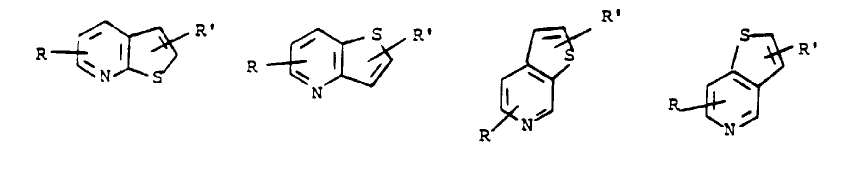 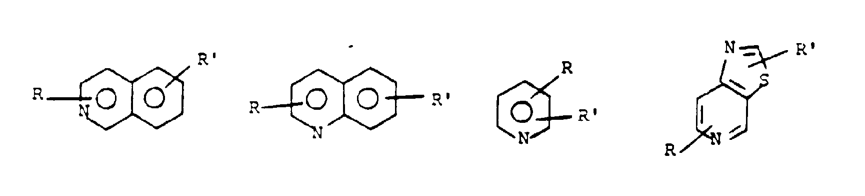 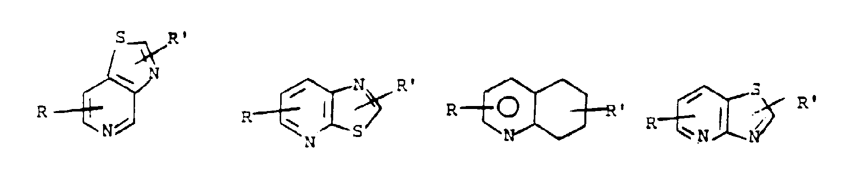 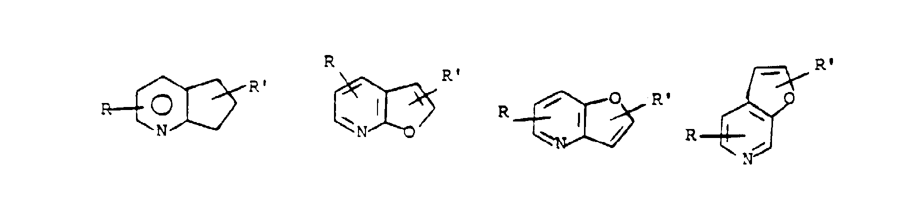 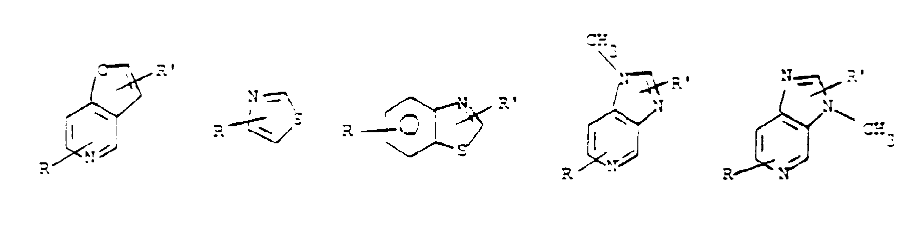 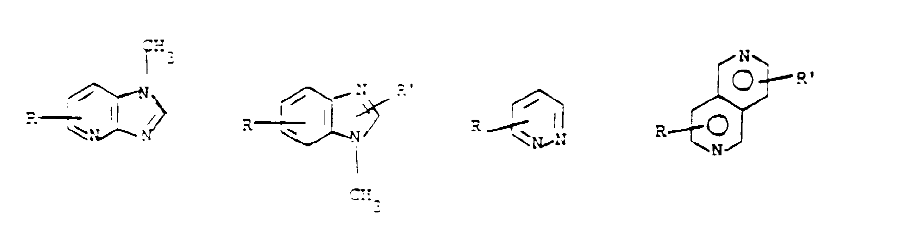 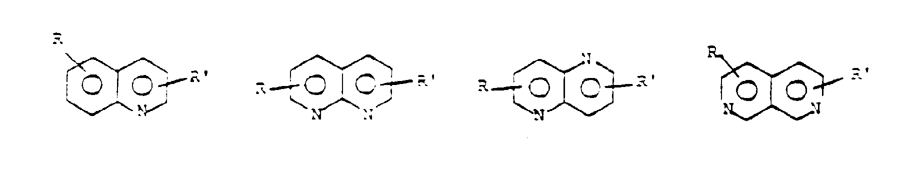 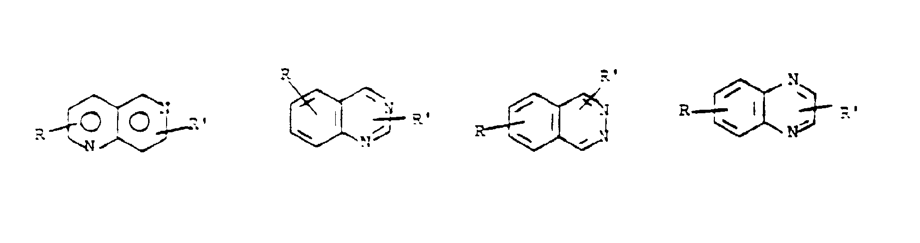 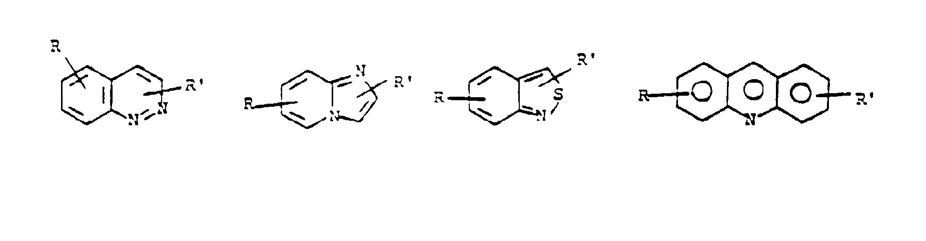 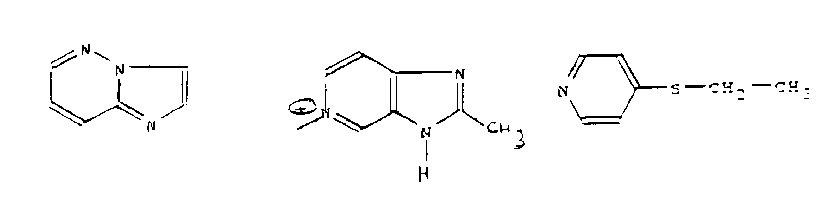 ou 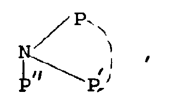 sous forme d'ammonium quaternaire, l'expression sous forme d'ammonium quartenaire indiquant que le radical R₁ est lié avec le groupement -CH=CH-CH₂- par le ou l'un des atomes d'azote qu'il comporte, dans lesquels R et R' identiques ou différents représentent un atome d'hydrogène, un radical alkyle renfermant de 1 à 4 atomes de carbone, un radical alcoxy renfermant de 1 à 4 atomes de carbone, un atome d'halogène, un radical CO₂-Q, 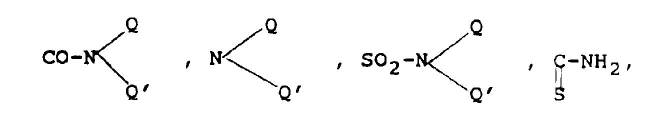 NH-CO-Q, CN, CH₂-CN, CH₂-SQ dans lesquels Q et Q' identiques ou différents représentent un atome d'hydrogène ou un radical alkyle renfermant de 1 à 4 atomes de carbone, P, P' et P" identiques ou différents représentent un radical alkyle renfermant au plus 4 atomes de carbone, éventuellement substitué par un des substituants indiqués ci-dessus pour R et R', le symbole) indiqant que P et P' peuvent éventuellement former avec l'atome d'azote auquel ils sont liés, un hétérocycle à 5 ou 6 chaînons
R_{b} et R_{c}, identiques ou différents représentent un atome d'hydrogène ou un groupement acyle, choisi parmi les radicaux acétyle, propionyle et benzoyle,
A et A'identiques ou différents représentent un atome d'hydrogène, un équivalent de métal alcalin, alcalino-terreux, de magnésium, d'ammonium ou d'une base organique aminée ou A et A' représentent le reste d'un groupement ester facilement clivable ou C0₂A représente CO₂⁻; le trait ondulé signifie que le groupement CH₂R₁ peut se trouver dans la position E ou Z ainsi que les sels des produits de formule (1) avec les acides minéraux ou organiques, caractérisé en ce que l'on fait agir un produit de formule (II) : 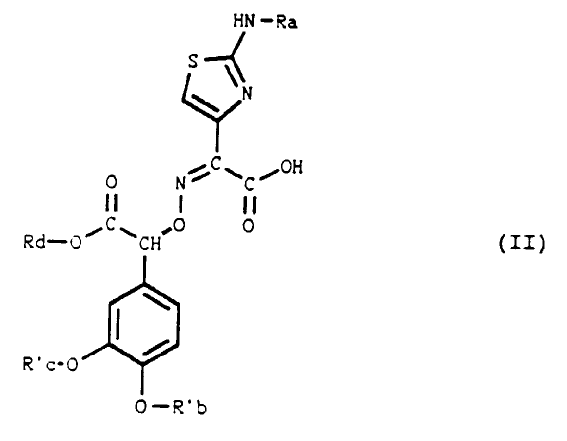 isomère syn, racémique ou optiquement actif ou un dérivé fonctionnel du produit de formule (II), dans laquelle Rₐ représente un atome d'hydrogène ou un groupement protecteur du radical amino, R'_{b} et R'_{c} identiques ou différents représentent un atome d'hydrogène ou un groupement protecteur du radical hydroxyle, R_{d} représente un atome d'hydrogène ou le reste d'un groupement ester facilement éliminable, avec un produit de formule (III) : 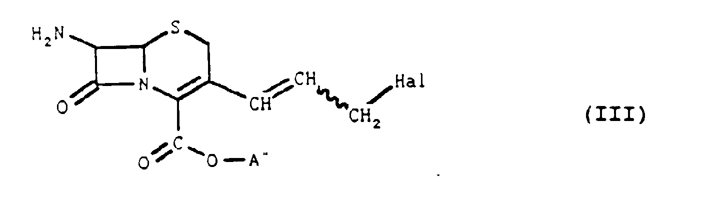 dans laquelle Hal représente un atome d'halogène, A" représente un atome d'hydrogène ou le reste d'un groupement ester facilement éliminable et le trait ondulé signifie que le groupement CH₂Hal peut se trouver dans la position E ou Z pour obtenir un produit de formule (IV) : 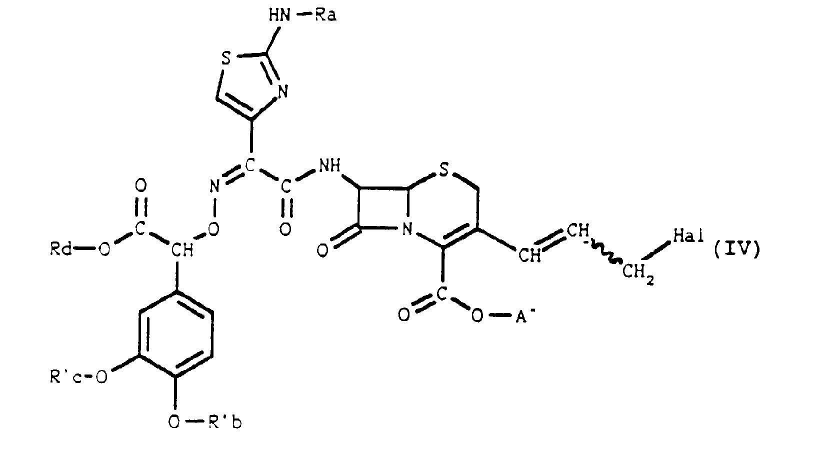 que l'on fait agir avec un réactif capable d'introduire le radical R₁ pour obtenir un produit de formule (V) : 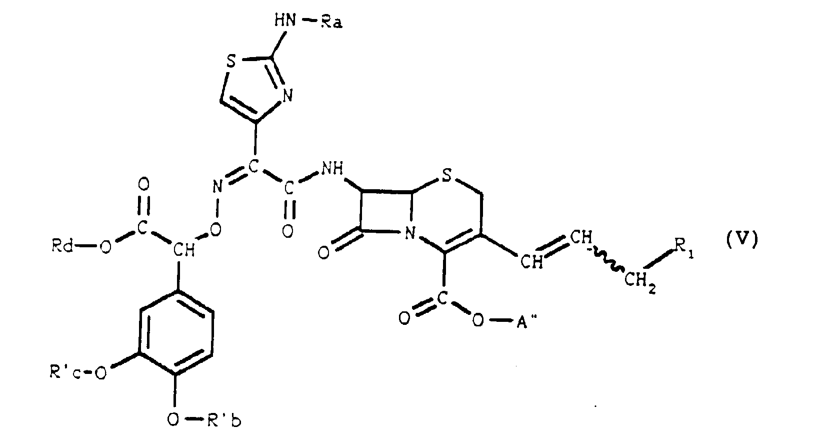 que, si désiré, l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :
a) coupure par hydrolyse ou par action de la thiourée de tout ou partie des groupements esters ou des groupements de protection du radical amino ou des radicaux hydroxyles,
b) estérification ou salification par une base du ou des radicaux carboxyliques,
c) salification par un acide du radical amino,
d) séparation des produits sous forme de mélange R,S en R ou S.

2. Procédé de préparation selon la revendication 1 caractérisé en ce que le réactif capable d'introduire le radical R, est choisi parmi les réactifs de formule :

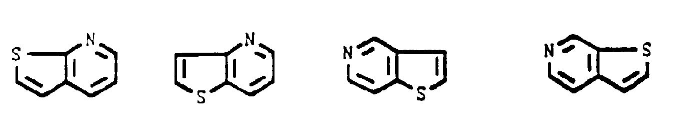

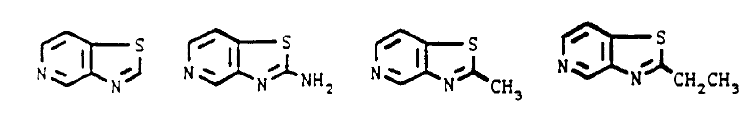

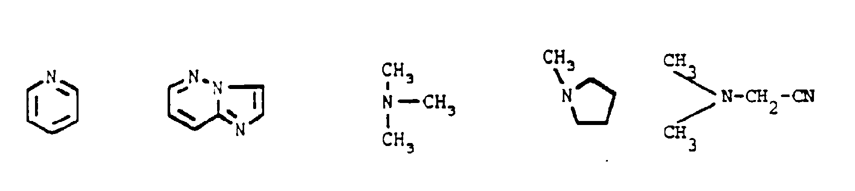

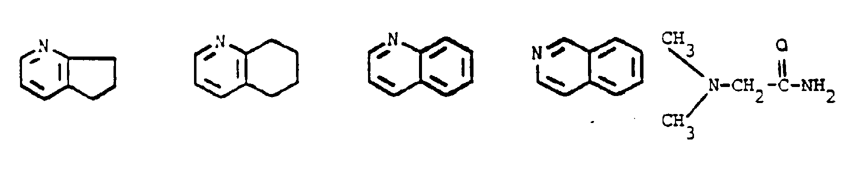

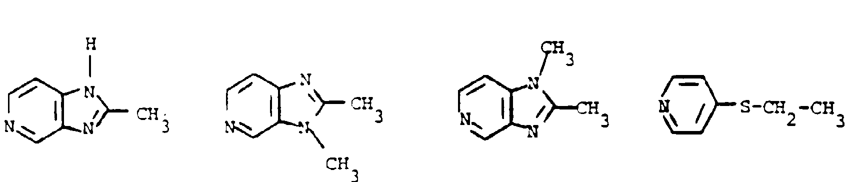

3. Procédé de préparation selon la revendication 1 ou 2, caractérisé en ce que le réactif capable d'introduire le radical R₁ est choisi parmi les réactifs : 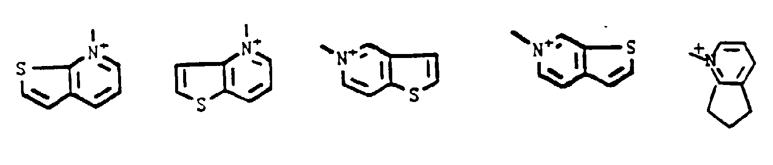 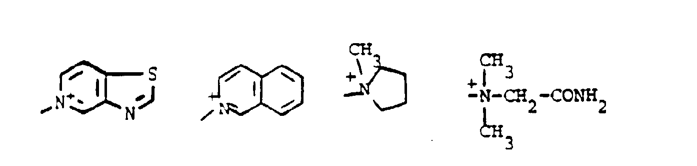 de préférence:

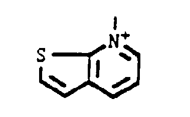

4. Procédé selon la revendication 1 pour la préparation des produits de formule (I) telle que définie à la revendication 1 répondant à la formule (I') : 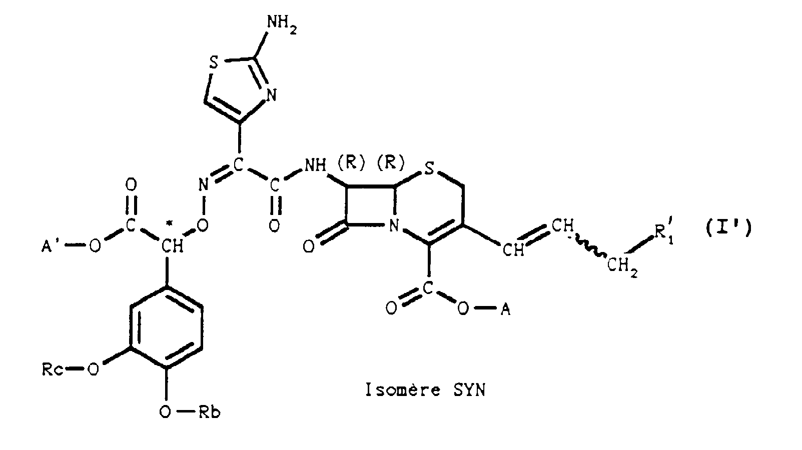 isomère sym, sous forme R ou S ou d'un mélange R, S, formule dans laquelle :
A, A', R_{b} et R_{c} conservent leur signification précédente et R'₁ représente un radical choisi parmi les radicaux suivants 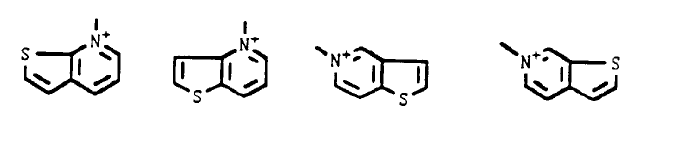 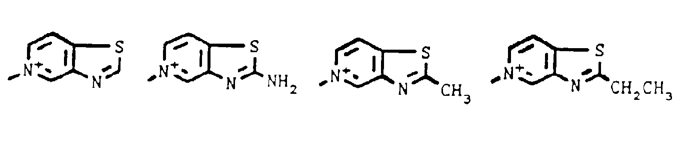 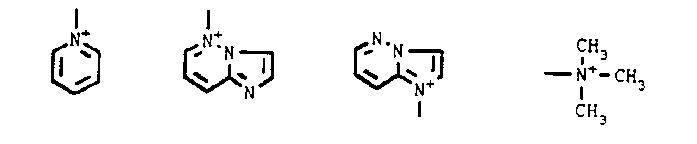 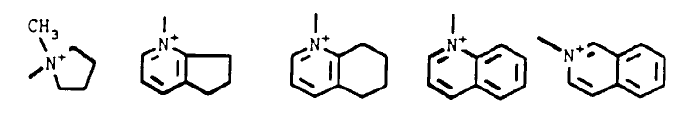 ainsi que les sels des produits de formule (I') avec les acides minéraux ou organiques, caractérisé en ce que l'on fait agir un produit de formule (II) sur un produit de formule (III) définis comme à la revendication 1 puis fait agir sur le produit de formule (IV) obtenu un réactif choisi parmi les réactifs de formule 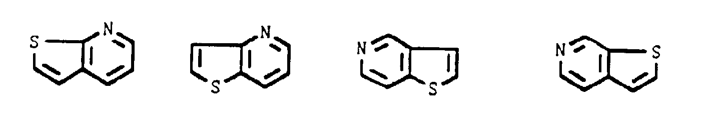 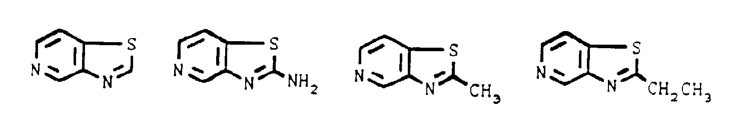 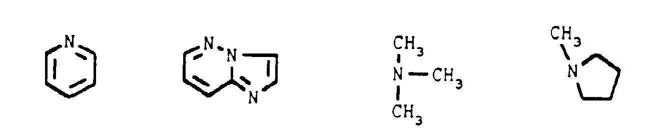 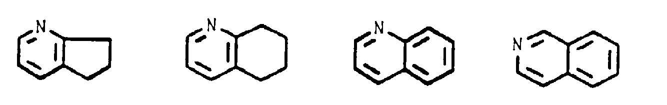 pour obtenir un produit de formule (V) : 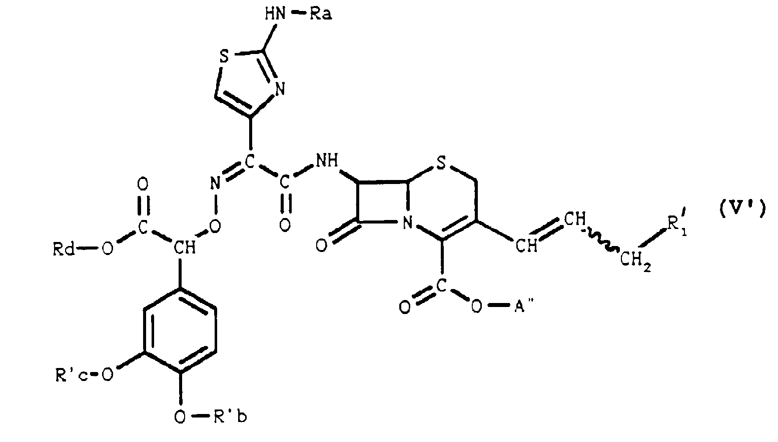 que, si désiré, l'on sépare en ses isomères E ou Z ou transforme les isomères Z en isomères E et produits de formule (V) que, si nécessaire ou si désiré, l'on soumet à une ou plusieurs des réactions suivantes, dans un ordre quelconque :
a) coupure par hydrolyse ou par action de la thiourée de tout ou partie des groupements esters ou des groupements de protection du radical amino ou des radicaux hydroxyles,
b) estérification ou salification par une base du ou des radicaux carboxyliques,
c) salification par un acide du radical amino,
d) séparation des produits sous forme de mélange R,S en R ou S.

5. Procédé selon la revendication 4, caractérisé en ce que le réactif utilisé sur le produit de formule (IV) est choisi parmi les réactifs : 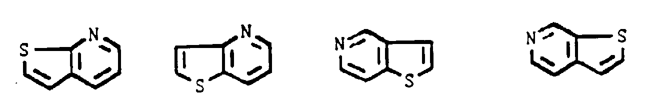 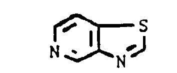 de préférence :

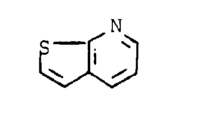

6. Procédé selon l'une quelconque des revendications 1 à 5, caractérisé en ce que les produits de départ et le réactif utilisés sont choisis de manière telle que l'on prépare :
le [6R-[3(E), 6alpha, 7béta(Z)]] 5-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] thiazolo[4,5-c] pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] 7-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] thiéno[2,3-b] pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables et particulièrement sous forme S,
- le [6R-[3(E), 6alpha, 7béta(Z)]] 2-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] isoquinoléinium sous forme R ou S ou d'un mélange. R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl]2-propényl] 1-méthyl pyrrolidinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] 1-[3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo[4,2,0]oct-2-en-3-yl] 2-propényl] 6,7-dihydro 5H-pyridinium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables,
le [6R-[3(E), 6alpha, 7béta(Z)]] N-(2-amino 2-oxoéthyl) 3-[7-[[(2-amino 4-thiazolyl) [[1-(3,4-dihydroxyphényl) 2-hydroxy 2-oxoéthoxy] imino] acétyl] amino] 2-carboxy 8-oxo 5-thia 1-azabicyclo-[4,2,0]oct-2-en-3-yl] N,N-diméthyl 2-propèn-1-aminium sous forme R ou S ou d'un mélange R,S et sous forme de sel interne ou de sel avec les métaux alcalins, alcalino-terreux, le magnésium, l'ammoniaque, les bases organiques aminées, les acides et ses esters facilement clivables.

7. Procédé de préparation de compositions pharmaceutiques caractérisé en ce que l'on met à titre de principe actif l'un au moins des dérivés de formule (I) tels que définis à la revendication 1 ainsi que leurs sels d'acides pharmaceutiquement acceptables, sous une forme destinée à cet usage.

8. Procédé de préparation de compositions pharmaceutiques caractérisé en ce que l'on met à titre de principe actif l'un au moins des dérivés de formule (l') tels que définis à la revendication 4 ainsi que leurs sels d'acides pharmaceutiquement acceptables, sous une forme destinée à cet usage.

9. Procédé de préparation de compositions pharmaceutiques caractérisé en ce que l'on met à titre de principe actif l'un au moins des dérivés de formule (I) tels que définis à la revendication 6 ainsi que leurs sels d'acides pharmaceutiquement acceptables, sous une forme destinée à cet usage.

10. A titre de produits industriels, les produits de formule (IV) et les produits de formule (V) dans laquelle Rₐ représente un groupement protecteur du radical amino, les formules (IV) et (V) étant telles que définies à la revendication 5.

## Patentansprüche (Patentansprüche für folgende(n) Vertragsstaat(en) : AT, BE, CH, DE, DK, FR, GB, IT, LI, LU, NL, SE)

1. Verbindungen der allgemeinen Formel (I) : 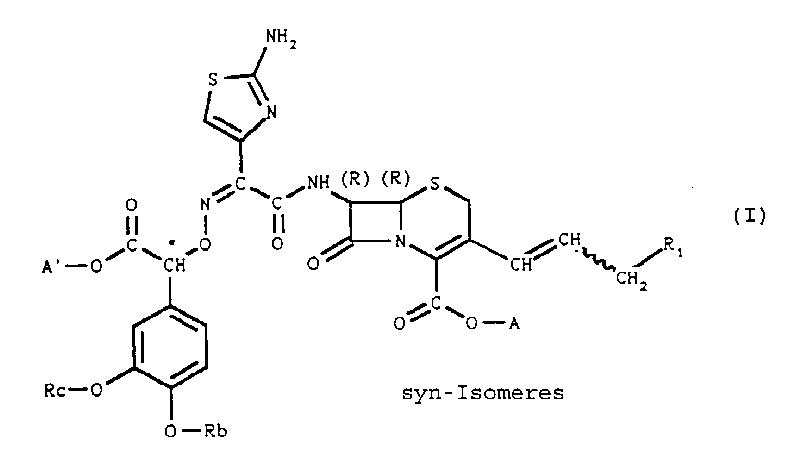 syn-Isomeres, in der R- oder S-Form oder in Form eines R,S-Gemisches, wobei in der Formel:
R₁ einen unter folgenden Resten ausgewählten Rest bedeutet: 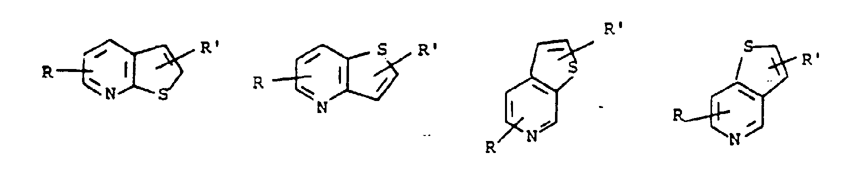 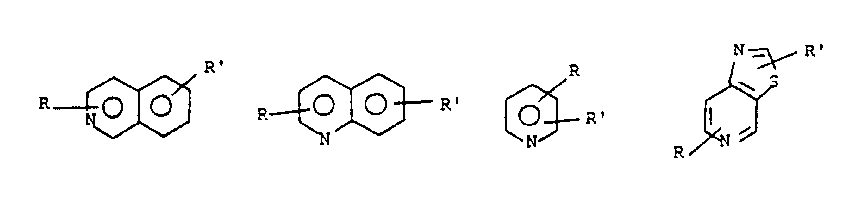 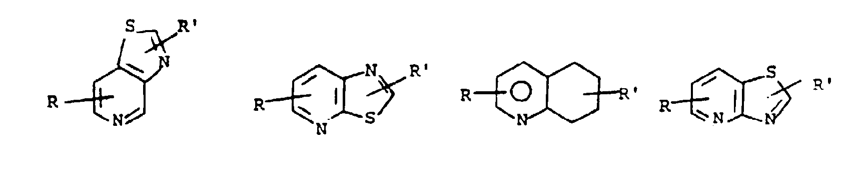 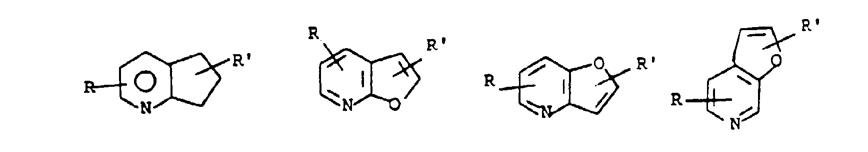 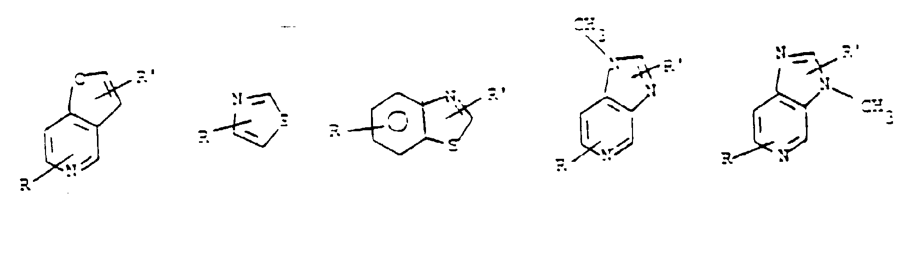 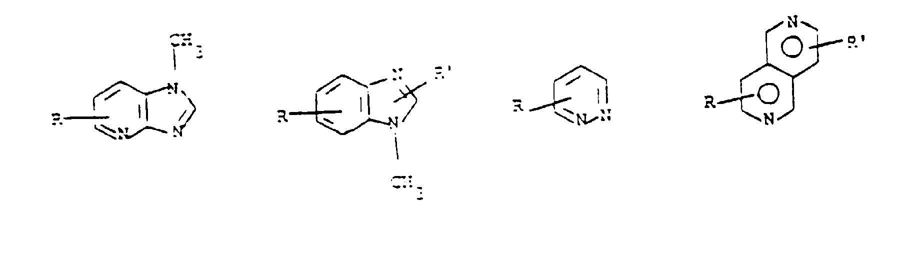 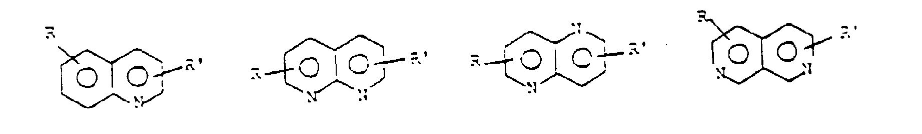 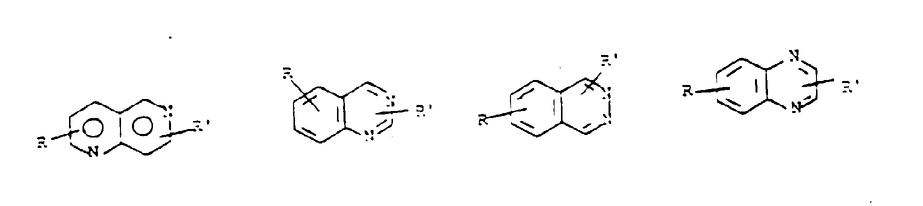 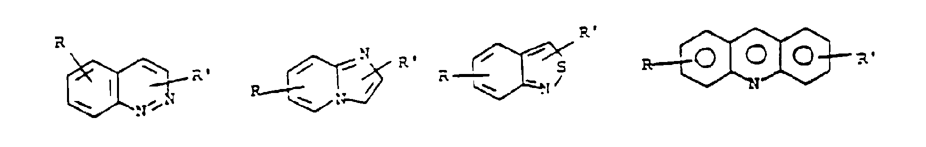 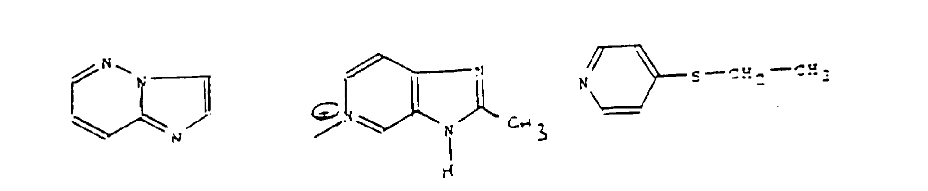 oder 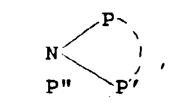 in der Form von quaternärem Ammonium, wobei der Ausdruck "in der Form von quaternärem Ammonium" bedeutet, daß der Rest R₁ mit der Gruppe -CH=CH-CH₂- über das Stickstoffatom oder über eines der Stickstoffatome, die er aufweist, gebunden ist,
worin R und R' gleich oder verschieden sind und ein Wasserstoffatom, einen Alkylrest mit 1 bis 4 Kohlenstoffatomen, einen Alkoxyrest mit 1 bis 4 Kohlenstoffatomen, ein Halogenatom, einen Rest der Formeln CO₂-Q, 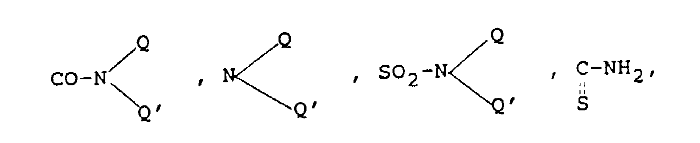 NH-CO-Q, CN, CH₂-CN und CH₂-SQ bedeuten, wobei Q und Q' gleich oder verschieden sind und ein Wasserstoffatom oder einen Alkylrest mit 1 bis 4 Kohlenstoffatomen bedeuten, P, P' und P" gleich oder verschieden sind und einen Alkylrest mit höchstens 4 Kohlenstoffatomen bedeuten, der ggf. durch einen der vorstehend für R und R'angegebenen Substituenten substituiert ist, wobei das Symbol angibt, daß P und P' ggf. mit dem Stickstoffatom, an das sie gebunden sind, einen Heterocyclus mit 5 oder 6 Kettengliedern bilden können,
R_{b} und R_{c} gleich oder verschieden sind und ein Wasserstoffatom oder eine Acylgruppe bedeuten, die unter Acetyl-, Propionyl- und Benzoylresten ausgewählt ist,
A und A' gleich oder verschieden sind und ein Wasserstoffatom, ein Äquivalent eines Alkalimetalls, Erdalkalimetalls, von Magnesium, Ammonium oder einer organischen Aminbase bedeutet oder A und A' den Rest einer leicht spaltbaren Estergruppe bedeutet oder C0₂A die Bedeutung CO₂⁻ hat; wobei die Wellenlinie bedeutet, daß die Gruppe CH₂R₁ sich in der E- oder Z-Stellung befinden kann,
sowie die Salze der Verbindungen der Formel (I) mit anorganischen oder organischen Säuren.

2. Verbindungen der allgemeinen Formel (I) gemäß der Definitionen in Anspruch 1, wobei R₁ unter folgenden Resten ausgewählt ist:

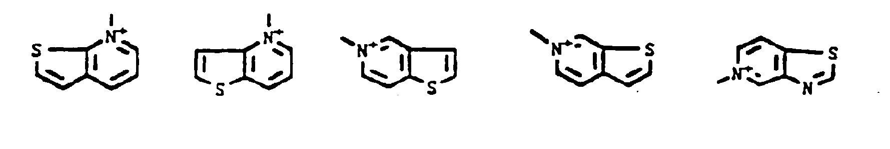

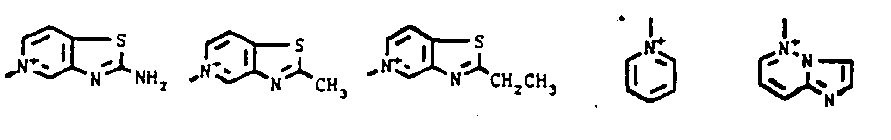

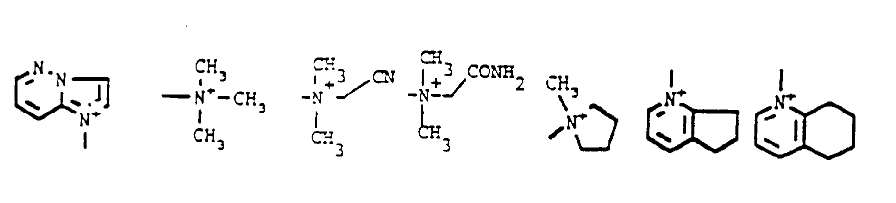

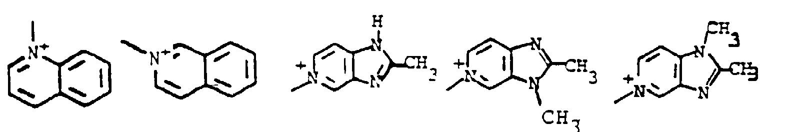

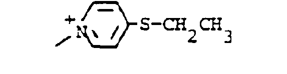

3. Verbindungen der allgemeinen Formel (I) gemäß der Definition in Anspruch 1 oder 2, wobei R₁ unter folgenden Resten ausgewählt ist:

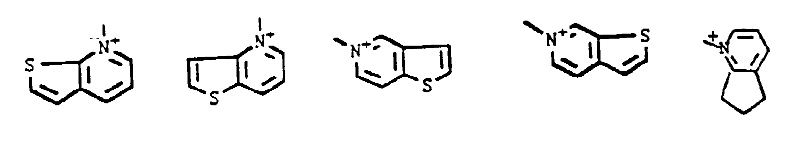

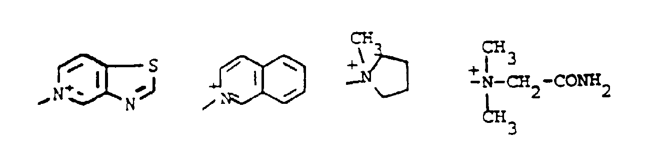

4. Verbindungen der allgemeinen Formel (I) gemäß der Definition in Anspruch 3, wobei Rᵢ den folgenden Rest bedeutet:

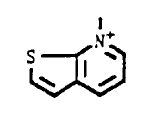

5. Verbindungen der allgemeinen Formel (I) nach Anspruch 1 mit folgenden Bezeichnungen:
- [6R-[3(E),6α,7β(Z)]]-5-[3-[7-[[(2-Amino-4-thiozolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-thiazolo[4,5-c]pyridinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon,
- [6R-[3(E),6α,7β(Z)]]-7-[3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-thieno[2,3-b]pyridinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon und insbesondere in der S-Form,
- [6R-3(E),6a,7ß(Z)]]-2-[3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2.0]oct-2-en-3-yl]-2-propenyl]-isochinolinium in der R-oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon,
- [6R-[3(E),6α,7β(Z)]]-1-[3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-1-methylpyrrolidinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon,
- [6R-[3(E),6α,7β(Z)]]-1-(3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-6,7-dihydro-5H-pyridinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon und
- [6R[3(E),6α,7β(Z)]]-N-(2-Amino-2-oxoethyl)-3-[7-[[(2-amino-4-thiazolyl)-[[1-(3,4-dihydroxyphenyl)-2-hydroxy-2-oxoethoxy]-imino]-acetyl]-amino]-2-carboxy-8-oxo-5-thia-1-azabicylo[4,2,0]oct-2-en-3-yl]-N,N-dimethyl-2-propen-1-aminium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon.

6. Verfahren zur Herstellung der Verbindung in der Formel (I) gemäß der Definition in Anspruch 1, dadurch gekennzeichnet, daß man eine Verbindung der Formel (II) 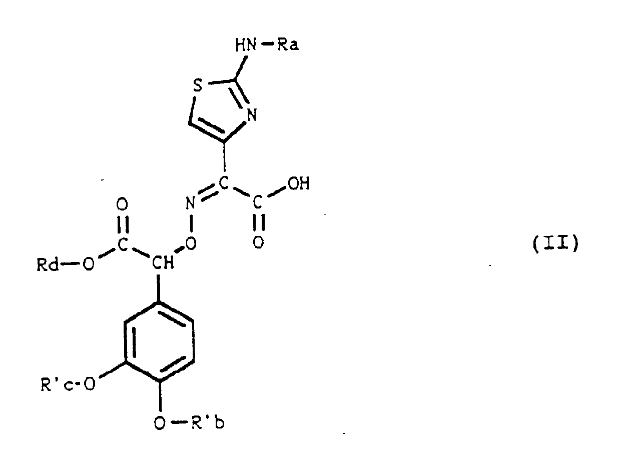 syn-Isomeres, racemisch oder optisch aktiv oder ein funktionelles Derivat der Verbindung der Formel (II), wobei Rₐ ein Wasserstoffatom oder eine Aminoschutzgruppe bedeutet, R'_{b} und R'_{c} gleich oder verschieden sind und ein Wasserstoffatom oder eine Hydroxylschutzgruppe bedeuten, R_{d} ein Wasserstoffatom oder den Rest einer leicht zu beseitigenden Estergruppe bedeutet, mit einer Verbindung der Formel (III) umsetzt: 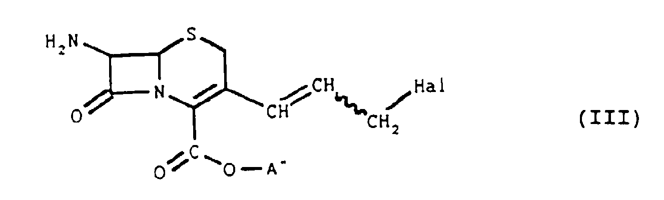 worin Hal ein Halogenatom bedeutet, A" ein Wasserstoffatom oder den Rest einer leicht zu beseitigenden Estergruppe bedeutet und die Wellenlinie bedeutet, daß die CH₂Hal-Gruppe sich in der E- oder Z-Stellung befinden kann, wodurch man eine Verbindung der Formel (IV) erhält: 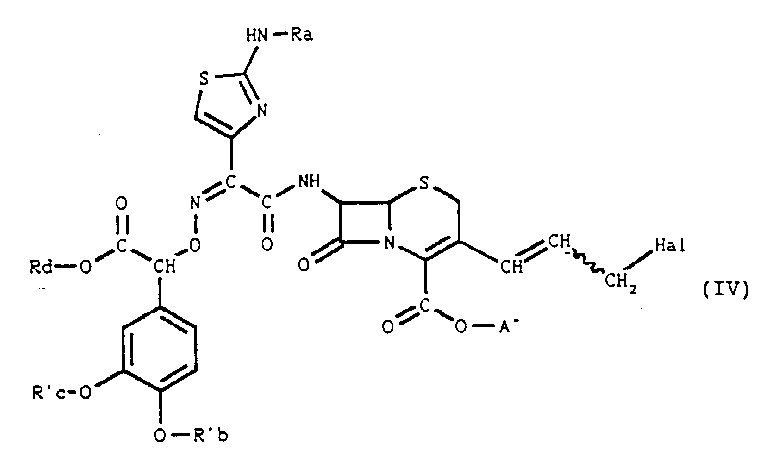 die man mit einem Reagenz, das zur Einführung des Restes R₁ befähigt ist, umsetzt, wodurch man eine Verbindung der Formel (V) erhält: 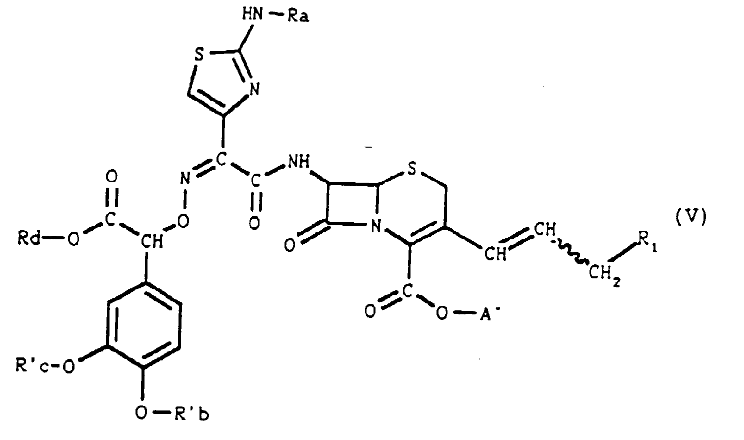 die man ggf. in ihre E- oder Z-Isomeren auftrennt, oder die Z-Isomeren in E-Isomere umwandelt, wobei man ggf. die Verbindungen der Formel (V) einer oder mehreren der folgenden Reaktionen in einer beliebigen Reihenfolge unterwirft:
a) Abspalten der Gesamtheit oder eines Teils der Estergruppen, der Aminoschutzgruppen oder der Hydroxylschutzgruppen durch Hydrolyse oder durch Einwirkung von Thioharnstoff,
b) Veresterung oder Salzbildung des oder der Carboxylreste mit einer Base,
c) Salzbildung des Aminorestes mit einer Säure und
d) Auftrennung der Produkte in Form des R,S-Gemisches in die R- oder S-Form.

7. Verfahren nach Anspruch 6, dadurch gekennzeichnet, daß das Reagenz, das zur Einführung des Restes R, befähigt ist, unter folgenden Reagenzien ausgewählt ist:

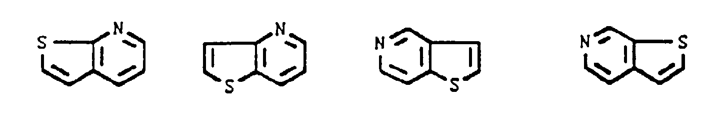

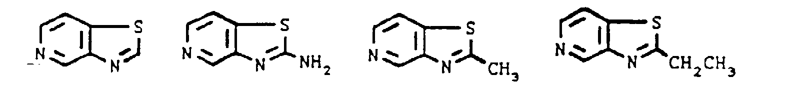

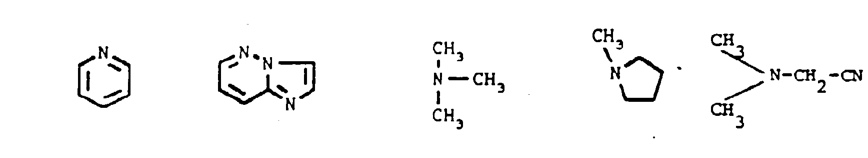

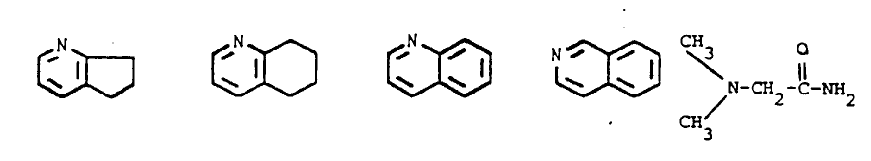

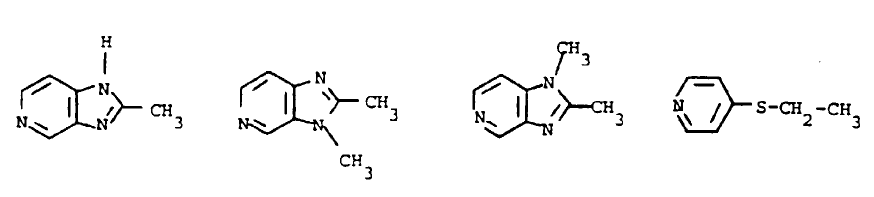

8. Verbindungen der Formel (I) gemäß der Definition in Anspruch 1 sowie deren pharmazeutisch verträgliche Salze mit Säuren als Arzneistoffe.

9. Verbindungen nach einem der Ansprüche 2 bis 5 sowie deren pharmazeutisch verträgliche Salze als Arzneistoffe.

10. Pharmazeutische Zusammensetzungen, enthaltend als Wirkstoff mindestens einen Arzneistoff nach einem der Ansprüche 8 oder 9.

11. Verbindungen der Formel (IV) und Verbindungen der Formel (V), worin Rₐ eine Aminoschutzgruppe bedeutet, wobei die Formeln (IV) und (V) der Definition in Anspruch 6 entsprechen, als gewerblich einzusetzende Verbindungen.

## Patentansprüche (Patentansprüche für folgende(n) Vertragsstaat(en) : ES)

1. Verfahren zur Herstellung der Verbindungen der allgemeinen Formel (I) : 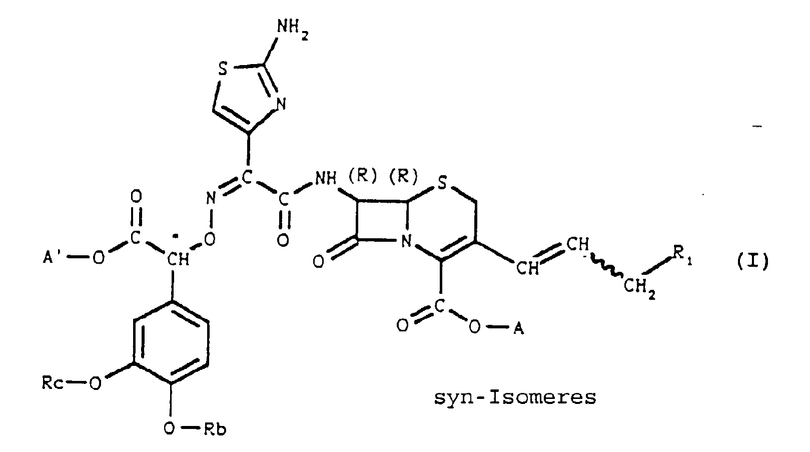 syn-Isomeres, in der R- oder S-Form oder in Form eines R,S-Gemisches, wobei in der Formel:
R₁ einen unter folgenden Resten ausgewählten Rest bedeutet: 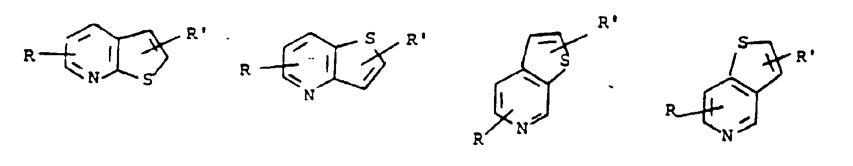 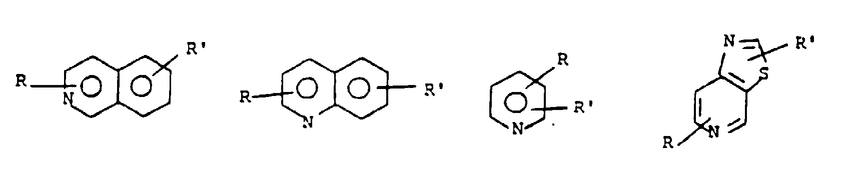 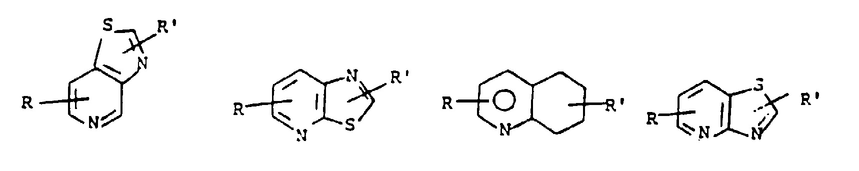 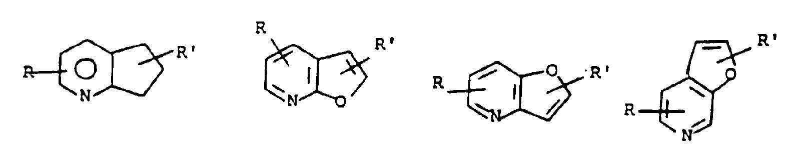 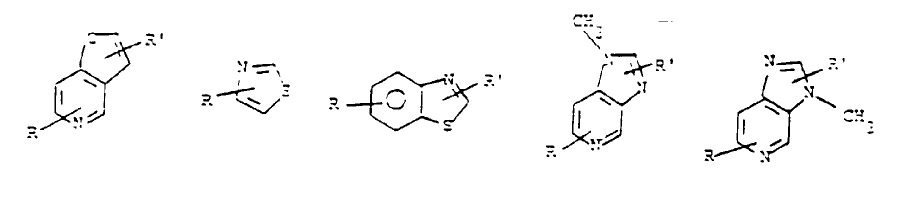 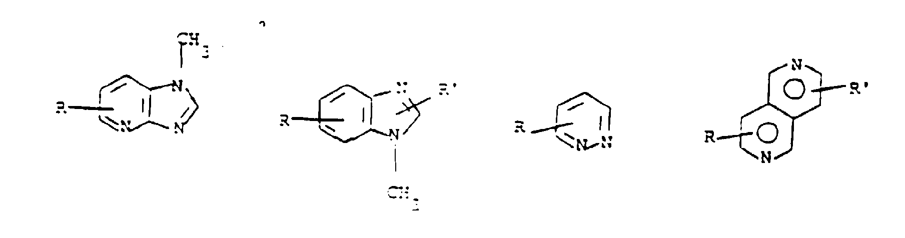 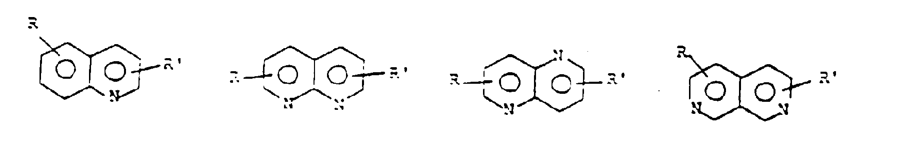 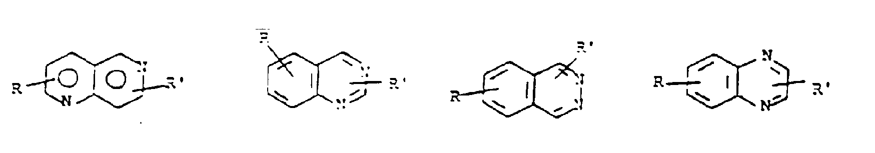 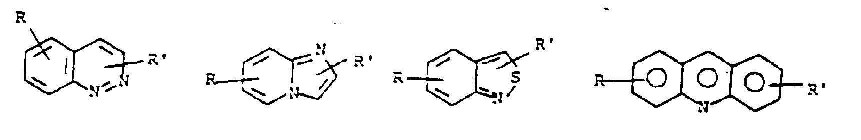 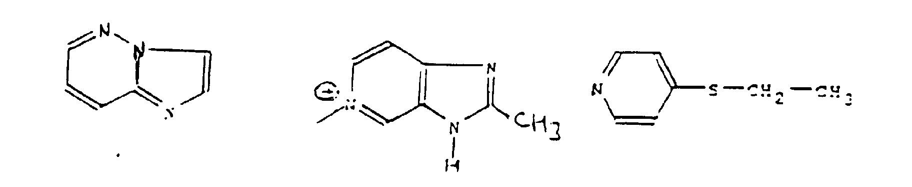 oder 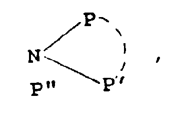 in der Form von quaternärem Ammonium, wobei der Ausdruck "in der Form von quaternärem Ammonium" bedeutet, daß der Rest R₁ mit der Gruppe -CH=CH-CH₂- über das Stickstoffatom oder über eines der Stickstoffatome, die er aufweist, gebunden ist,
worin R und R' gleich oder verschieden sind und ein Wasserstoffatom, einen Alkylrest mit 1 bis 4 Kohlenstoffatomen, einen Alkoxyrest mit 1 bis 4 Kohlenstoffatomen, ein Halogenatom, einen Rest der Formeln CO₂-Q, 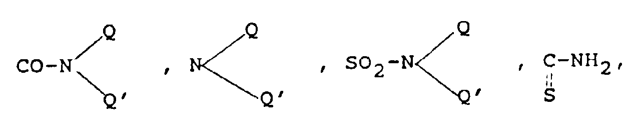 NH-CO-Q, CN, CH₂-CN und CH₂-SQ bedeuten, wobei Q und Q' gleich oder verschieden sind und ein Wasserstoffatom oder einen Alkylrest mit 1 bis 4 Kohlenstoffatomen bedeuten, P, P' und P" gleich oder verschieden sind und einen Alkylrest mit höchstens 4 Kohlenstoffatomen bedeuten, der ggf. durch einen der vorstehend für R und R' angegebenen Substituenten substituiert ist, wobei das Symbol angibt, daß P und P' ggf. mit dem Stickstoffatom, an das sie gebunden sind, einen Heterocyclus mit 5 oder 6 Kettengliedern bilden können,
R_{b} und R_{c} gleich oder verschieden sind und ein Wasserstoffatom oder eine Acylgruppe bedeuten, die unter Acetyl-, Propionyl- und Benzoylresten ausgewählt ist,
A und A' gleich oder verschieden sind und ein Wasserstoffatom, ein Äquivalent eines Alkalimetalls, Erdalkalimetalls, von Magnesium, Ammonium oder einer organischen Aminbase bedeutet oder A und A' den Rest einer leicht spaltbaren Estergruppe bedeutet oder C0₂A die Bedeutung CO₂⁻ hat; wobei die Wellenlinie bedeutet, daß die Gruppe CH₂R₁ sich in der E-oder Z-Stellung befinden kann,
sowie der Salze der Verbindungen der Formel (I) mit anorganischen oder organischen Säuren,
dadurch gekennzeichnet, daß man eine Verbindung der Formel (II) 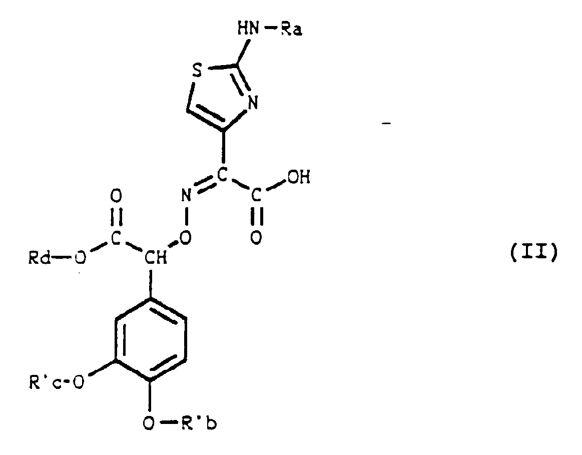 syn-Isomeres, racemisch oder optisch aktiv oder ein funktionelles Derivat der Verbindung der Formel (II), wobei Rₐ ein Wasserstoffatom oder eine Aminoschutzgruppe bedeutet, R'_{b} und R'_{c} gleich oder verschieden sind und ein Wasserstoffatom oder eine Hydroxylschutzgruppe bedeuten, R_{d} ein Wasserstoffatom oder den Rest einer leicht zu beseitigenden Estergruppe bedeutet, mit einer Verbindung der Formel (III) umsetzt: 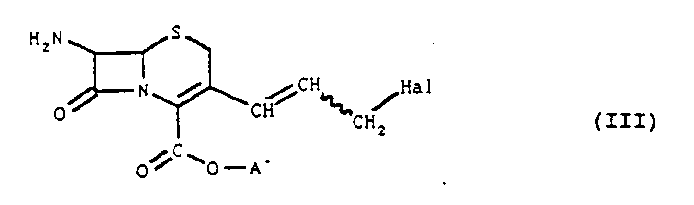 worin Hal ein Halogenatom bedeutet, A" ein Wasserstoffatom oder den Rest einer leicht zu beseitigenden Estergruppe bedeutet und die Wellenlinie bedeutet, daß die CH₂Hal-Gruppe sich in der E- oder Z-Stellung befinden kann, wodurch man eine Verbindung der Formel (IV) erhält: 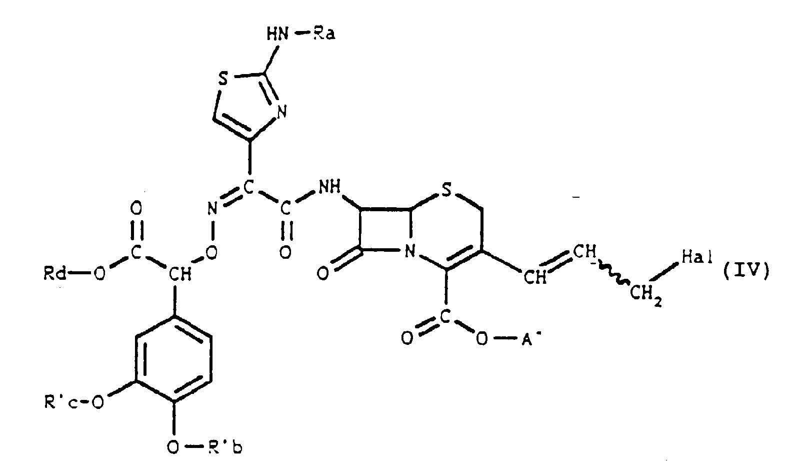 die man mit einem Reagenz, das zur Einführung des Restes R₁ befähigt ist, umsetzt, wodurch man eine Verbindung der Formel (V) erhält: 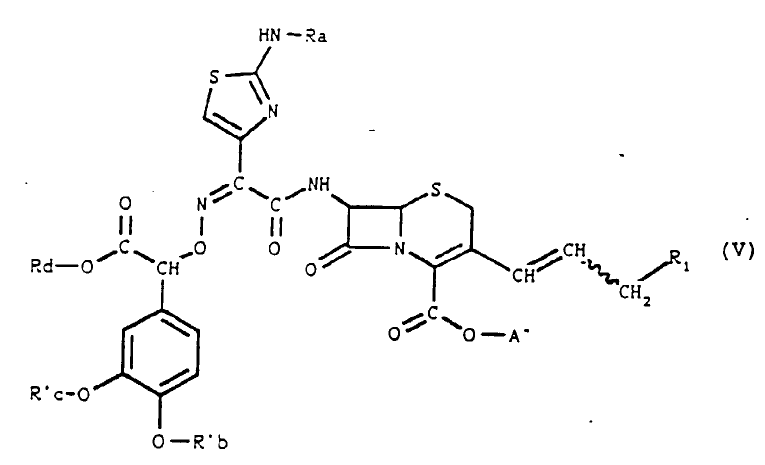 die man ggf. in ihre E- oder Z-Isomeren auftrennt, oder die Z-Isomeren in E-Isomere umwandelt, wobei man ggf. die Verbindungen der Formel (V) einer oder mehreren der folgenden Reaktionen in einer beliebigen Reihenfolge unterwirft:
a) Abspalten der Gesamtheit oder eines Teils der Estergruppen, der Aminoschutzgruppen oder der Hydroxylschutzgruppen durch Hydrolyse oder durch Einwirkung von Thioharnstoff,
b) Veresterung oder Salzbildung des oder der Carboxylreste mit einer Base,
c) Salzbildung des Aminorestes mit einer Säure und
d) Auftrennung der Produkte in Form des R,S-Gemisches in die R- oder S-Form.

2. Herstellungsverfahren nach Anspruch 1, dadurch gekennzeichnet, daß das Reagenz, das zur Einführung des Restes R, befähigt ist, unter Reagenzien der folgenden Formeln ausgewählt wird:

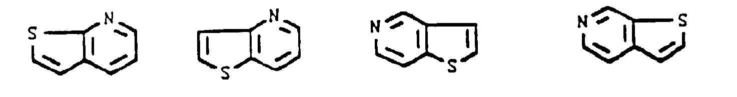

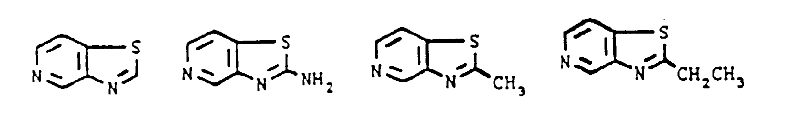

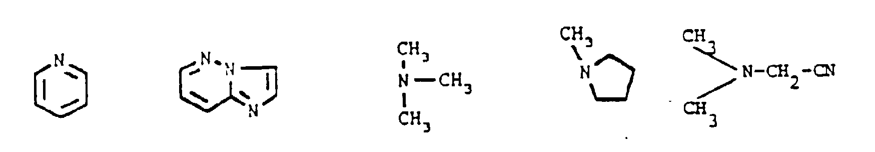

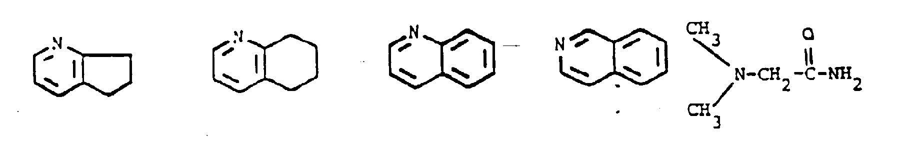

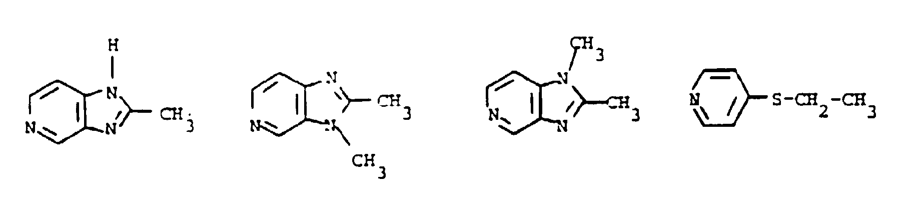

3. Herstellungsverfahren nach Anspruch 1 oder 2, dadurch gekennzeichnet, daß das Reagenz, das zur Einführung des Restes R₁ befähigt ist, unter folgenden Reagenzien ausgewählt wird: 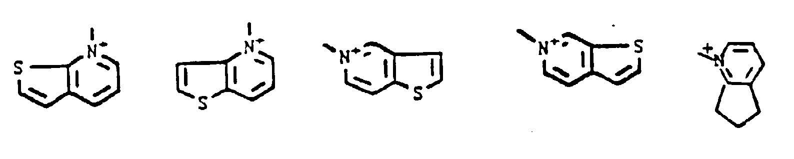 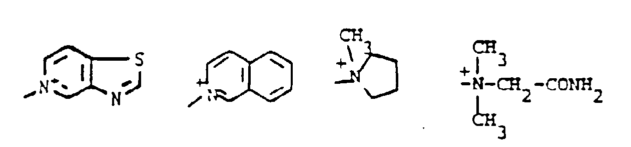 vorzugsweise:

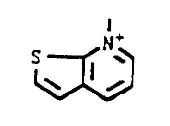

4. Verfahren nach Anspruch 1 zur Herstellung von Verbindungen der Formel (I) gemäß der Definition in Anspruch 1, entsprechend der Formel (I'): 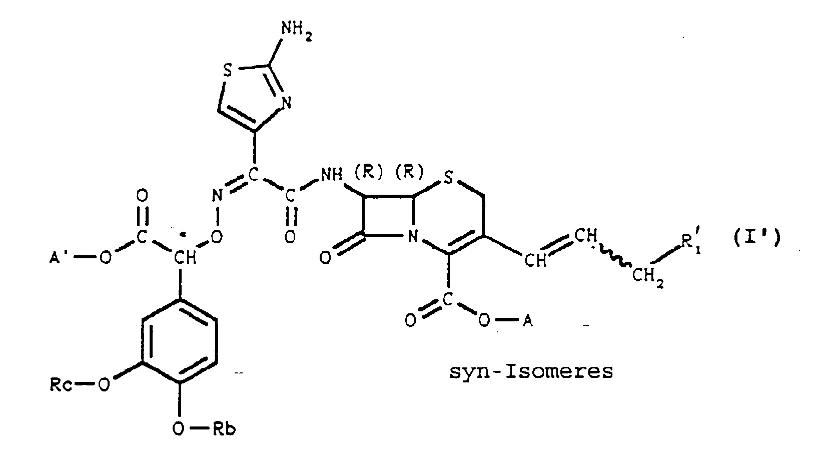 syn-Isomeres, in der R- oder S-Form oder in Form eines R,S-Gemisches, wobei in der Formel:
A, A', R_{b} und R_{c} eine unveränderte Bedeutung haben und R'₁ einen Rest bedeutet, der unter folgenden Resten ausgewählt ist: 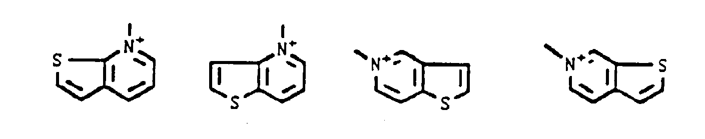 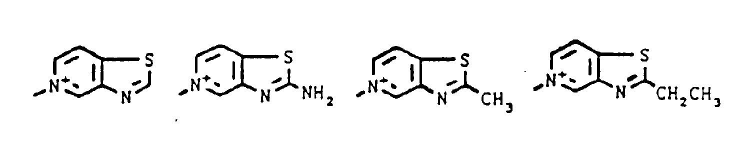 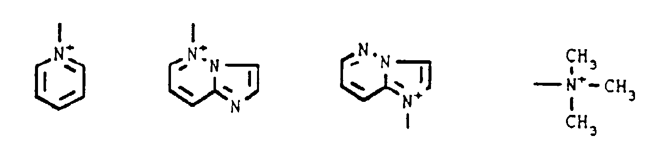 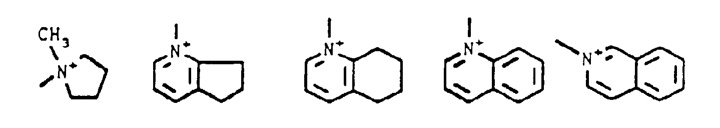 sowie von Salzen der Verbindungen der Formel (I') mit anorganischen oder organischen Säuren,
dadurch gekennzeichnet, daß man eine Verbindung der Formel (II) mit einer Verbindung der Formel (111), die der Definition in Anspruch 1 entsprechen, umsetzt, anschließend die erhaltene Verbindung der Formel (IV) mit einem Reagenz, das unter folgenden Reagenzien ausgewählt wird, umsetzt: 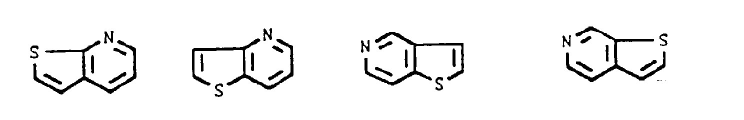 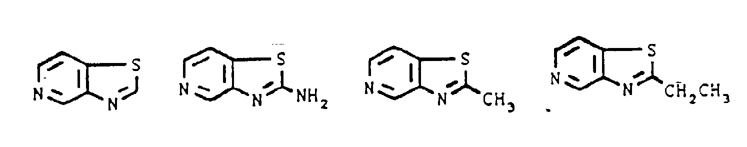 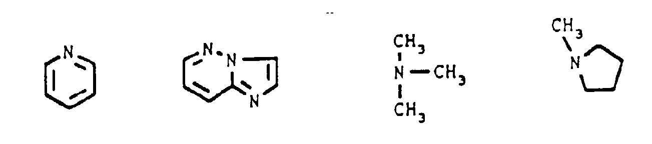 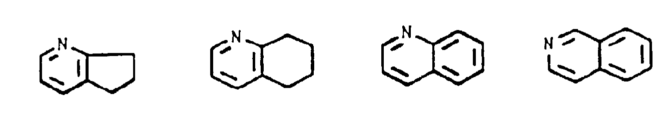 wodurch man eine Verbindung der Formel (V') erhält: 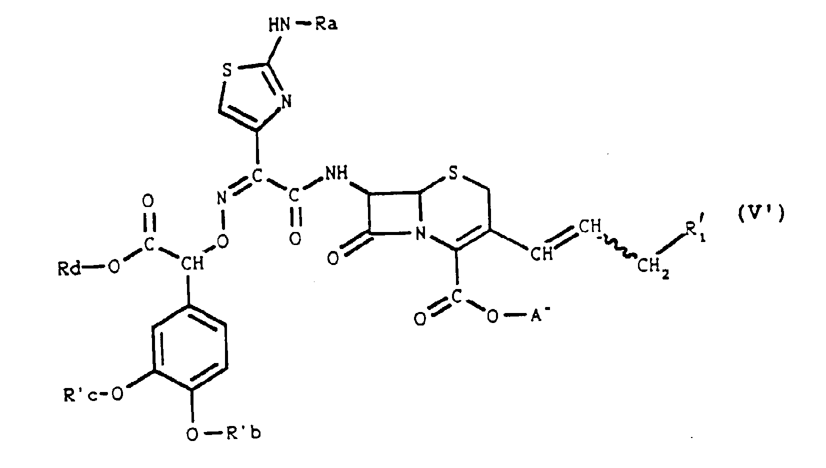 die man ggf. in ihre E- oder Z-Isomeren auftrennt, oder die Z-Isomeren in E-Isomere umwandelt, wobei man ggf. die Verbindungen der Formel (V) einer oder mehreren der folgenden Reaktionen in einer beliebigen Reihenfolge unterwirft:
a) Abspalten der Gesamtheit oder eines Teils der Estergruppen, der Aminoschutzgruppen oder der Hydroxylschutzgruppen durch Hydrolyse oder durch Einwirkung von Thioharnstoff,
b) Veresterung oder Salzbildung des oder der Carboxylreste mit einer Base,
c) Salzbildung des Aminorestes mit einer Säure und
d) Auftrennung der Produkte in Form des R,S-Gemisches in die R- oder S-Form.

5. Verfahren nach Anspruch 4, dadurch gekennzeichnet, daß das Reagenz, das zur Umsetzung mit der Verbindung der Formel (IV) verwendet wird, unter folgenden Reagenzien ausgewählt wird: 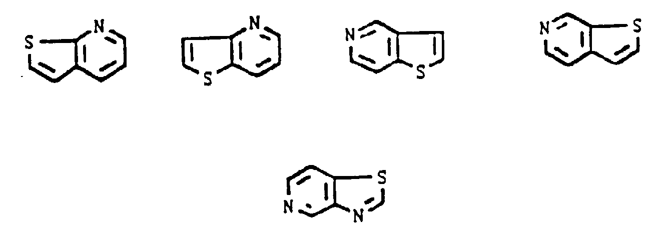 vorzugsweise:

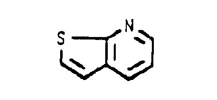

## Patentansprüche (Patentansprüche für folgende(n) Vertragsstaat(en) : GR)

1. Verfahren zur Herstellung der Verbindungen der allgemeinen Formel (I) : 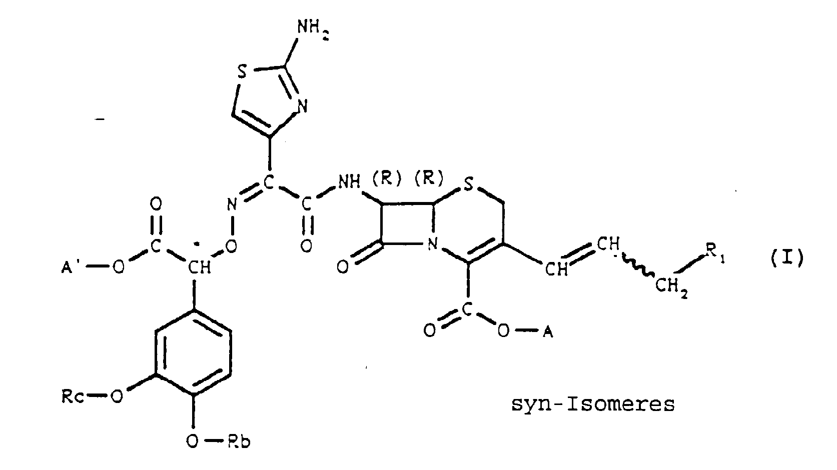 syn-Isomeres, in der R- oder S-Form oder in Form eines R,S-Gemisches, wobei in der Formel:
R₁ einen unter folgenden Resten ausgewählten Rest bedeutet: 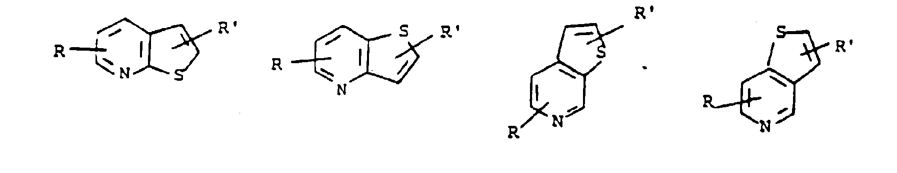 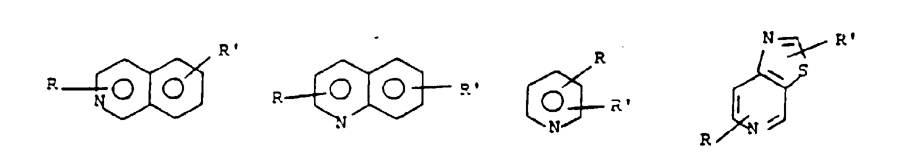 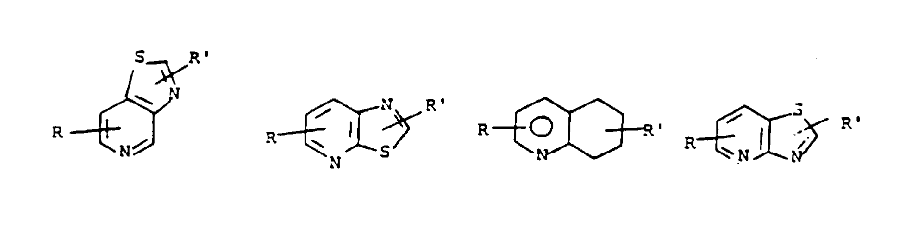 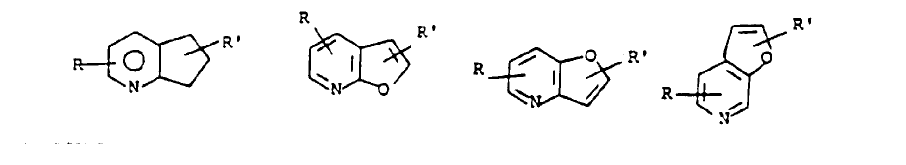 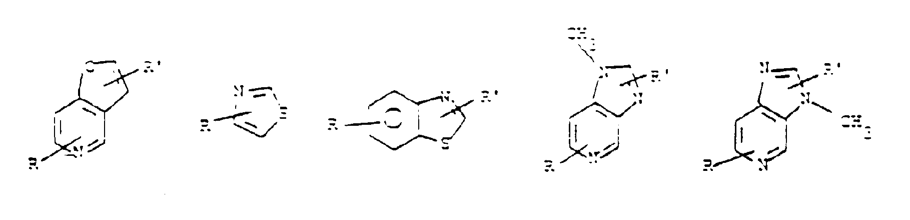 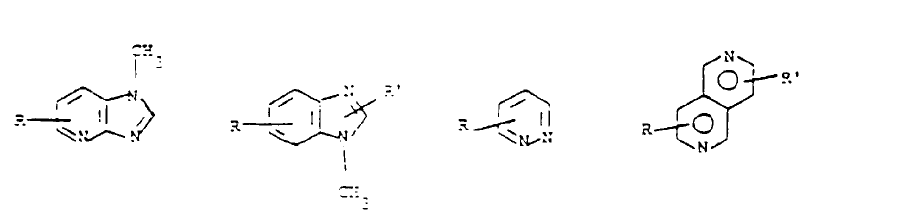 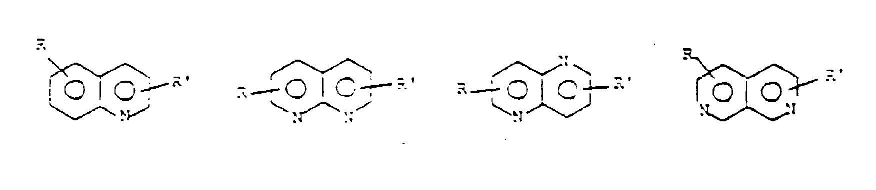 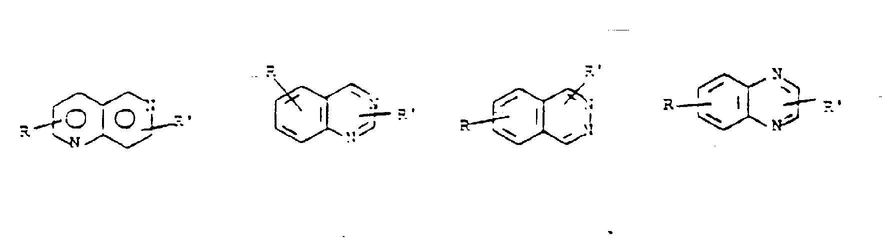 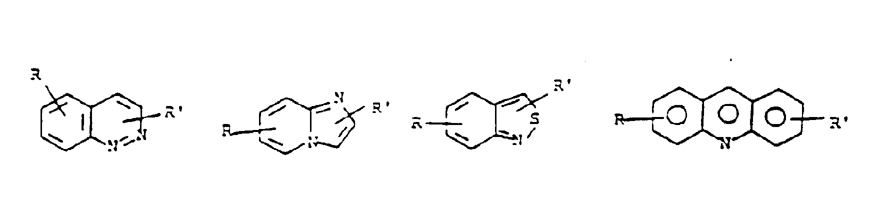 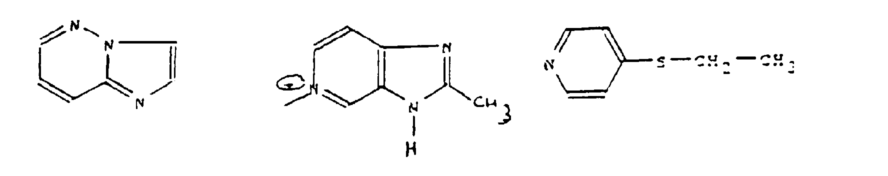 oder 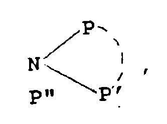 in der Form von quaternärem Ammonium, wobei der Ausdruck "in der Form von quaternärem Ammonium" bedeutet, daß der Rest R₁ mit der Gruppe -CH=CH-CH₂- über das Stickstoffatom oder über eines der Stickstoffatome, die er aufweist, gebunden ist,
worin R und R' gleich oder verschieden sind und ein Wasserstoffatom, einen Alkylrest mit 1 bis 4 Kohlenstoffatomen, einen Alkoxyrest mit 1 bis 4 Kohlenstoffatomen, ein Halogenatom, einen Rest der Formeln CO₂-Q, 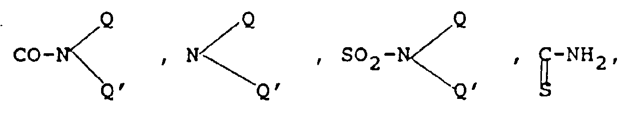 NH-CO-Q, CN, CH₂-CN und CH₂-SQ bedeuten, wobei Q und Q' gleich oder verschieden sind und ein Wasserstoffatom oder einen Alkylrest mit 1 bis 4 Kohlenstoffatomen bedeuten, P, P' und P" gleich oder verschieden sind und einen Alkylrest mit höchstens 4 Kohlenstoffatomen bedeuten, der ggf. durch einen der vorstehend für R und R' angegebenen Substituenten substituiert ist, wobei das Symbol angibt, daß P und P' ggf. mit dem Stickstoffatom, an das sie gebunden sind, einen Heterocyclus mit 5 oder 6 Kettengliedern bilden können,
R_{b} und Rₑ gleich oder verschieden sind und ein Wasserstoffatom oder eine Acylgruppe bedeuten, die unter Acetyl-, Propionyl- und Benzoylresten ausgewählt ist,
A und A' gleich oder verschieden sind und ein Wasserstoffatom, ein Äquivalent eines Alkalimetalls, Erdalkalimetalls, von Magnesium, Ammonium oder einer organischen Aminbase bedeutet oder A und A' den Rest einer leicht spaltbaren Estergruppe bedeutet oder C0₂A die Bedeutung CO₂⁻ hat; wobei die Wellenlinie bedeutet, daß die Gruppe CH₂R₁ sich in der E- oder Z-Stellung befinden kann,
sowie der Salze der Verbindungen der Formel (I) mit anorganischen oder organischen Säuren,
dadurch gekennzeichnet, daß man eine Verbindung der Formel (II) 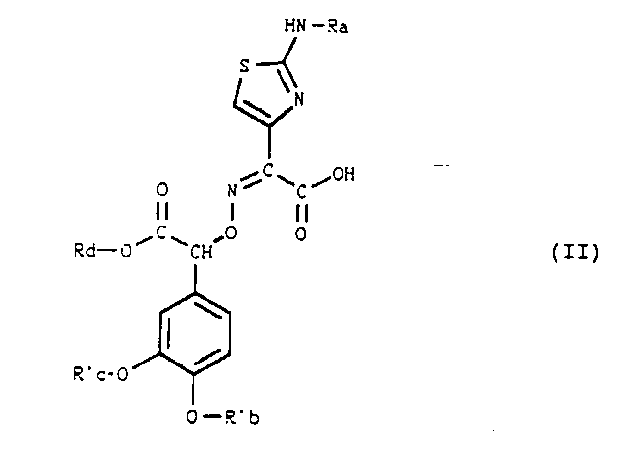 syn-Isomeres, racemisch oder optisch aktiv oder ein funktionelles Derivat der Verbindung der Formel (II), wobei Rₐ ein Wasserstoffatom oder eine Aminoschutzgruppe bedeutet, R'_{b} und R'_{c} gleich oder verschieden sind und ein Wasserstoffatom oder eine Hydroxylschutzgruppe bedeuten, R_{d} ein Wasserstoffatom oder den Rest einer leicht zu beseitigenden Estergruppe bedeutet, mit einer Verbindung der Formel (III) umsetzt: 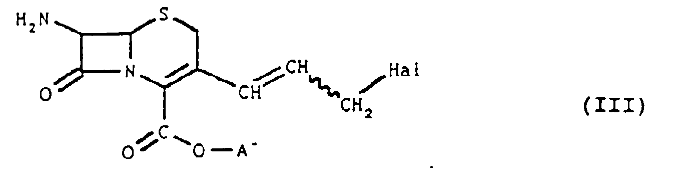 worin Hal ein Halogenatom bedeutet, A" ein Wasserstoffatom oder den Rest einer leicht zu beseitigenden Estergruppe bedeutet und die Wellenlinie bedeutet, daß die CH₂Hal-Gruppe sich in der E- oder Z-Stellung befinden kann, wodurch man eine Verbindung der Formel (IV) erhält: 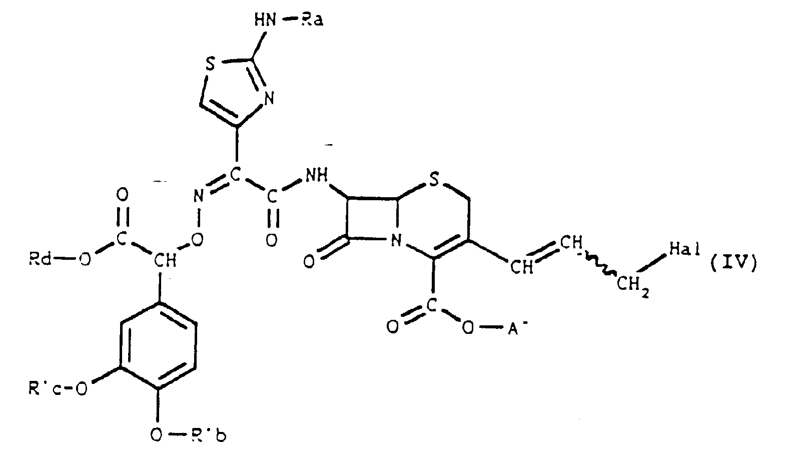 die man mit einem Reagenz, das zur Einführung des Restes R₁ befähigt ist, umsetzt, wodurch man eine Verbindung der Formel (V) erhält: 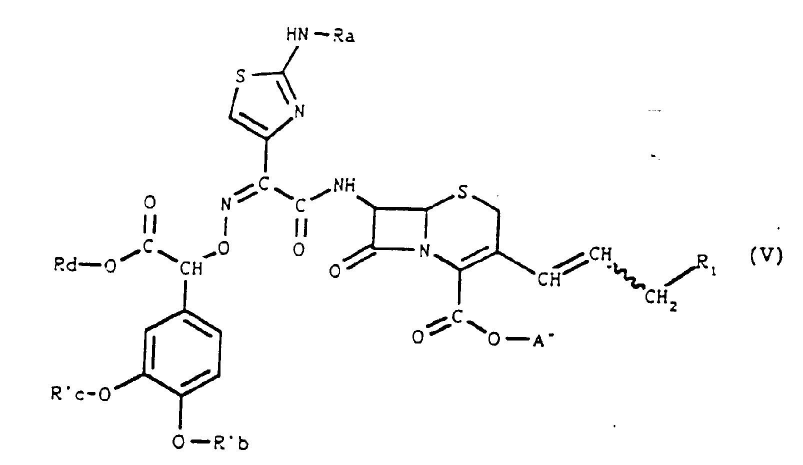 die man ggf. in ihre E- oder Z-Isomeren auftrennt, oder die Z-Isomeren in E-Isomere umwandelt, wobei man ggf. die Verbindungen der Formel (V) einer oder mehreren der folgenden Reaktionen in einer beliebigen Reihenfolge unterwirft:
a) Abspalten der Gesamtheit oder eines Teils der Estergruppen, der Aminoschutzgruppen oder der Hydroxylschutzgruppen durch Hydrolyse oder durch Einwirkung von Thioharnstoff,
b) Veresterung oder Salzbildung des oder der Carboxylreste mit einer Base,
c) Salzbildung des Aminorestes mit einer Säure und
d) Auftrennung der Produkte in Form des R,S-Gemisches in die R- oder S-Form.

2. Herstellungsverfahren nach Anspruch 1, dadurch gekennzeichnet, daß das Reagenz, das zur Einführung des Restes R₁ befähigt ist, unter Reagenzien der folgenden Formeln ausgewählt wird:

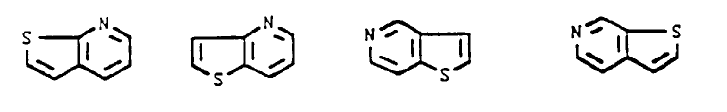

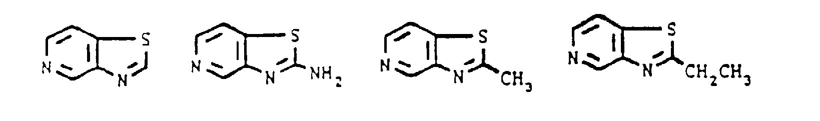

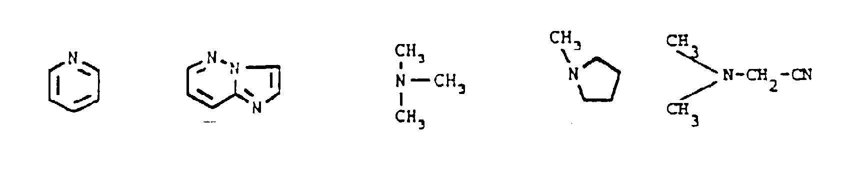

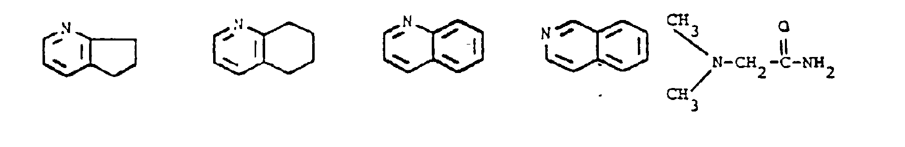

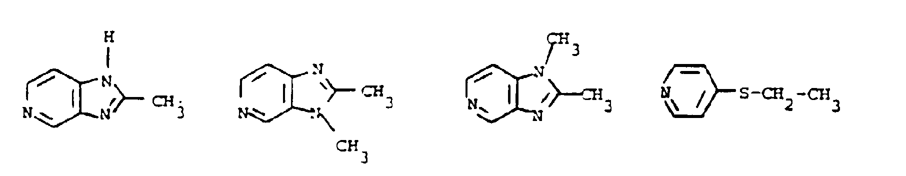

3. Herstellungsverfahren nach Anspruch 1 oder 2, dadurch gekennzeichnet, daß das Reagenz, das zur Einführung des Restes R₁ befähigt ist, unter folgenden Reagenzien ausgewählt wird: 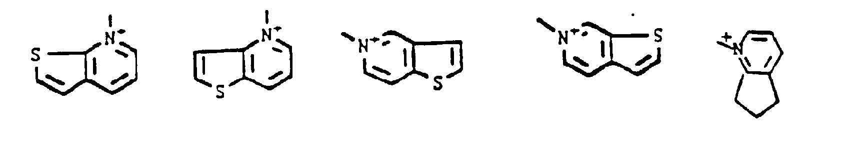 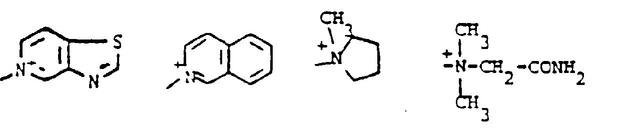 vorzugsweise

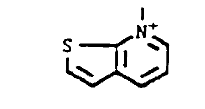

4. Verfahren nach Anspruch 1 zur Herstellung von Verbindungen der Formel (I) gemäß der Definition in Anspruch 1, entsprechend der Formel (I'): 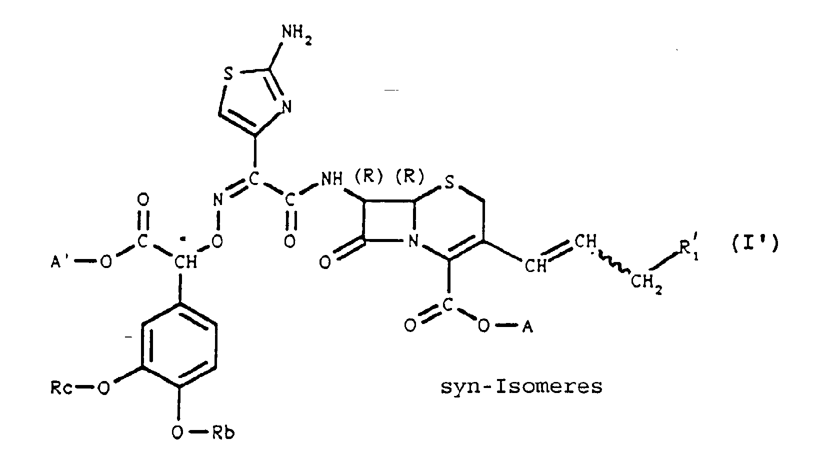 syn-Isomeres, in der R- oder S-Form oder in Form eines R,S-Gemisches, wobei in der Formel:
A, A', R_{b} und R_{c} eine unveränderte Bedeutung haben und R'₁ einen Rest bedeutet, der unter folgenden Resten ausgewählt ist: 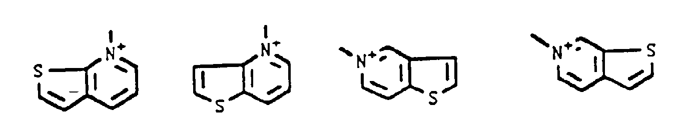 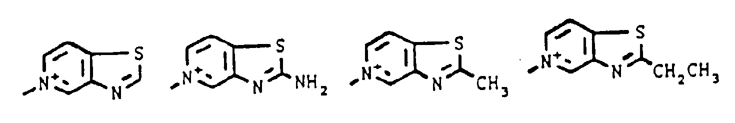 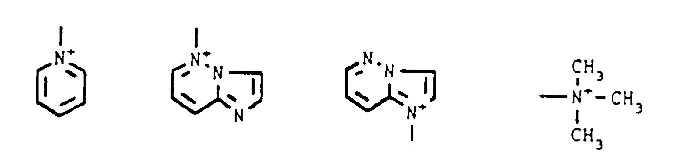 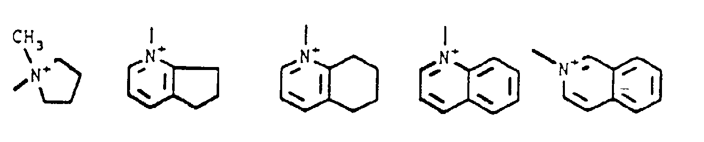 sowie von Salzen der Verbindungen der Formel (I') mit anorganischen oder organischen Säuren,
dadurch gekennzeichnet, daß man eine Verbindung der Formel (II) mit einer Verbindung der Formel (III), die der Definition in Anspruch 1 entsprechen, umsetzt, anschließend die erhaltene Verbindung der Formel (IV) mit einem Reagenz, das unter folgenden Reagenzien ausgewählt wird, umsetzt: 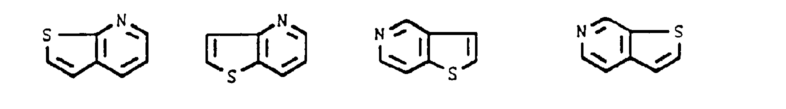 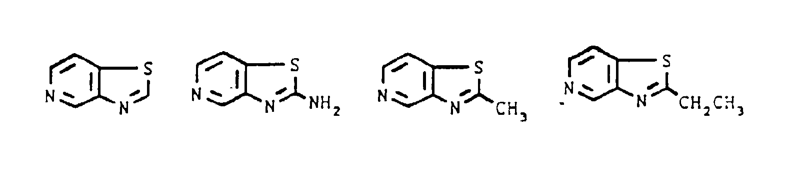 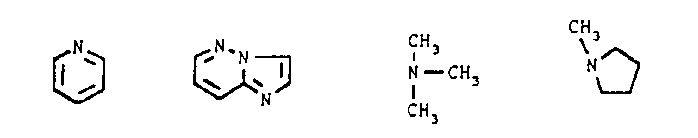 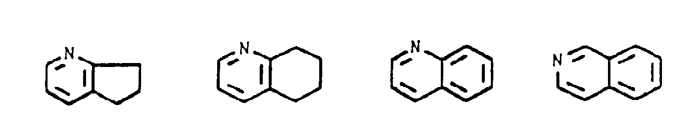 wodurch man eine Verbindung der Formel (V') erhält: 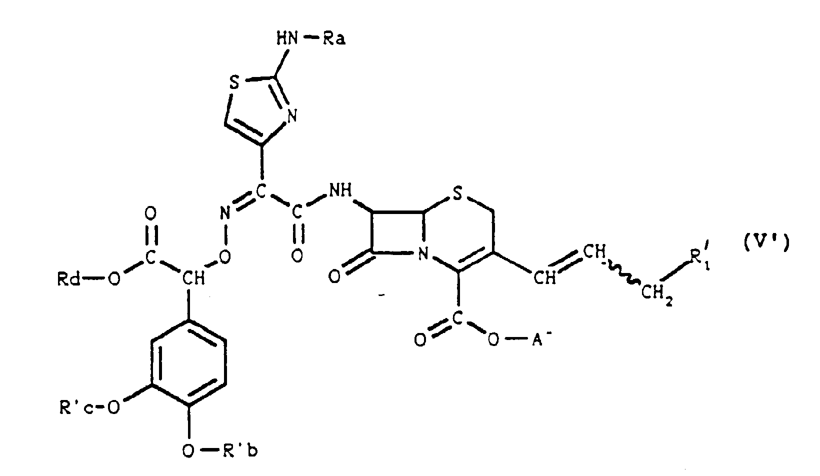 die man ggf. in ihre E- oder Z-Isomeren auftrennt, oder die Z-Isomeren in E-Isomere umwandelt, wobei man ggf. die Verbindungen der Formel (V) einer oder mehreren der folgenden Reaktionen in einer beliebigen Reihenfolge unterwirft:
a) Abspalten der Gesamtheit oder eines Teils der Estergruppen, der Aminoschutzgruppen oder der Hydroxylschutzgruppen durch Hydrolyse oder durch Einwirkung von Thioharnstoff,
b) Veresterung oder Salzbildung des oder der Carboxylreste mit einer Base,
c) Salzbildung des Aminorestes mit einer Säure und
d) Auftrennung der Produkte in Form des R,S-Gemisches in die R- oder S-Form.

5. Verfahren nach Anspruch 4, dadurch gekennzeichnet, daß das Reagenz, das zur Umsetzung mit der Verbindung der Formel (IV) verwendet wird, unter folgenden Reagenzien ausgewählt wird: 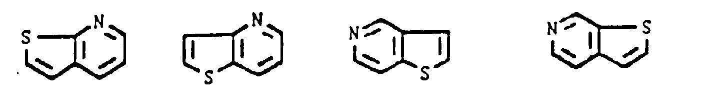 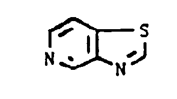 vorzugsweise:

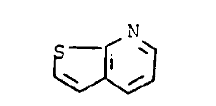

6. Verfahren nach einem der Ansprüche 1 bis 5, dadurch gekennzeichnet, daß man die verwendeten Ausgangsverbindungen und Reagenzien so auswhält, daß man eine der folgenden Verbindungen herstellt:
- [6R-[3(E),6a, 7ß(Z)]]-5-[3-[7-[[(2-Amino-4-thiozolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-thiazolo[4,5-c]pyridinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon,
- [6R-[3(E),6α,7β(Z)]]-7-[3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-thieno[2,3-b]pyridinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon und insbesondere in der S-Form,
- [6R-3(E),6a,7ß(Z)]]-2-[3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2.0]oct-2-en-3-yl]-2-propenyl]-isochinolinium in der R-oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon,
- [6R-[3(E),6α,7β(Z)]]-1-[3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-1-methylpyrrolidinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon,
- [6R-[3(E),6α,7β(Z)]]-1-(3-[7-[[(2-Amino-4-thiazolyl)[[1-(3,4-dihydroxyphenyl)-2-hydroxy-2-oxoethoxy]-imino]-acetyl]-amino]-2-carboxy-8-oxo-5-thia-1-azabicyclo[4,2,0]oct-2-en-3-yl]-2-propenyl]-6,7-dihydro-5H-pyridinium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon und
- [6R[3(E),6α,7β(Z)]]-N-(2-Amino-2-oxoethyl)-3-[7-[[(2-amino-4-thiazolyl)-[[1-(3,4-dihydroxyphenyl)-2-hydroxy-2-oxoethoxy]-imino]-acetyl]-amino]-2-carboxy-8-oxo-5-thia-1-azabicylo[4,2,0]oct-2-en-3-yl]-N,N-dimethyl-2-propen-1-aminium in der R- oder S-Form oder in Form eines R,S-Gemisches und in Form eines internen Salzes oder eines Salzes mit Alkalimetallen, Erdalkalimetallen, Magnesium, Ammoniak, organischen Aminbasen, Säuren und leicht spaltbaren Estern davon.

7. Verfahren zur Herstellung von pharmazeutischen Zusammensetzungen, dadurch gekennzeichnet, daß man als Wirkstoff mindestens eines der Derivate der Formel (I) gemäß der Definition in Anspruch 1 sowie deren pharmazeutisch verträgliche Salze mit Säuren in einer für diese Verwendung bestimmten Form bereitstellt.

8. Verfahren zur Herstellung von pharmazeutischen Zusammensetzungen, dadurch gekennzeichnet, daß man als Wirkstoff mindestens eines der Derivate der Formel (I') gemäß der Definition in Anspruch 4 sowie deren pharmazeutisch verträgliche Salze mit Säuren in einer für diese Verwendung bestimmten Form bereitstellt.

9. Verfahren zur Herstellung von pharmazeutischen Zusammensetzungen, dadurch gekennzeichnet, daß man als Wirkstoff mindestens eines der Derivate der Formel (I) gemäß der Definition in Anspruch 6 sowie deren pharmazeutisch verträgliche Salze mit Säuren in einer für diese Verwendung bestimmten Form bereitstellt.

10. Verbindungen der Formel (IV) und Verbindungen der Formel (V), worin Rₐ eine Aminoschutzgruppe bedeutet, wobei die Formeln (IV) und (V) der Definition in Anspruch 5 entsprechen, als gewerblich einzusetzende Verbindungen.

## Claims (Claims for the following Contracting State(s) : AT, BE, CH, DE, DK, FR, GB, IT, LI, LU, NL, SE)

1. The products of general formula (I): 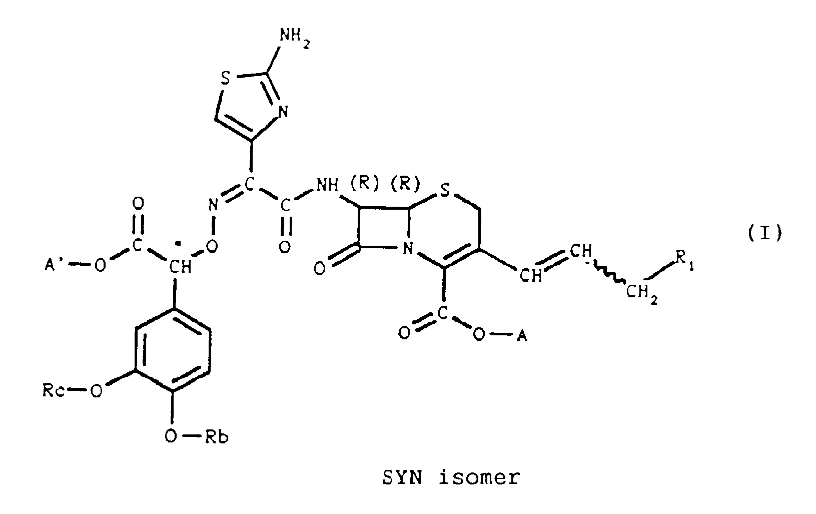 syn isomer, in R or S form or in the form of an R, S, mixture, formula in which: R₁ represents a radical chosen from the following radicals: 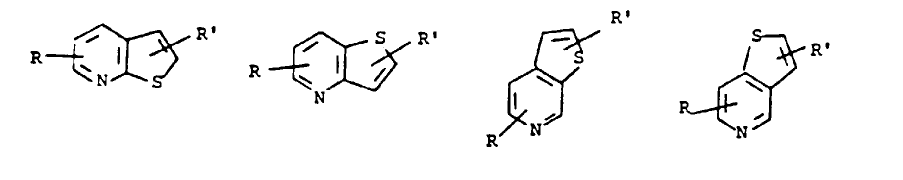 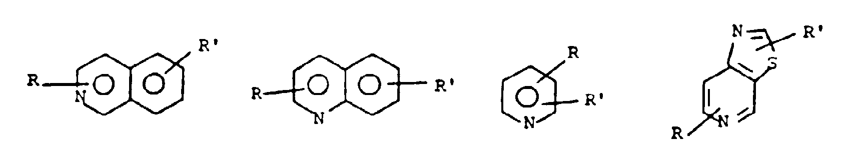 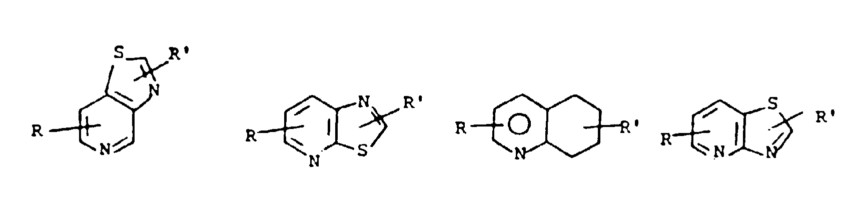 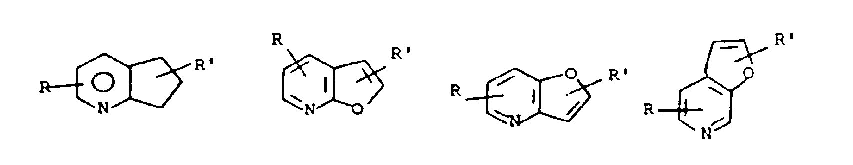 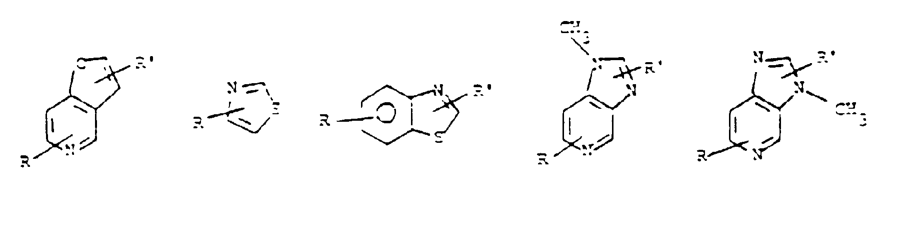 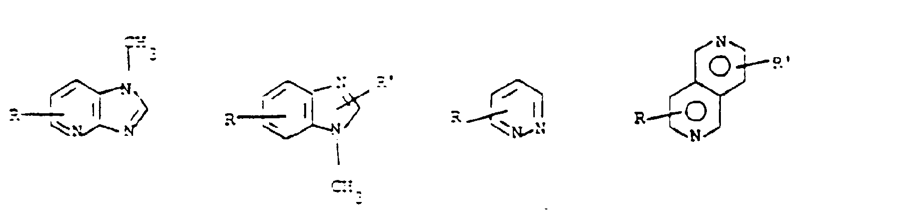 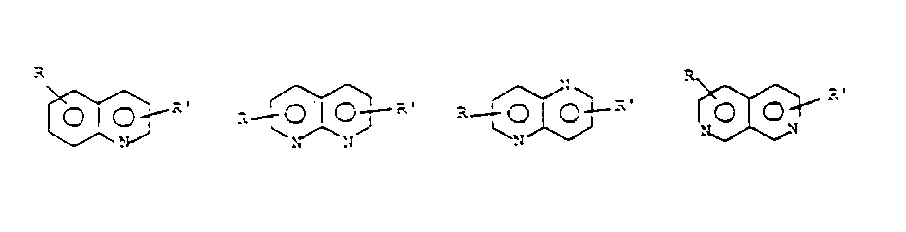 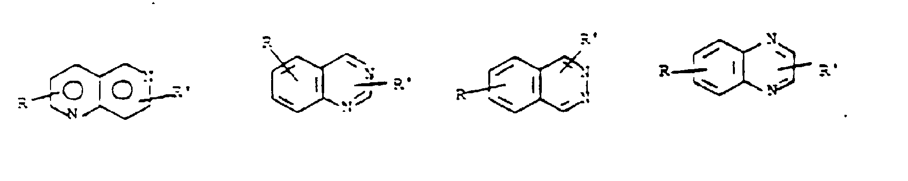 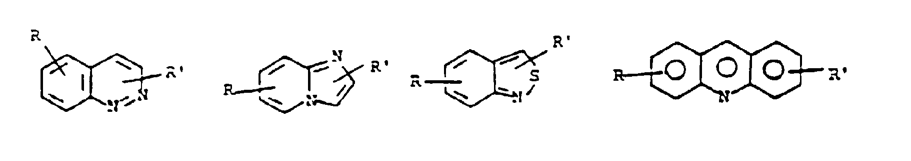 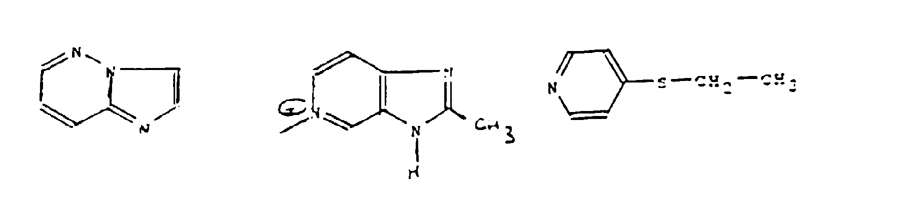 or 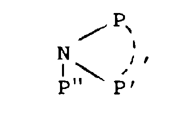 in the quaternary ammonium form, the expression in quaternary ammonium form indicating that the R₁ radical is linked to the -CH=CH=CH₂ group by the nitrogen atom or atoms which it contains,
in which R and R', identical or different, represent a hydrogen atom, an alkyl radical containing 1 to 4 carbon atoms, an alkoxy radical containing 1 to 4 carbon atoms, a halogen atom, one of the following radicals: C0₂-Q, 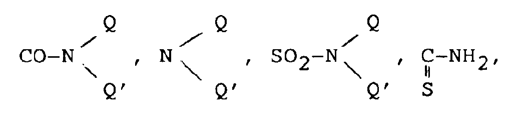 NH-CO-Q, CN, CH₂-CN, CH₂-SQ in which Q and Q', identical or different, represent a hydrogen atom or an alkyl radical containing 1 to 4 carbon atoms, P, P' and P", identical or different, represent an alkyl radical containing at most 4 carbon atoms, optionally substituted by one of the substituents indicated above for R and R', the symbol indicating that P and P" can optionally form, with the nitrogen atom to which they are linked, a heterocycle with 5 or 6 members.
R_{b} and R_{c}, identical or different, represent a hydrogen atom or an acyl group, chosen from the acetyl, propionyl and benzoyl radicals,
A and A', identical or different, represent a hydrogen atom, an equivalent of an alkali metal, an alkaline-earth metal, magnesium, ammonium or an amino organic base or A and A' represent the remainder of an easily cleavable ester group or C0₂A represents CO₂⁻; the wavy line means that the CH₂R₁ group can be found in E or Z position as well as the salts of the products of formula (I) with the mineral or organic acids.

2. The products of general formula (I) as defined in claim 1 in which R₁ is chosen from the following radicals: 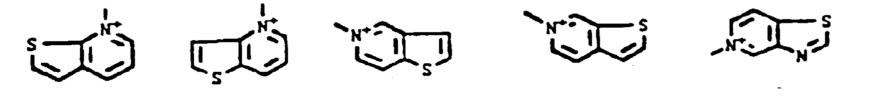 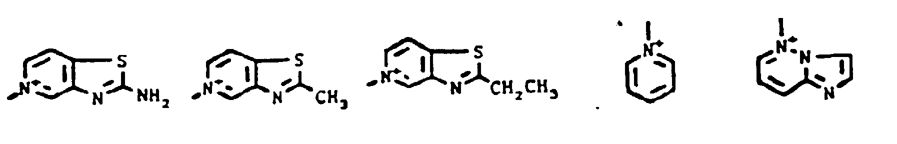 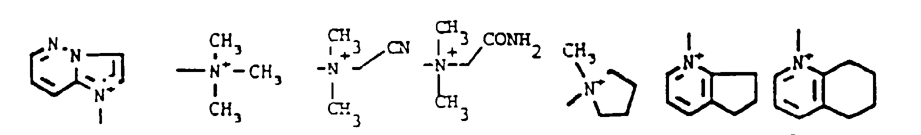 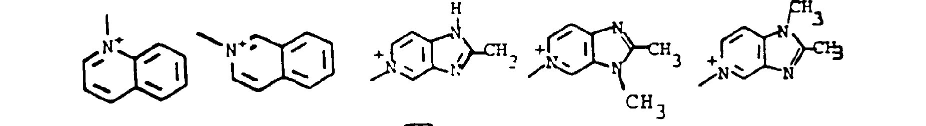 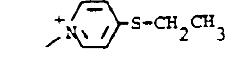 The products of general formula (I) as defined in claim 1 or 2 in which R₁ is chosen from the following radicals:

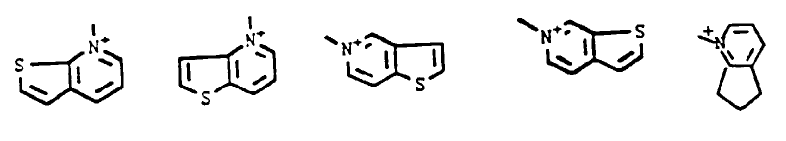

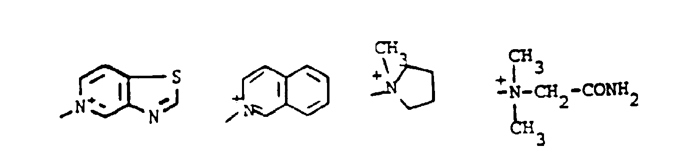

4. The products of general formula (I) as defined in claim 3 in which R₁ represents the radical:

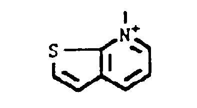

5. The product of general formula (I) according to claim 1 the name og which follows:
- [6R-[3(E), 6alpha, 7beta(Z)]]-5-[3-[7-[[(2-amino-4-thiazolyl)-[[1-(3,4-dihydroxyphenyl)-2-hydroxy-2-ox- oethoxy]imino]-acetyl]-amino]-2-carboxy-8-oxo-5-thia-1-azabicyclo [4,2,0]-oct-2-en-3-yl]-2-propenyl]-thiazolo-[4,5-c]pyridinium in the R or S form or in the form of an R, S mixture and in the form of an internal salt or a salt with alkali metals, alkaline-earth metals, magnesium, ammonia, amino organic bases, acids and its easily cleavable esters,
- [6R-[3(E),6alpha, 7beta(Z)]]-7-[3-[7-[[(2-amino-4-thiazolyl)-[[1-(3,4-dihydroxyphenyl)-2-hydroxy-2-oxoethoxy] imino]-acetyl]-amino]-2-carboxy-8-oxo-5-thia-1-azabicyclo [4,2,0]-oct-2-en-3-yl]-2-propenyl]-thieno-[2,3-b] pyridinium in the R or S form or in the form of an R, S mixture and in the form of an internal salt or a salt with alkali metals, alkaline-earth metals, magnesium, ammonia, amino organic bases, acids and its easily cleavable esters and particularly in the S form,
- [6R-[3(E), 6alpha, 7beta(Z)]]-2-[3-[7-[[(2-amino-4-thiazolyl)-[[1-(3,4-dihydroxyphenyl)-2-hydroxy-2-oxoeth- oxy]imino]-acetyl]-amino]-2-carboxy-8-oxo-5-thia-1-azabicyclo[4,2,0]-oct-2-en-3-yl]-2-propenyl] isoquinolinium in the R or S form or in the form of an R, S mixture and in the form of an internal salt or a salt with alkali metals, alkaline-earth metals, magnesium, ammonia, amino organic bases, acids and its easily cleavable esters,
- [6R-[3(E), 6alpha, 7beta(Z)]]-1-[3-[7-[[(2-amino-4-thiazolyl)-[[1-(3,4-dihydroxyphenyl)-2-hydroxy-2-ox- oethoxy]imino]-acetyl]-amino]-2-carboxy-8-oxo-5-thia-1-azabicyclo [4,2,0]-oct-2-en-3-yl]-2-propenyl]-1-methyl pyrrolidinium in the R or S form or the form of an R, S mixture and in the form of an internal salt or a salt with alkali metals, alkaline-earth metals, magnesium, ammonia, amino organic bases, acids and its easily cleavable esters,
- [6R-[3(E), 6alpha, 7beta(Z)]]-1-[3-[7-[[(2-amino-4-thiazolyl)-[[1-(3,4-dihydroxyphenyl)-2-hydroxy-2-oxoeth- oxy]imino]-acetyl]-amino]-2-carboxy-8-oxo-5-thia-1-azabicyclo[4,2,0]-oct-2-en-3-yl]-2-propenyl]-6,7-dihydro-5H-pyridinium in the R or S form or in the form of an R, S mixture and in the form of an internal salt or a salt with alkali metals, alkaline-earth metals, magnesium, ammonia, amino organic bases, acids and its easily cleavable esters,
- [6R-[3(E), 6alpha, 7beta(Z)]]-N-(2-amino-2-oxoethyl)-3-[7-[[(2-amino-4-thiazolyl)-[[1-(3,4-dihydroxyphenyl)-2-hydroxy-2-oxoethoxy]-imino]-acetyl]-amino]-2-carboxy-8-oxo-5-thia-1 -azabicyclo-[4,2,0]-oct-2-en-3-yl]-N, N-dimethyl-2-propen-1-aminium in the R or S form or in the form of an R, S mixture and in the form of an internal salt or a salt with alkali metals, alkaline-earth metals, magnesium, ammonia, amino organic bases, acids and its easily cleavable esters.

6. Preparation process for the products of formula (I) as defined in claim 1, characterized in that a product of formula (II): 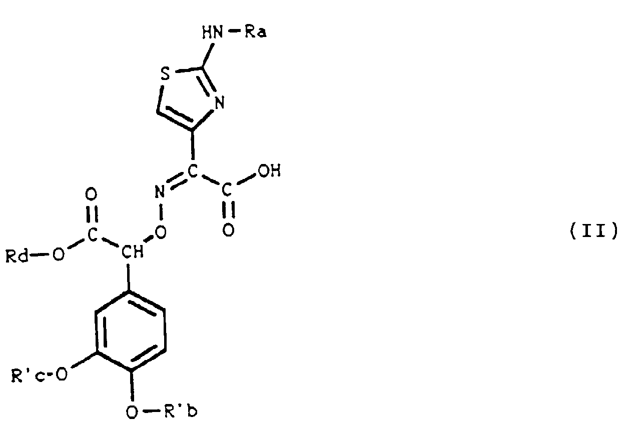 racemic or optically active syn isomer or a functional derivative of the product of formula (II), in which Rₐ represents a hydrogen atom or a protective group of the amino radical, R'_{b} and R'_{c}, identical or different, represent a hydrogen atom or a protective group of the hydroxyl radical, R_{d} represents a hydrogen atom or the remainder of an easily eliminable ester group, is reacted with a product of formula (III): 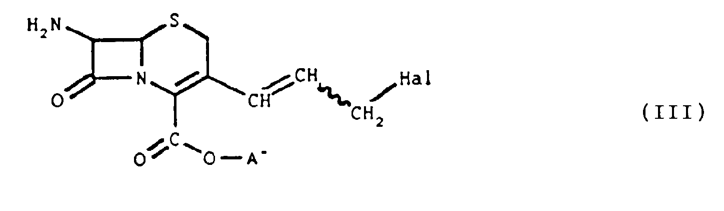 in which Hal represents a halogen atom, A" represents a hydrogen atom or the remainder of an easily eliminable ester group and the wavy line indicates that the CH₂Hal group can be found in E or Z position, in order to obtain a product of formula (IV): 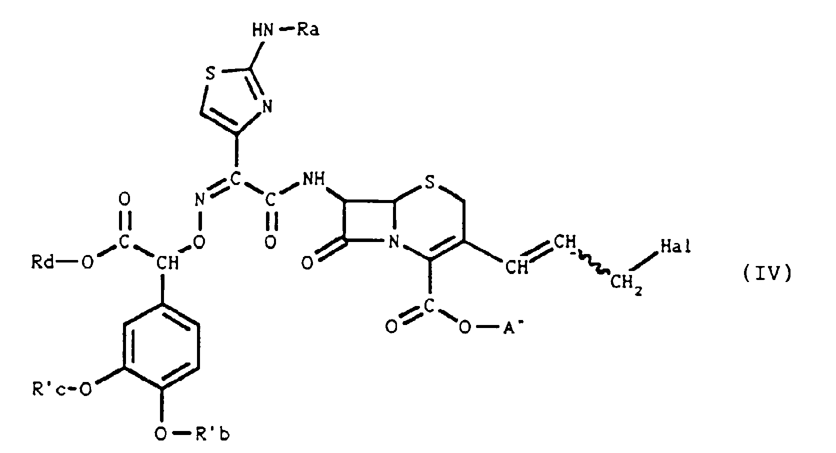 which is reacted with a reagent capable of introducing the R₁ radical in order to obtain a product of formula (V): 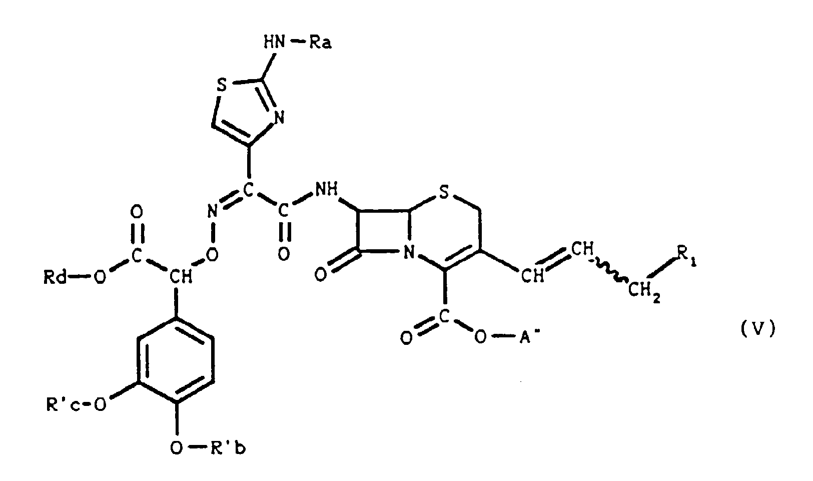 which, if desired, is separated into its E or Z isomers or the Z isomers are converted into E isomers and which products of formula (V), if necessary or if desired, are subjected to one or more of the following reactions, in any order:
a) cleaving, by hydrolysis or by the action of thiourea, of all or part of the ester groups or protective groups of the amino radical or the hydroxyl radicals,
b) esterification or salification of the carboxylic radical or radicals by a base,
c) salification of the amino radical by an acid,
d) separation of products in the form of an R, S mixture into R or S.

7. Preparation process according to claim 6 characterized in that the reagent capable of introducing the R, radical is chosen from the reagents of formulae:

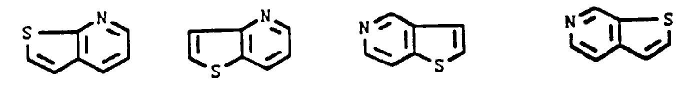

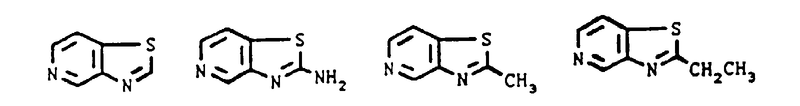

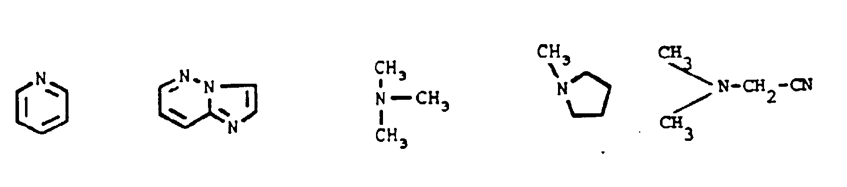

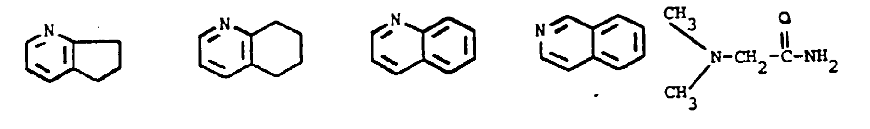

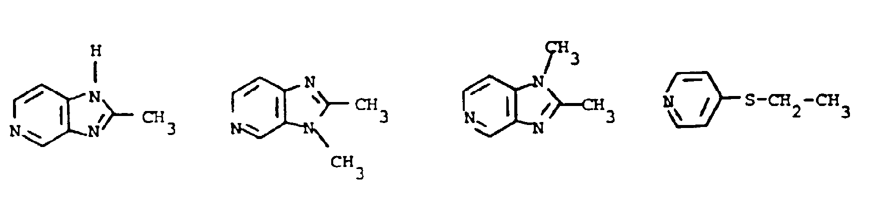

8. As medicaments, the products corresponding to formula (I) as defined in claim 1, as well as their pharmaceutically acceptable acid salts.

9. As medicaments, the products as defined in any one of claims 2 to 5 as well as their pharmaceutically acceptable acid salts.

10. Pharmaceutical compositions containing, as active ingredient, at least one medicament according to one of claims 8 or 9.

11. As industrial products, the products of formula (IV) and the products of formula (V) in which Rₐ represents a protective group of the amino radical, formulae (IV) and (V) being as defined in claim 6.

## Claims (Claims for the following Contracting State(s) : ES)

1. Preparation process for the products of general formula (I): 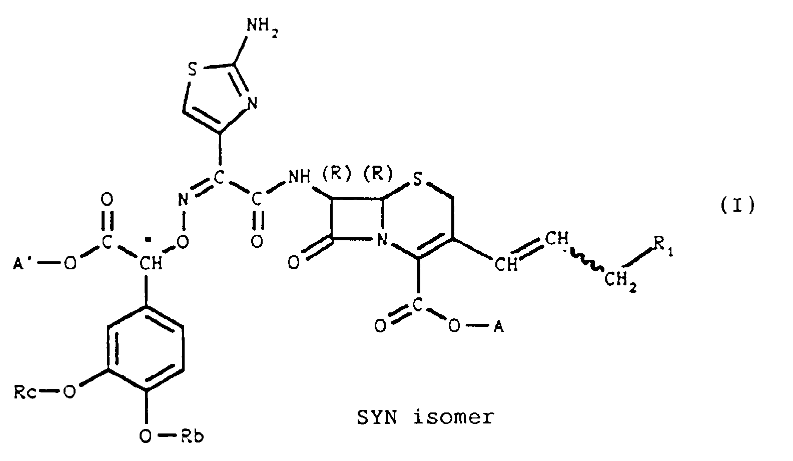 syn isomer, in R or S form or in the form of an R, S, mixture, formula in which: R₁ represents a radical chosen from the following radicals: 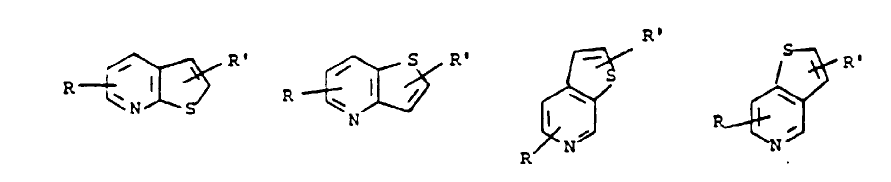 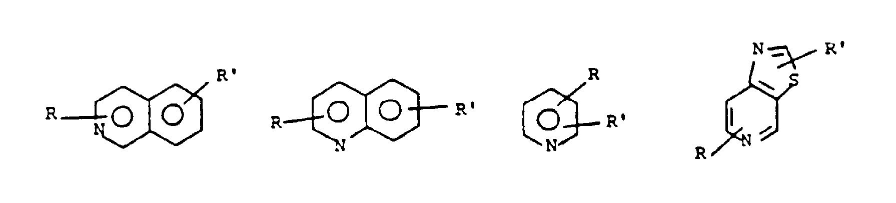 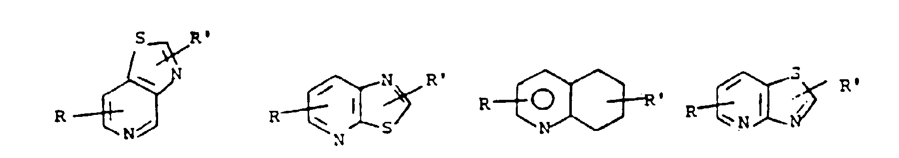 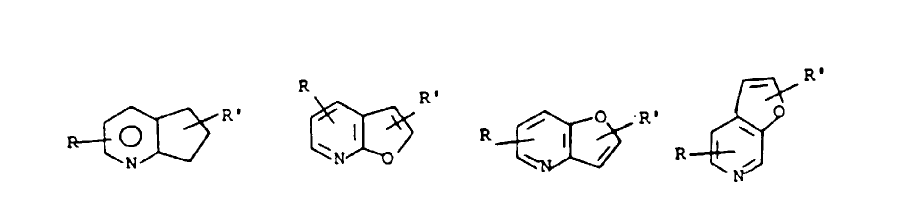 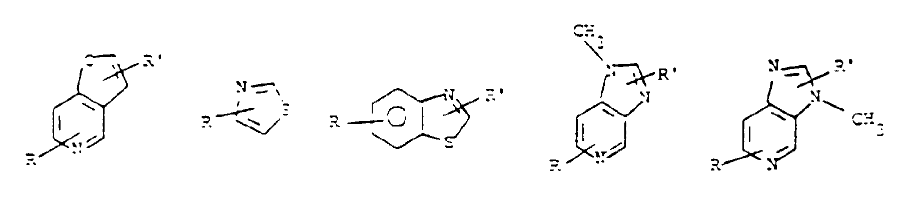 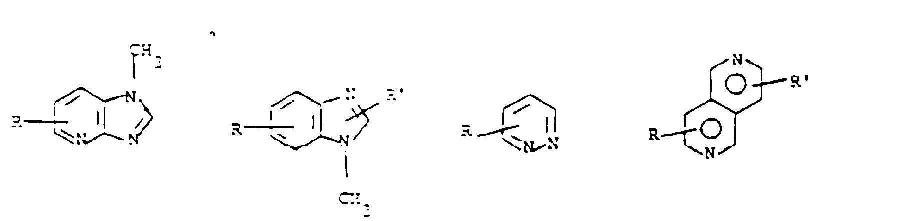 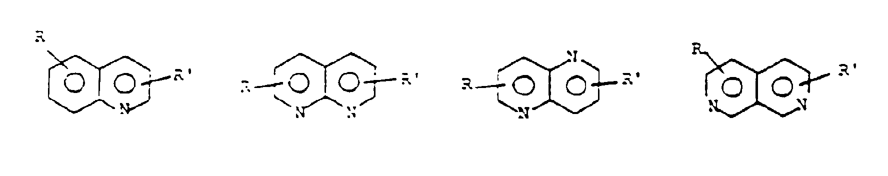 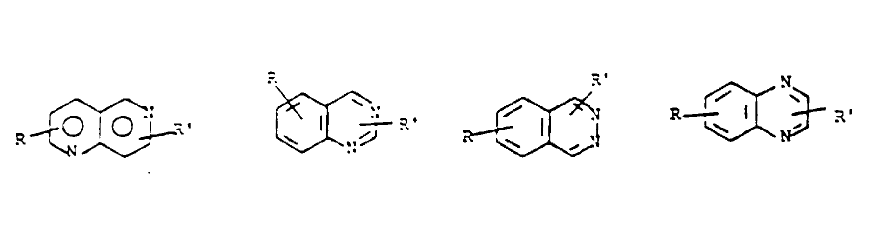 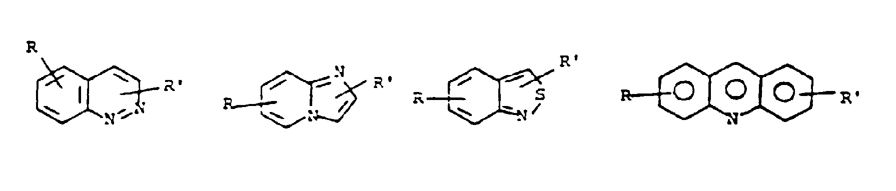 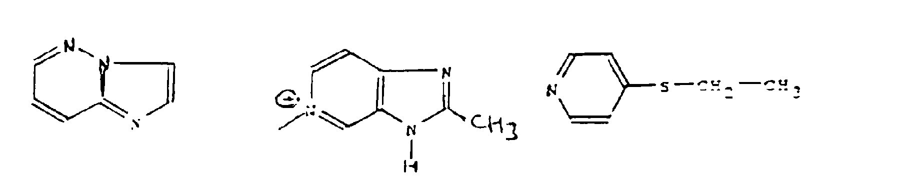 or 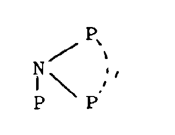 in the quaternary ammonium form, the expression in quaternary ammonium form indicating that the R₁ radical is linked to the -CH=CH=CH₂ group by the nitrogen atom or atoms which it contains,
in which R and R', identical or different, represent a hydrogen atom, an alkyl radical containing 1 to 4 carbon atoms, an alkoxy radical containing 1 to 4 carbon atoms, a halogen atom, one of the following radicals a C0₂-Q, 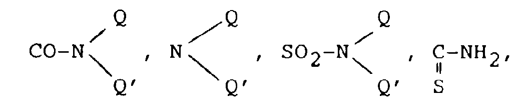 NH-CO-Q, CN, CH₂-CN, CH₂-SQ in which Q and Q', identical or different, represent a hydrogen atom or an alkyl radical containing 1 to 4 carbon atoms, P, P' and P", identical or different, represent an alkyl radical containing at most 4 carbon atoms, optionally substituted by one of the substituents indicated above for R and R', the symbo indicating that P and P' can optionally form, with the nitrogen atom to which they are linked, a heterocycle with 5 or 6 members.
R_{b} and R_{c}, identical or different, represent a hydrogen atom or an acyl group, chosen from the acetyl, propionyl and benzoyl radicals,
A and A', identical or different, represent a hydrogen atom, an equivalent of an alkali metal, an alkaline-earth metal, magnesium, ammonium or an amino organic base or A and A' represent the remainder of an easily cleavable ester group or C0₂A represents CO₂⁻; the wavy line means that the CH₂R, group can be found in E or Z position as well as the salts of the products of formula (I) with the mineral or organic acids, characterized in that a product of formula (II): 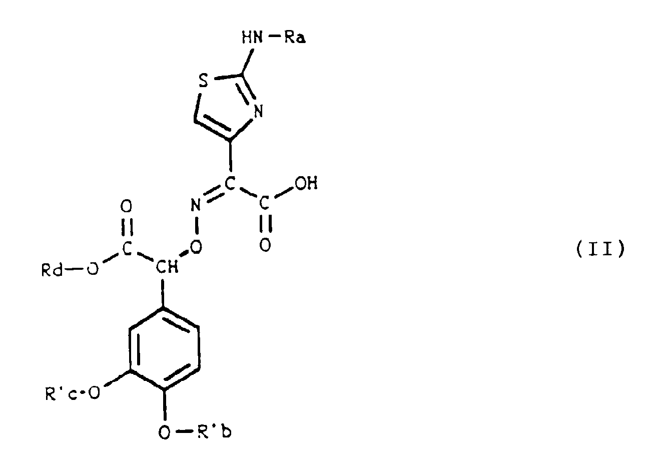 racemic or optically active syn isomer or a functional derivative of the product of formula (II), in which Rₐ represents a hydrogen atom or a protective group of the amino radical, R'_{b} and R'_{c}, identical or different, represent a hydrogen atom or a protective group of the hydroxyl radical, R_{d} represents a hydrogen atom or the remainder of an easily eliminable ester group, is reacted with a product of formula (III): 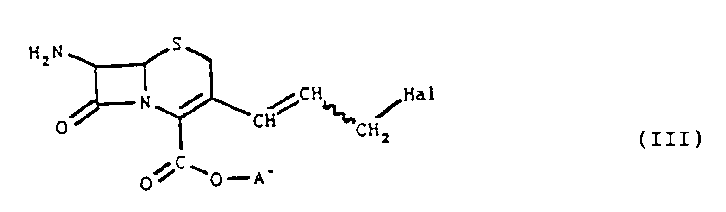 in which Hal represents a halogen atom, A" represents a hydrogen atom or the remainder of an easily eliminable ester group and the wavy line indicates that the CH₂Hal group can be found in E or Z position, in order to obtain a product of formula (IV): 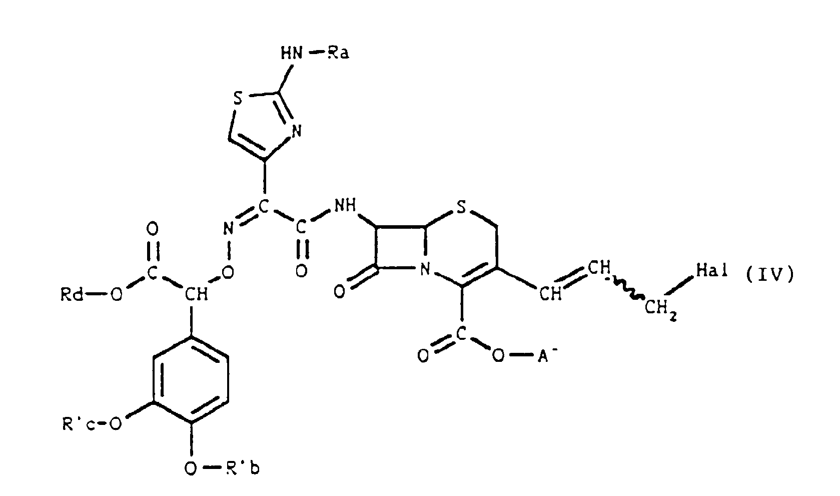 which is reacted with a reagent capable of introducing the Rᵢ radical in order to obtain a product of formula (V): 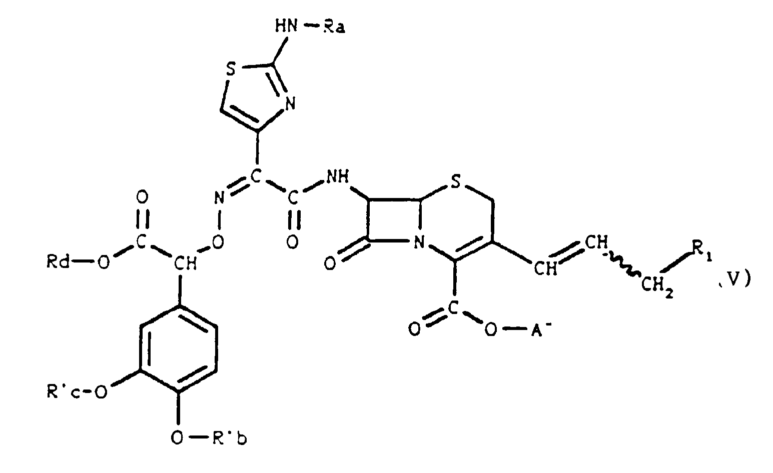 which, if desired, is separated into its E or Z isomers or the Z isomers are converted into E isomers and which products of formula (V), if necessary or if desired, are subjected to one or more of the following reactions, in any order:
a) cleaving, by hydrolysis or by the action of thiourea, of all or part of the ester groups or protective groups of the amino radical or the hydroxyl radicals,
b) esterification or salification of the carboxylic radical or radicals by a base,
c) salification of the amino radical by an acid,
d) separation of products in the form of an R, S mixture into R or S.

2. Preparation process according to claim 1 characterized in that the reagent capable of introducing the R₁ radical is chosen from the reagents of formulae:

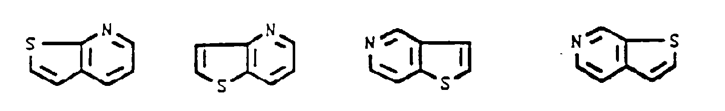

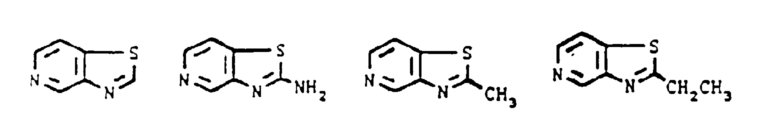

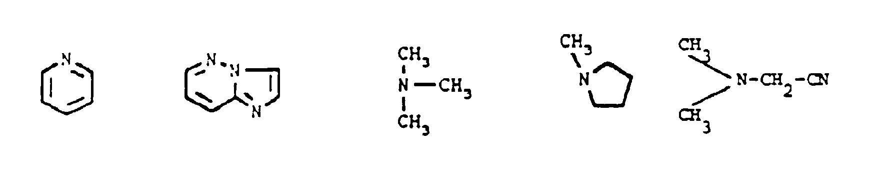

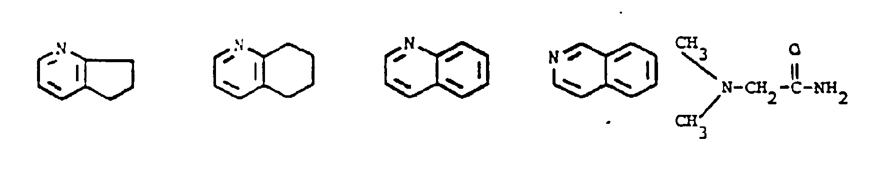

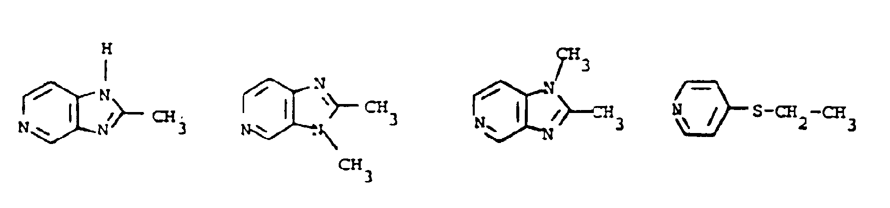

3. Preparation process according to claim 1 or 2, characterized in that the reagent capable of introducing the R₁ radical is chosen from the reagents: 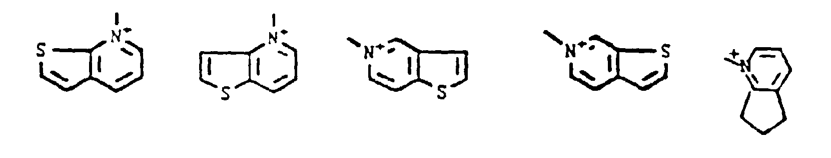 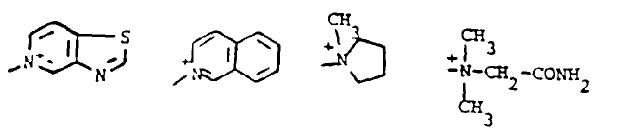 preferably:

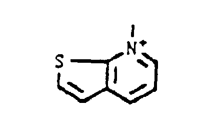

4. Process according to claim 1 for the preparation of the products of formula (I) as defined in claim 1 corresponding to formula (I'): 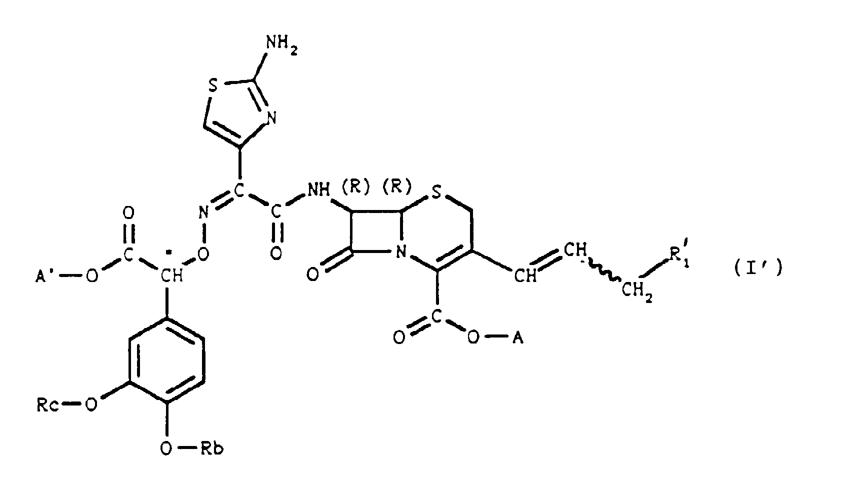 SYN isomer syn isomer, in R or S form or in the form of an R, S, mixture, formula in which:
A, A' R_{b} and R_{c} retain their previous meaning and R'₁ represents a radical chosen from the following radicals: 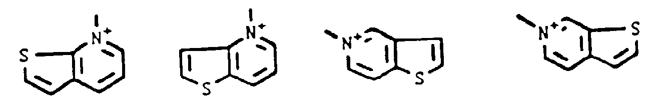 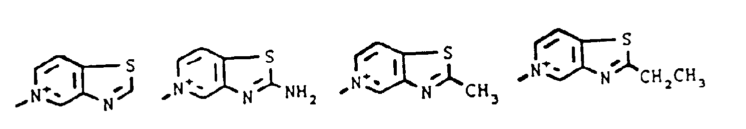 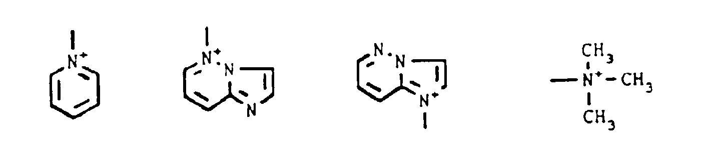 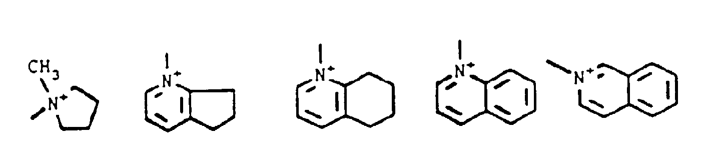 as well as the salts of the products of formula (I') with mineral or organic acids, characterized in that a product of formula (II) is reacted with a product of formula (III) defined as in claim 1, then a reagent chosen from the reagents of formula: 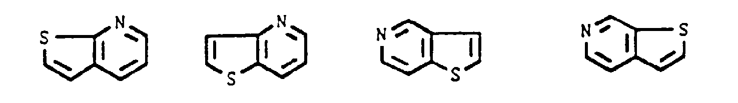 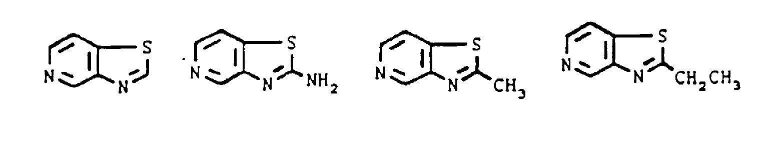 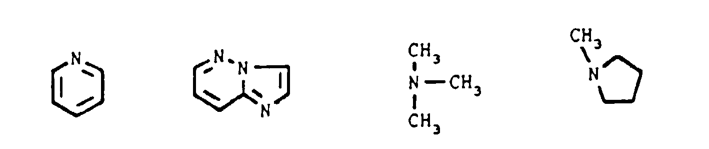 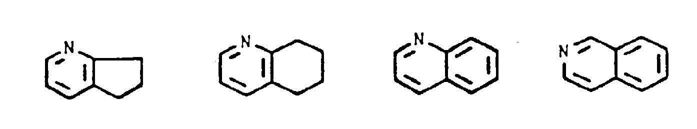 is reacted with the product of formula (IV) obtained, in order to obtain a product of formula (V'): 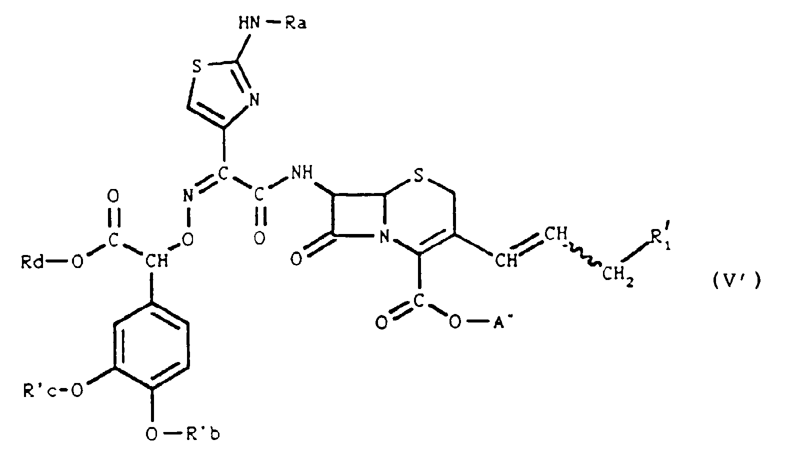 which, if desired, is separated into its E or Z isomers or the Z isomers are converted into E isomers and which products of formula (V), if necessary or if desired, are subjected to one or more of the following reactions, in any order:
a) cleaving, by hydrolysis or by the action of the thiourea, of all or part of the ester groups or protective groups of the amino radical or the hydroxyl radicals,
b) esterification or salification of the carboxylic radical or radicals by a base,
c) salification of the amino radical by an acid,
d) separation of products in the form of an R, S mixture into R or S

5. Process according to claim 4, characterized in that the reagent used on the product of formula (IV) is chosen from the reagents: 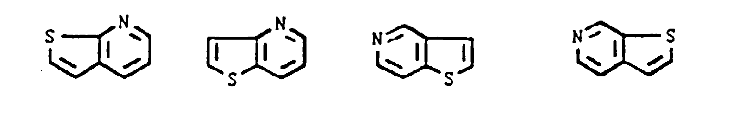 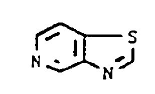 preferably:

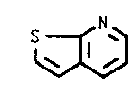

## Claims (Claims for the following Contracting State(s) : GR)

1. Preparation process for the products of general formula (I): 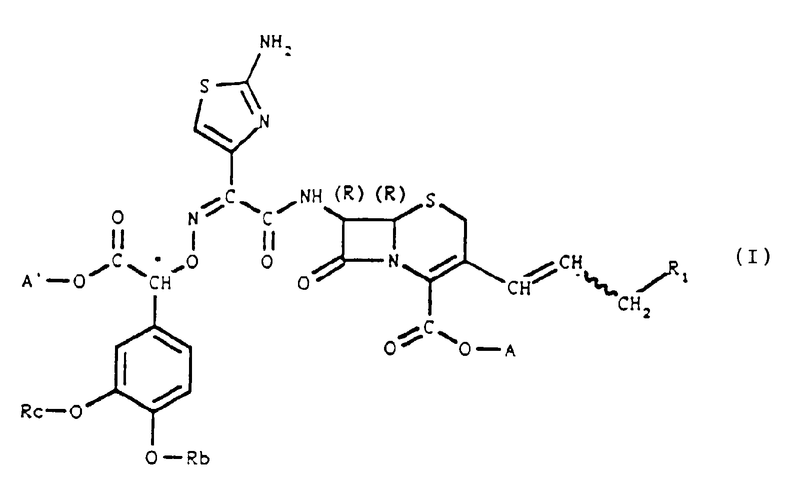 SYN isomer syn isomer, in R or S form or in the form of an R, S, mixture, formula in which:
R₁ represents a radical chosen from the following radicals: 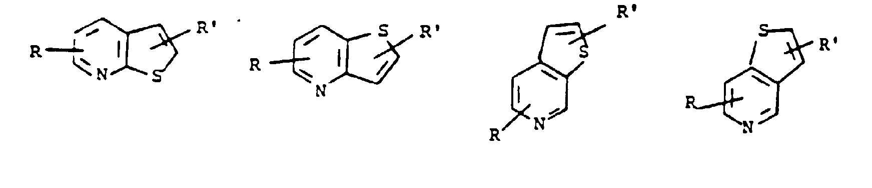 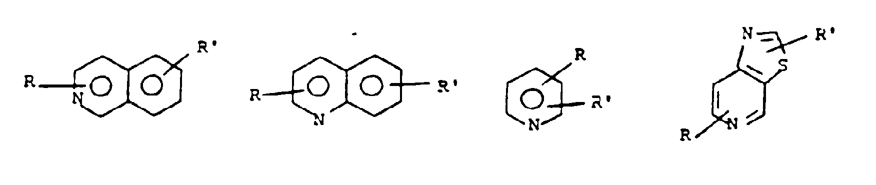 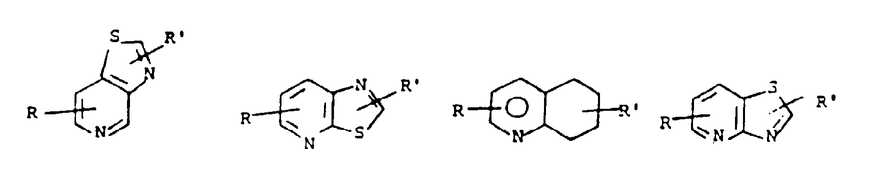 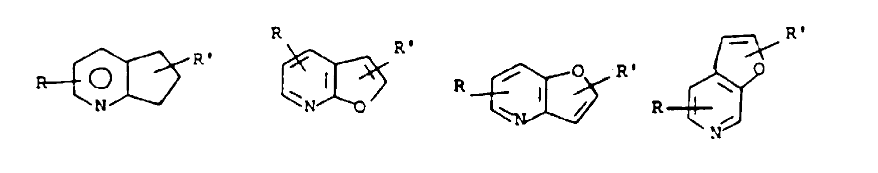 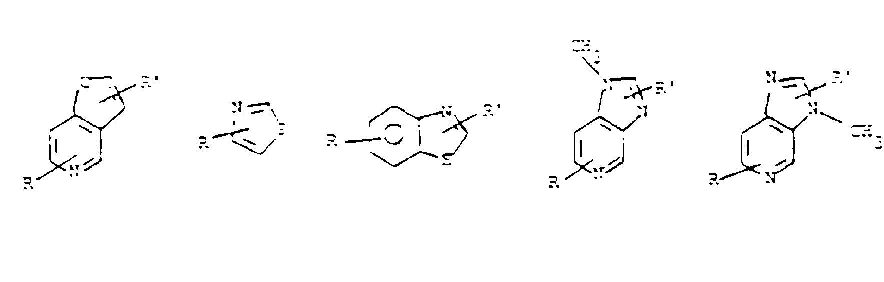 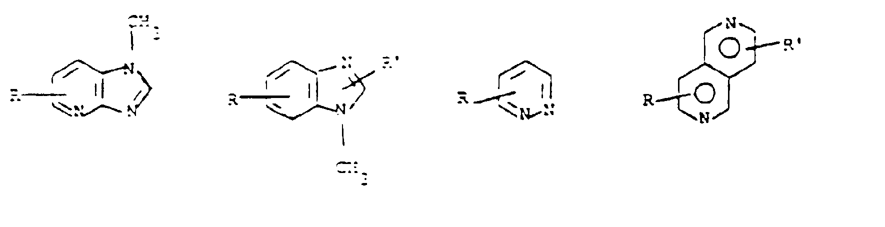 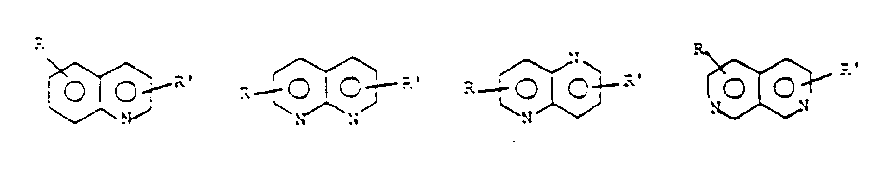 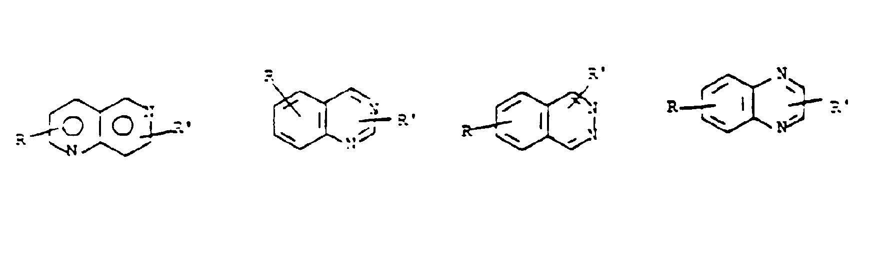 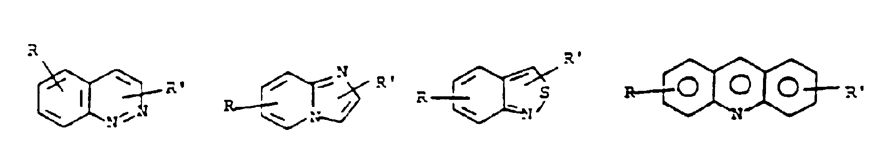 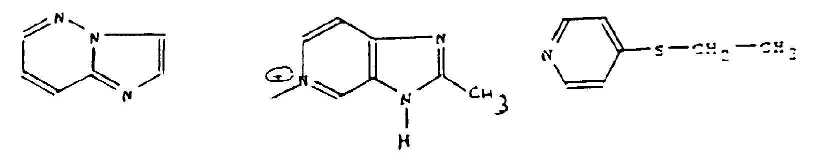 or 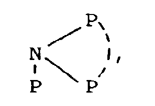 in the quaternary ammonium form, the expression in quaternary ammonium form indicating that the R₁ radical is linked to the -CH=CH=CH₂ group by the nitrogen atom or atoms which it contains,
in which Rand R', identical or different, represent a hydrogen atom, an alkyl radical containing 1 to 4 carbon atoms, an alkoxy radical containing 1 to 4 carbon atoms, a halogen atom, one of the following radicals a C0₂-Q, 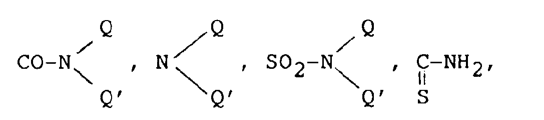 NH-CO-Q, CN, CH₂-CN, CH₂-SQ in which Q and Q', identical or different, represent a hydrogen atom or an alkyl radical containing 1 to 4 carbon atoms, P, P' and P", identical or different, represent an alkyl radical containing at most 4 carbon atoms, optionally substituted by one of the substituents indicated above for R and R', the symbol indicating that P and P' can optionally form, with the nitrogen atom to which they are linked, a heterocycle with 5 or 6 members.
R_{b} and R_{c}, identical or different, represent a hydrogen atom or an acyl group, chosen from the acetyl, propionyl and benzoyl radicals,
A and A', identical or different, represent a hydrogen atom, an equivalent of an alkali metal, an alkaline-earth metal, magnesium, ammonium or an amino organic base or A and A' represent the remainder of an easily cleavable ester group or C0₂A represents CO₂⁻; the wavy line means that the CH₂R₁ group can be found in E or Z position as well as the salts of the products of formula (I) with the mineral or organic acids, characterized in that a product of formula (II): 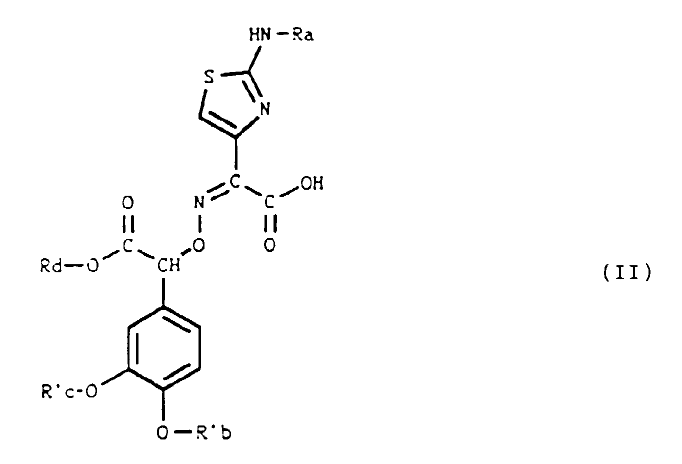 racemic or optically active syn isomer or a functional derivative of the product of formula (II), in which Rₐ represents a hydrogen atom or a protective group of the amino radical, R'_{b} and R'_{c}, identical or different, represent a hydrogen atom or a protective group of the hydroxyl radical, R_{d} represents a hydrogen atom or the remainder of an easily eliminable ester group, is reacted with a product of formula (III): 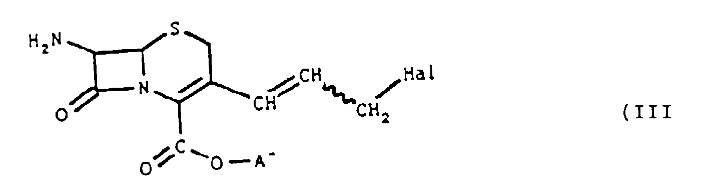 in which Hal represents a halogen atom, A" represents a hydrogen atom or the remainder of an easily eliminable ester group and the wavy line indicates that the CH₂Hal group can be found in E or Z position, in order to obtain a product of formula (IV): 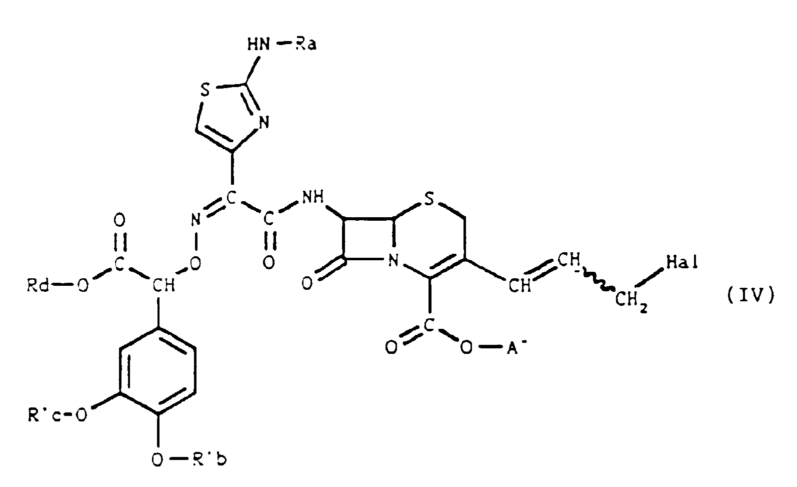 which is reacted with a reagent capable of introducing the Rᵢ radical in order to obtain a product of formula (V): 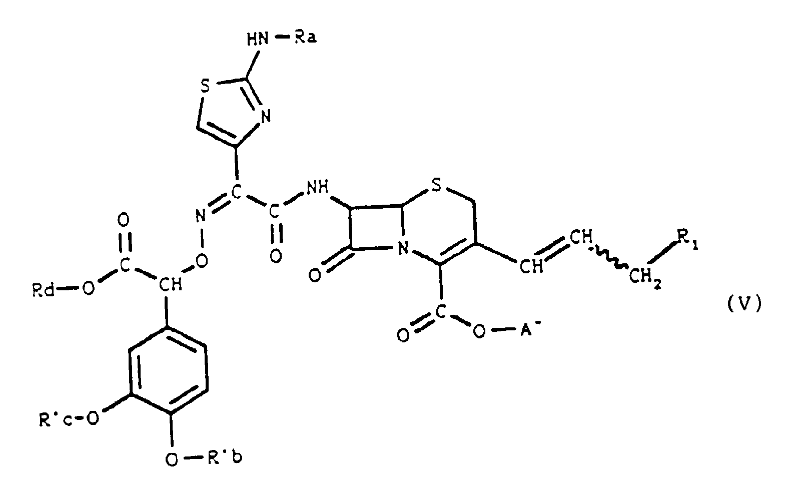 which, if desired, is separated into its E or Z isomers or the Z isomers are converted into E isomers and which products of formula (V), if necessary or if desired, are subjected to one or more of the following reactions, in any order:
a) cleaving, by hydrolysis or by the action of thiourea, of all or part of the ester groups or protective groups of the amino radical or the hydroxyl radicals,
b) esterification or salification of the carboxylic radical or radicals by a base,
c) salification of the amino radical by an acid,
d) separation of products in the form of an R, S mixture into R or S.

2. Preparation process according to claim 1 characterized in that the reagent capable of introducing the R₁ radical is chosen from the reagents of formulae:

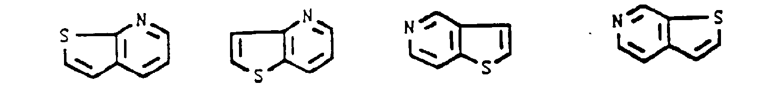

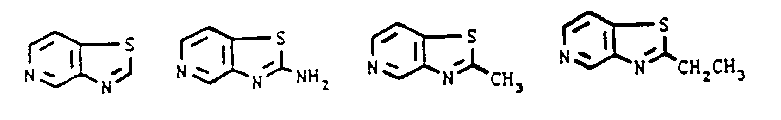

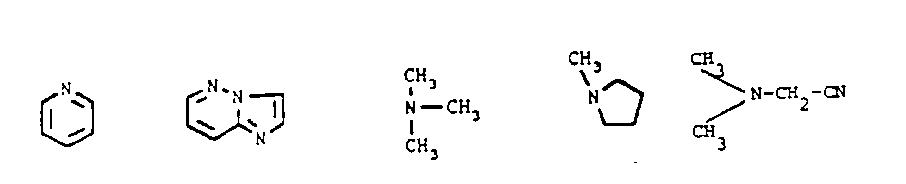

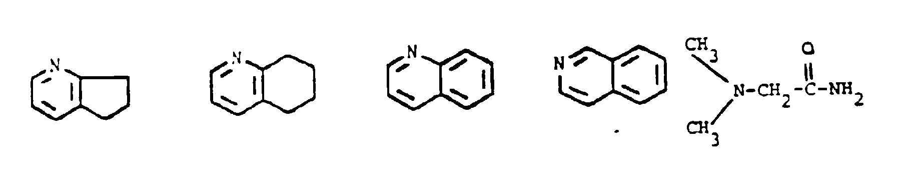

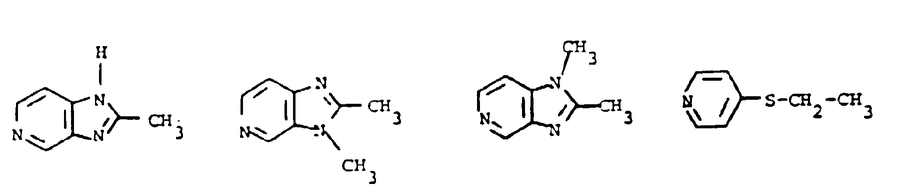

3. Preparation process according to claim 1 or 2, characterized in that the reagent capable of introducing the R₁ radical is chosen from the reagents: 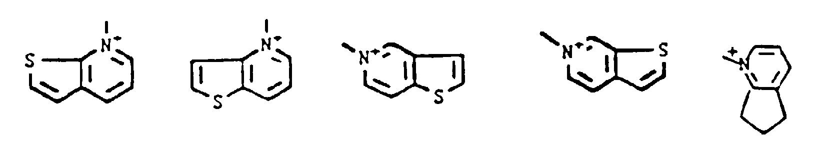 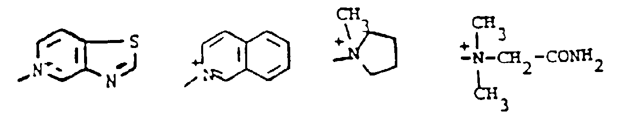 preferably:

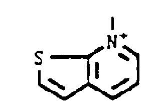

4. Process according to claim 1 for the preparation of the products of formula (I) as defined in claim 1 corresponding to formula (I'): 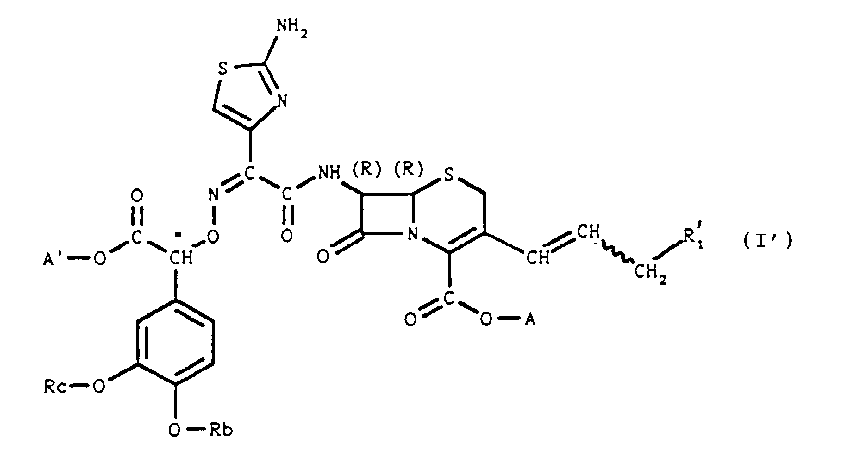 SYN isomer syn isomer, in R or S form or in the form of an R, S, mixture, formula in which:
A, A' R_{b} and R_{c} retain their previous meaning and R'₁ represents a radical chosen from the following radicals: 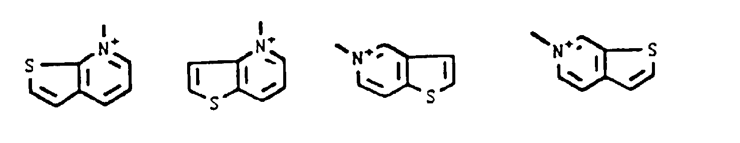 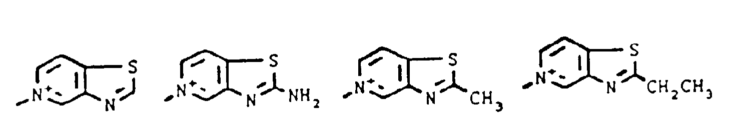 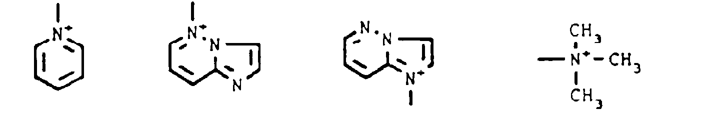 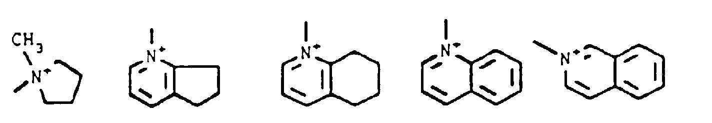 as well as the salts of the products of formula (I') with mineral or organic acids, characterized in that a product of formula (II) is reacted with a product of formula (III) defined as in claim 1, then a reagent chosen from the reagents of formula: 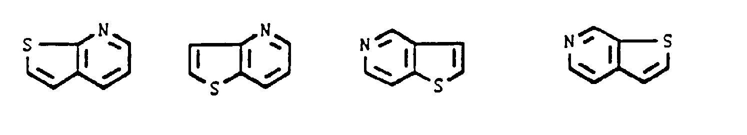 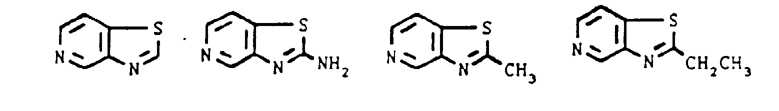 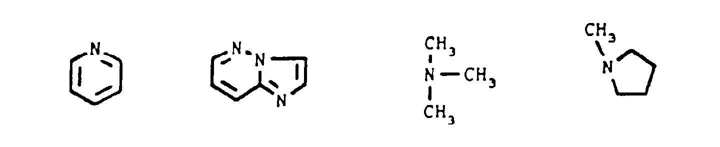 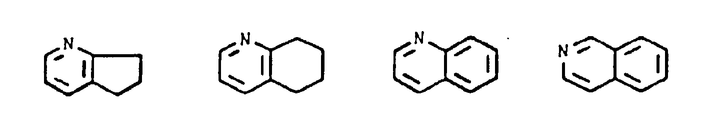 is reacted with the product of formula (IV) obtained, in order to obtain a product of formula (V'): 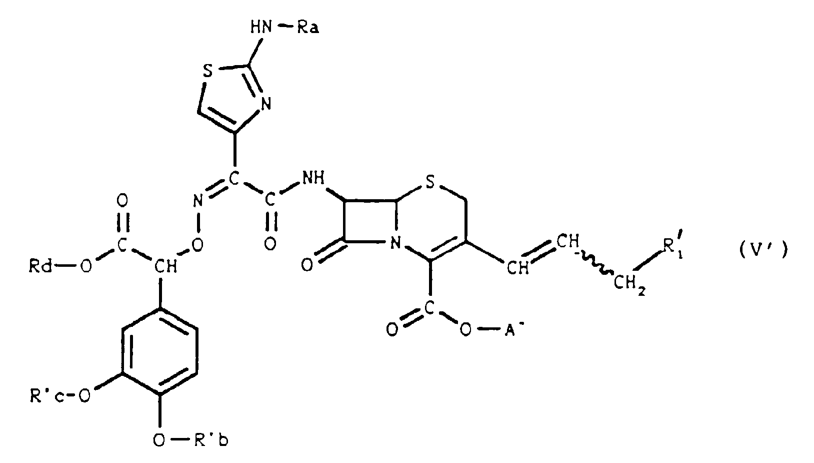 which, if desired, is separated into its E or Z isomers or the Z isomers are converted into E isomers and which products of formula (V), if necessary or if desired, are subjected to one or more of the following reactions, in any order:
a) cleaving, by hydrolysis or by the action of thiourea, of all or part of the ester groups or protective groups of the amino radical or the hydroxyl radicals,
b) esterification or salification of the carboxylic radical or radicals by a base,
c) salification of the amino radical by an acid,
d) separation of products in the form of an R, S mixture into R or S.

5. Process according to claim 4, characterized in that the reagent used on the product of formula (IV) is chosen from the reagents: 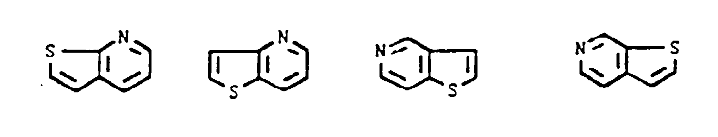 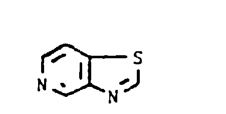 preferably:

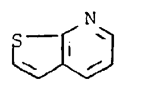

6. Process according to any one of claims 1 to 5, characterized in that the starting products and the reagent used are chosen so as to prepare:
- [6R-[3(E), 6alpha, 7beta(Z)]]-5-[3-[7-[[(2-amino-4-thiazolyl)-[[1-(3,4-dihydroxyphenyl)-2-hydroxy-2-ox- oethoxy]imino]-acetyl]-amino]-2-carboxy-8-oxo-5-thia-1-azabicyclo [4,2,0]-oct-2-en-3-yl]-2-propenyl]-thiazolo-[4,5-c]pyridinium in the R or S form or in the form of an R, S mixture and in the form of an internal salt or a salt with alkali metals, alkaline-earth metals, magnesium, ammonia, amino organic bases, acids and its easily cleavable esters,
- [6R-[3(E),6alpha, 7beta(Z)]]-7-[3-[7-[[(2-amino-4-thiazolyl)-[[1-(3,4-dihydroxyphenyl)-2-hydroxy-2-oxoethoxy] imino]-acetyl]-amino]-2-carboxy-8-oxo-5-thia-1 -azabicyclo-[4,2,0]-oct-2-en-3-yl]-2-propenyl]-thieno-[2,3-b] pyridinium in the R or S form or in the form of an R, S mixture and in the form of an internal salt or a salt with alkali metals, alkaline-earth metals, magnesium, ammonia, amino organic bases, acids and its easily cleavable esters and particularly in the S form,
- [6R-[3(E), 6alpha, 7beta(Z)]]-2-[3-[7-[[(2-amino-4thiazolyl)-[[1-(3,4-dihydroxyphenyl)-2-hydroxy-2-oxoethoxy] imino]-acetyl]-amino]-2-carboxy-8-oxo-5-thia-1-azabicyclo[4,2,0]-oct-2-en-3-yl]-2-propenyl] isoquinolinium in the R or S form or in the form of an R, S mixture and in the form of an internal salt or a salt with alkali metals, alkaline-earth metals, magnesium, ammonia, amino organic bases, acids and its easily cleavable esters,
- [6R-[3(E), 6alpha, 7beta(Z)]]-1-[3-[7-[[(2-amino-4-thiazolyl)-[[1-(3,4-dihydroxyphenyl)-2-hydroxy-2-ox- oethoxy]imino]-acetyl]-amino]-2-carboxy-8-oxo-5-thia-1-azabicyclo[4,2,0]-oct-2-en-3-yl]-2-propenyl]-1-methyl pyrrolidinium in the R or S form or the form of an R, S mixture and in the form of an internal salt or a salt with alkali metals, alkaline-earth metals, magnesium, ammonia, amino organic bases, acids and its easily cleavable esters,
- [6R-[3(E), 6alpha, 7beta(Z)]]-1-[3-[7-[[(2-amino-4-thiazolyl)-[[1-(3,4-dihydroxyphenyl)-2-hydroxy-2-ox- oethoxy]imino]-acetyl]-amino]-2-carboxy-8-oxo-5-thia-1-azabicyclo-[4,2,0]-oct-2-en-3-yl]-2-propenyl]-6,7-dihydro-5H-pyridinium in the R or S form or in the form of an R, S mixture and in the form of an internal salt or a salt with alkali metals, alkaline-earth metals, magnesium, ammonia, amino organic bases, acids and its easily cleavable esters,
- [6R-[3(E), 6alpha, 7beta(Z)]]-N-(2-amino-2-oxoethyl)-3-[7-[[(2-amino-4-thiazolyl)-[[1-(3,4-dihydroxyphenyl)-2-hydroxy-2-oxoethoxy]-imino]-acetyl]-amino]-2-carboxy-8-oxo-5-thia-1-azabicyclo-[4, 2,0]-oct-2-en-3-yl]-N, N-dimethyl-2-propen-1- aminium in the R or S form or in the form of an R, S mixture and in the form of an internal salt or a salt with alkali metals, alkaline-earth metals, magnesium, ammonia, amino organic bases, acids and its easily cleavable esters.

7. Preparation process for pharmaceutical compositions characterized in that at least one of the derivatives of formula (I) as defined in claim 1, as well as their salts with pharmaceutically acceptable acids, is used as active ingredient in a form intended for this use.

8. Preparation process for pharmaceutical compositions characterized in that at least one of the derivatives of formula (I') as defined in claim 4, as well as their salts with pharmaceutically acceptable acids, is used as active ingredient in a form intended for this use.

9. Preparation process for pharmaceutical compositions characterized in that at least one of the derivatives of formula (I) as defined in claim 6, as well as their salts with pharmaceutically acceptable acids, is used as active ingredient in a form intended for this use.

10. As industrial products, the products of formula (IV) and the products of formula (V) in which Rₐ represents a protective group of the amino radical, formulae (IV) and (V) being as defined in claim 5.
